# EUROPEAN PATENT APPLICATION

(11) **EP 4 148 132 A1**
(43) Date of publication of application: **15.03.2023**
(21) Application number: 21799558.8
(22) Date of filing: 07.05.2021
(51) Int. Cl.: C12N 15/115, C07K 14/76, A61K 31/7088, A61K 38/00, C12N 15/10, A61P 35/00

(54) **NOVEL NUCLEIC ACID LIGAND, AND METHOD FOR IDENTIFYNG SAME**

(30) Priority: 07.05.2020 KR 20200054192
(71) Applicant: Interoligo Corporation, Gyeonggi-do 14058 (KR)
(72) Inventor: LEE, Jong Ook, Seongnam-si Gyeonggi-do 13457 (KR); LEE, Jung Hwan, Yongin-si, Gyeonggi-do 16803 (KR); KIM, Do Young, Uiwang-si Gyeonggi-do 16004 (KR); PARK, Han Seul, Seoul 07026 (KR); LEE, Se Na, Anyang-si Gyeonggi-do 14016 (KR); LEE, Da Gyeong, Goyang-si Gyeonggi-do 10303 (KR); KIM, So Yeon, Gwacheon-si Gyeonggi-do 13836 (KR); CHOI, Ji Ah, Uiwang-si Gyeonggi-do 16003 (KR)
(74) Representative: Kilger, Ute
(86) International application number: PCT/KR2021/005773
(87) International publication number: WO 2021/225423

(57) **Abstract**

One aspect according to the present disclosure relates to a novel nucleic acid ligand which is a new class of nucleic acid compound, the existence of which was considered impossible in the prior art. The novel nucleic acid ligand has specific binding affinity with respect to at least two different targets having three-dimensional structures, and the binding sites for the at least two targets are formed in or from a single nucleic acid ligand. The novel nucleic acid ligand according the present disclosure can simultaneously solve several problems of existing aptamers that the prior art could not solve. One aspect according to the present disclosure relates to a novel screening method for identifying the above-mentioned novel nucleic acid ligand. The novel screening method uses a step for sequentially contacting at least two different targets having three-dimensional structures to screen a novel nucleic acid ligand that was previously thought impossible.

## Description

### TECHNICAL FIELD

The present disclosure relates to a novel nucleic acid ligand and a method for identifying the same.

### BACKGROUND

### 1. SELEX technology for aptamer detection

Aptamers are single-stranded nucleic acid (DNA or RNA) fragments that can bind to various types of targets, including low molecular compounds and proteins, with high affinity and specificity. Aptamers can specifically bind only to target molecules, and the binding affinity therefor is very high, of several µM to several nM levels. Similar to monoclonal antibodies, aptamers specifically recognize and bind to targets. Thus, aptamers can be used for diagnostic reagents, analytical reagents, harmful substance detection, and the like in place of monoclonal antibodies and can be applied to the fields of biosensors, bio-imaging, drug delivery, and medicines. For example, since the first aptamer drug, Macugen, has been approved as a medicine for the treatment of macular degeneration by the FDA in 2004, aptamer-based medicines have been actively researched and developed worldwide. In Korea, academia and biotechnology companies are becoming increasingly interested in aptamers.

The detection and development of aptamers are accomplished through a method called systematic evolution of ligands by exponential enrichment (SELEX). SELEX, first developed in 1990, is a process of repeatedly screening oligonucleotides binding to a desired target from nucleic acid libraries having random sequences, whereby nucleic acid aptamers having high binding affinity for "one specific target" can be obtained. That is, in the SELEX method, target-bound oligonucleotides are selected and separated from target-unbound oligonucleotides in the SELEX nucleic acid library pool including 10¹⁴ to 10¹⁶ single-stranded random oligonucleotides each generally consisting of 40 to 100 nucleotides. The target-bound oligonucleotides are amplified by PCR, and then the amplified oligonucleotides are again used as a library pool. The above selection process is repeated several times to exclude target-unbound oligonucleotides, thereby obtaining aptamers that specifically bind to "only one target" with high affinity.

Based on such technology, research and development has focused on how to detect and screen aptamer specifically binding to one target more quickly and accurately or how to search aptamers actually having utility or therapeutic effects. For example, in the classic SELEX process, research and development is focused on changing the binding conditions, platforms on which selection is performed, the type of target, the design of nucleic acid libraries, or the matrix on which oligonucleotide libraries are immobilized in order to simplify the process or produce improved aptamers (Non-Patent Document 1). Hence, Negative SELEX and Counter SELEX were developed after the initial classic SELEX, and thereafter, additional techniques, such as IP-SELEX, Capture-SELEX, Cell-SELEX, CE-SELEX, M-SELEX, AFM-SELEX, AEGIS-SELEX, and Animal-SELEX, have been developed (Non-Patent Document 1).

The direction of SELEX and aptamer research that has been developed so far has an unchanging precondition that an oligonucleotide binds to only one target. The direction is for finding out an oligonucleotide having specific binding affinity for only one target, that is, an aptamer. Various additional studies are being conducted to use aptamers, developed and studied by the existing SELEX method, for a variety of purposes, but problems described below have been raised in using aptamers as drugs.

### 2. Limitations of aptamers from conventional SELEX technology

The aptamers obtained by conventional SELEX technology have a fatal disadvantage to be actually used as a drug in vivo due to their physicochemical properties. That is, instability and fast renal clearance due to the degradation by nucleases have been main obstacles to clinical application of aptamers (Non-Patent Document 2). Various studies have been conducted to solve these problems.

Aptamers have a half-life of about 5 minutes in serum and a half-life of about 1 hour in cells due to the degradation by nucleases that are commonly present in the living body (Non-Patent Document 3). To protect aptamers from the degradation by nucleases, aptamers may be chemically modified, wherein there have been known various methods, such as modifying both ends of oligonucleotides, sugar structures of nucleic acids, or phosphodiester backbones. In addition, aptamers have an average diameter of less than 5 nm and a molecular weight of about 6-30 kDa. Therefore, even though the metabolic instability of aptamers was solved to some extent by using modified nucleotides, the clinical application of aptamers is limited since aptamers administered through blood vessels are rapidly excreted from the body through the kidneys due to their small molecular weights and thus has a shortened retention time (Non-Patent Document 2). To overcome this problem, aptamers are formulated in the form of being conjugated to macromolecules, such as polyethylene glycol (PEG), proteins, cholesterols, fatty acids, or liposomes, and thus are not easily filtered through the kidneys, thereby extending the retention time in the body. Of these, PEG is the most widely used.

However, aptamers, which are single-stranded oligonucleotides, naturally form various three-dimensional shapes, such as helices and loops, and these three-dimensional structures are involved in target recognition and binding. The affinity and specificity of aptamers for targets are sensitively affected by their structures. Therefore, the modification of aptamers with macromolecules in the post-SELEX process is sufficiently predictable to have negative effects on the properties and folding structures of the aptamers.

Especially, cases have been reported that the use of PEGylated aptamers to increase the circulating half-life of aptamers in the body causes severe allergic responses in phase III trials due to antibodies to PEG already present in the body (Non-Patent Document 4). In addition, the use of aptamers together with macromolecules, such as PEG, may lose therapeutic effectiveness since such molecules affect the structures of aptamers to reduce affinity or specificity for desired targets. Moreover, the modification of aptamers or the use of aptamers with other substances may change quality or performance of aptamers for each batch for aptamer production, and thus the quality of aptamers is difficult to control constantly, and as a result, aptamers, when actually manufactured into drugs, cannot ensure the consistency of effects thereof. The high cost required for quality control reduces the advantage of aptamers that production costs are low.

Additionally, the aptamers that have been studied so far could not be actively delivered alone into the cytoplasm via the cell membrane. Therefore, most aptamers have been developed to target only membrane proteins or secretory proteins, and aptamers targeting proteins present in the cytoplasm or the membranes of intracellular organelles were mostly selected from purified proteins, wherein carriers, such as transferrin proteins, cholesterol, and liposomes, were used to improve the cell permeability of the selected aptamers. As such, there are various problems that need to be overcome in order to utilize aptamers as drugs actually applied in clinical trials, and various attempts have been made to overcome the problems, but eventually, cases of failure have continued in clinical trials. Macugen is the only aptamer drug that has been approved so far. Hence, there is a continuing need for a new substance that can be clinically used and realized as an actual drug by solving the problems of aptamers.

### 3. Bispecific aptamers and limitations thereof

As described above, an aptamer has a property of binding to only one target. To overcome this limitation, bi- or multi-specific aptamers having two or more aptamers with specific binding affinities for different targets have been continuously studied.

Bi-specific aptamers are composed of two distinct aptamers, and such characteristics can promote or regulate, for example, an immune response between a functional molecule and a target cell, and thus have been continuously studied mainly in the field of immune-anticancer drugs (Non-Patent Document 5).

However, each single-specific aptamers constituting the bi-specific aptamer have a unique structure that specifically binds to its target, and thus these aptamers that are coupled to each other with or without a linker may interfere with each other, and thus may affect specific binding affinity of each aptamer. The simply binding of two aptamers specific to different targets does not maintain the binding affinities intact for both the two targets, nor can it be ensured. Therefore, to obtain a bi-specific aptamer that properly functions, there is a difficulty in finding aptamers that neither interfere with each other nor affect each other's structure, through numerous trials and errors. Moreover, it needs to be checked whether a linker does not affect aptamers even during the coupling of the aptamers (Patent Document 2: U.S. Patent No. 8,969,318). Therefore, the detection of bi-specific aptamers is very exhausting.

In addition, bi-specific aptamers are difficult to mass-produce, and the ease of manufacture, which is one of the advantages of aptamers, may be lost. As an aptamer is longer, a purely purified aptamer having a desired sequence is increasingly difficult to isolate during the manufacture or synthesis, resulting in increased impurities, and thus quality control is difficult. Therefore, to satisfy a large-scale production process that needs to consider the purity and synthesis yield of final products, the length of aptamers is preferably as short as possible. However, most bi-specific aptamers are obtained by coupling two aptamers via a linker, which is a substantial limitation in the commercialization of aptamers as medicines.

Moreover, bi-specific aptamers are difficult to utilize as drugs since the bi-specific aptamers have the above-mentioned disadvantages (in vivo instability, fast renal clearance, inability to deliver into cells, etc.) of aptamers as drugs.
(Patent Document 1) Korean Patent Publication No. 2019-0126356
(Patent Document 2) U.S. Patent No. 8,969,318
(Non-Patent Document 1) Zhang et al., Recent Advances in Aptamer Discovery and Applications, Molecules 2019, 24, 941
(Non-Patent Document 2) Zhou, J.; Rossi, J. Aptamers as Targeted Therapeutics: Current Potential and Challenges. Nat. Rev. Drug Discovery 2017, 16, 181-202.
(Non-Patent Document 3) Dougan, H.; Lyster, D. M.; Vo, C. V.; Stafford, A.; Weitz, J. I.; Hobbs, J. B. Extending the Lifetime of Anticoagulant Oligodeoxynucleotide Aptamers in Blood. Nucl. Med. Biol. 2000, 27, 289-297.
(Non-Patent Document 4) Povsic, T. J., et al. J ALLERGY CLIN IMMUNOL, 2016, 138(6), 1712
(Non-Patent Document 5) M. M. Soldevilla, H. Villanueva, F. Pastor, "Aptamers: A Feasible Technology in Cancer Immunotherapy", Journal of Immunology Research, vol. 2016, Article ID 1083738, 12 pages, 2016. https://doi.org/10.1155/2016/1083738

### SUMMARY

The present inventors discovered a novel nucleic acid ligand beyond the conventional aptamer concept and thus completed the present disclosure.

The conventional SELEX method and aptamers developed thereby showed limitations that were difficult to overcome in spite of long-term studies, and thus medicines using aptamers were difficult to provide. In fact, there have been no successful case of development for 15 years since Macugen was introduced.

The inventors of the present disclosure facing the above problems discovered that a novel nucleic acid ligand but not an aptamer could solve the limitations of conventional aptamers, and then completed the present disclosure. The aptamer is defined as a nucleic acid molecule that binds to a single target, such as a monoclonal antibody. However, the inventors of the present disclosure discovered a novel nucleic acid ligand capable of having specific binding affinities for two or more different targets and thus solved the problems of conventional aptamers.

A nucleic acid ligand according to one embodiment of the present disclosure may be obtained by utilizing the conventional SELEX method, and it has not been completely known in the art that a nucleic acid ligand capable of binding to two or more different targets can be detected. The researchers or scientists engaged in the art of the present disclosure thought that it was impossible to discover a nucleic acid ligand capable of binding to two or more different targets by using the conventional SELEX technology. The reason is that in the conventional SELEX technology, it was common knowledge to remove or exclude oligonucleotides not binding to desired targets or to remove or exclude oligonucleotides binding to other targets. The nucleic acid ligand of the present disclosure does not conform to the conventional definition of aptamer and is a breakthrough that does not follow the development trend or common knowledge in the art of aptamers.

Various embodiments of the present disclosure provide a novel nucleic acid ligand that simultaneously solves the limitations occurring when the above conventional aptamers are applied in clinical trials.

Various embodiments of the present disclosure provide a novel preparation method or novel screening method for a novel nucleic acid ligand.

Specifically, the present disclosure can provide a novel nucleic acid ligand capable of having binding affinities specific for two or more different targets, wherein binding sites of the nucleic acid ligand to two or more targets are not present separately from each other. In this regard, the nucleic acid ligand according to one embodiment of the present disclosure is not a bi-specific aptamer provided in the form in which two or more aptamers are coupled to each other.

In one embodiment, the single nucleic acid ligand that binds to a plasma protein (e.g., albumin) as a first target and binds to a cancer cell-specific molecule as a second target, when administered to the blood, uses the first target, that is, binds to the albumin protein in plasma and thus can reach a cancer tissue while avoiding degradation by nucleases or renal excretion in the body. The nucleic acid ligand, after reaching the cancer tissue, enters the cell via a pathway of endocytosis or the like to bind to a cancer cell-specific molecule in the cell, thereby exerting an intended therapeutic effect. As described above, the nucleic acid ligand according to one embodiment of the present disclosure is to solve the problems of conventional aptamers, such as in vivo instability, fast renal clearance, and inability to deliver into cells.
1. The present disclosure in one embodiment may relate to a non-natural nucleic acid ligand having specific binding affinities for two or more different targets, wherein each of the targets has a three-dimensional structure, and wherein the non-natural nucleic acid ligand is not a coupled body of a plurality of aptamers (non-coupling).
2. The present disclosure in one embodiment may relate to a non-natural nucleic acid ligand having specific binding affinities for two or more different targets, wherein each of the targets has a three-dimensional structure, and wherein in the nucleic acid ligand, all or part of a nucleic acid sequence forming a binding site to one target forms all or part of a nucleic acid sequence forming a binding site to another target.
3. The present disclosure in one embodiment may relate to a non-natural nucleic acid ligand having specific binding affinities for two or more different targets, wherein each of the targets has a three-dimensional structure, and wherein in the nucleic acid ligand, binding sites to two or more targets are not present separately from each other.
4. The present disclosure in one embodiment may relate to a non-natural nucleic acid ligand having specific binding affinities for two or more different targets, wherein each of the targets has a three-dimensional structure, and wherein binding sites to two or more targets are formed in one single nucleic acid ligand.
5. In one embodiment, the part of the nucleic acid sequence forming binding sites to the targets may be about 1% to about 99%, about 5% to about 95%, about 10% to about 90%, about 20% to about 80%, about 30% to about 70%, or about 40% to about 60% of the entire sequence of the non-natural nucleic acid ligand.
6. In one embodiment, the non-natural nucleic acid ligand may have specific binding specificities to two different targets.
7. In one embodiment, the targets may be selected from the group consisting of cells, viruses, and proteins.
8. In one embodiment, one of the targets may be a plasma protein and the other may be a therapeutic target.
9. In one embodiment, the plasma protein may be selected from the group consisting of albumin, alpha 1 globulin, alpha 2 globulin, beta globulin, gamma globulin, immunoglobulin A, immunoglobulin G, lipoproteins, fibrinogen, transferrin, and transthyretin.
10. In one embodiment, the therapeutic target may be a protein present in a cell or a protein present in a cellular membrane.
11. In one embodiment, the therapeutic target may be selected from the group consisting of membrane proteins, transmembrane proteins, glycoproteins, immune antibodies, viruses, viral envelope glycoproteins, viral enzymes, secretory proteins, serine proteases, peptide hormones, neurotransmitters, hormones, dehydrogenases, cytokines, E3 ubiquitin ligases, neuropeptides, hydrolases, serine protease inhibitors, phosphatase, chemokine proteins, methyl transferases, oxidases, growth factors, bacteria, bacterial proteins, intracellular proteins, extracellular matrices, receptors, transcription factors, and tumor proteins.
12. In one embodiment, the therapeutic target may be selected from the group consisting of 4-1BB, acetylcholine receptors, alpha thrombin, amylin, angiopoietin 1, angiopoietin 2, AXL, BCL-2, BMPR-1R, BRD1, BRD2, BRD3, BRD4, BRDT, BTLA, calcitonin gene-related peptides, CREB-binding protein (CPB), CCK4/PTK7, CD16a, CD16b, CD19, CD20, CD200, CD200R, CD27, CD28, CD3, CD30, CD32A, CD32B, CD33, CD4, CD40L, CD52, CD80, CD94, CSF1R, CTLA-4, DDR1, DDR2, E2F1, EGFR, EPH, ERBB2, FGF, FGFR, ghrelin, GITR, glypican 3, gonadotropin-releasing hormone 1, HIV gp120, HIV-1 integrase, HIV-1 reverse transcriptase, HRAS, HVEM, ICOS, IDH1, IGF1R, immunoglobulin E, interferon-gamma, KIT, KRAS, LAG3, LFA, L-selectin, LTK, MDM2, MDMX, mucin 1, MYC, neurotensin 1, neutrophil elastase, NF-Kb, NKG2D, nociceptin, NTRK1, NTRK2, OX-40, PD-1, PDGF, PDGFRfamily, PD-L1, PD-L2, phospholipase A2, plasminogen activation inhibitor 1, PP2A, PPM1D, PPP2CA, protein tyrosine phosphatase, P-selectin, PSMA, respiratory syncytial virus, RET, SDF1b, SetDB1, SLAMF7, Staphylococcus enterotoxin B, STAT3, tenascin, TGFBR, TIGIT, TIM3, TNFSF7, tyrosinase, VCAM-1, VEGF, VEGFR family, αᵥβ₃ integrin, and mutants thereof.
13. In one embodiment, the therapeutic target may be selected from the group consisting of KRAS G12D, KRAS G12V, KRAS G12C, KRAS G12A, KRAS G12S, KRAS G12R, KRAS G13D, KRAS Q61H, and combinations thereof.
14. In one embodiment, the non-natural nucleic acid ligand may consist of about 100 or less nucleotides.
15. In one embodiment, the nucleotides may be selected from the group consisting of DNA nucleotides, RNA nucleotides, modified nucleotides thereof, and combinations thereof.
16. In one embodiment, the non-natural nucleic acid ligand may consist of one nucleic acid sequence selected from the group consisting of SEQ ID NO: 6 to SEQ ID NO: 183.
17. In one embodiment, the nucleic acid may be selected from the group consisting of single-stranded RNA, double-stranded RNA, single-stranded DNA, and double-stranded DNA.
18. A pharmaceutical composition for prevention or treatment of cancer according to one embodiment of the present disclosure may include the non-natural nucleic acid ligand according to one embodiment.
19. In one embodiment, the non-natural nucleic acid ligand may have specific binding affinities for a plasma protein as a first target and a therapeutic target protein as a second target.
20. In one embodiment, the pharmaceutical composition may be administered intravenously to a patient.
21. In one embodiment, the cancer may be a solid cancer.
22. In one embodiment, the cancer may express a PD-L1 protein on the surface of cancer cells.
23. In one embodiment, the cancer may be at least one selected from the group consisting of brain cancer, lung cancer, colorectal cancer, small intestine cancer, stomach cancer, testicular cancer, thyroid cancer, cervical cancer, skin cancer, bladder cancer, ovarian cancer, kidney cancer, liver cancer, pancreatic cancer, urothelial cell cancer, and breast cancer.
24. In one embodiment, cancer cells of the cancer may have a KRAS mutant protein.
25. In one embodiment, the cancer may be at least one selected from the group consisting of lung cancer, colorectal cancer, pancreatic cancer, breast cancer, ovarian cancer, endometrial cancer, cervical cancer, bladder cancer, gallbladder cancer, biliary tract cancer, skin cancer, stomach cancer, brain cancer, kidney cancer, and acute myeloid leukemia.
26. In one embodiment, the non-natural nucleic acid ligand may consist of one nucleic acid sequence selected from the group consisting of SEQ ID NO: 24, SEQ ID NO: 27, SEQ ID NO: 30, SEQ ID NO: 84, and SEQ ID NO: 100.
27. A diagnostic composition according to one embodiment of the present disclosure may include the non-natural nucleic acid ligand according to one embodiment.
28. A contrast medium according to one embodiment of the present disclosure may include the non-natural nucleic acid ligand according to one embodiment.
29. A radiopharmaceutical according to one embodiment of the present disclosure may include the non-natural nucleic acid ligand according to one embodiment.
30. A composition for diagnosis of cancer according to one embodiment of the present disclosure may include the non-natural nucleic acid ligand according to one embodiment.
31. A method for identifying a non-natural nucleic acid ligand having specific binding affinities for two or more different targets, according to one embodiment of the present disclosure, may include contacting one or more nucleic acid ligand candidate sequences with each of two or more different targets in a sequential manner, wherein the contacting with each of the targets includes identifying one or more nucleic acid ligand candidate sequences having specific binding affinity for said target, and wherein each of the targets has a three-dimensional structure.
32. In one embodiment, the method may include: (a) contacting one or more first nucleic acid ligand candidate sequences with a target and identifying one or more second nucleic acid ligand candidate sequences specifically binding to the target; and (b) contacting (i) the one or more second nucleic acid ligand candidate sequences obtained in step (a) or (ii) one or more third nucleic acid ligand candidate sequences prepared using the one or more second nucleic acid ligand candidate sequences obtained in step (a) with a target different from the target in step (a) and identifying one or more fourth nucleic acid ligand candidate sequences specifically binding to the different target.
33. In one embodiment, step (b) may further include preparing the one or more third nucleic acid ligand candidate sequences by linking a random sequence consisting of a plurality of nucleotides to at least one of both ends of the one or more second nucleic acid ligand candidate sequences obtained in step (a).
34. In one embodiment, the random sequence may be linked to any one of both ends of the second nucleic acid ligand candidate sequences, and in the third nucleic acid ligand candidate sequences, the second nucleic acid ligand candidate sequence or a part thereof may be used as a primer sequence for nucleic acid amplification.
35. In one embodiment, the random sequence may consist of about 20 to about 40 consecutive nucleotides, or the third nucleic acid ligand candidate sequences consist of about 40 to about 100 consecutive nucleotides.
36. In one embodiment, the method may be performed by systematic evolution of ligands by exponential enrichment (SELEX).
37. A method for identifying a non-natural nucleic acid ligand having specific binding affinities for two or more different targets, according to one embodiment of the present disclosure, the nucleic acid ligand being identified by systematic evolution of ligands by exponential enrichment (SELEX) starting from one nucleic acid library, the method may include: selecting one target from the group consisting of two or more different targets each having a three-dimensional structure in each SELEX round; and selecting a nucleic acid ligand having specific binding affinity for the selected target in each SELEX round.
38. In one embodiment, after SELEX performed in one or more rounds for one target is completed, SELEX performed in one or more rounds for another target may be performed.
39. In one embodiment, the method may include: (i) performing one or more SELEX rounds on one target; (ii) performing one or more SELEX rounds on a target different from the target in step (i) by using a nucleic acid library obtained in step (i); (iii) performing step (i) again by using a nucleic acid library obtained in step (ii); and (iv) repeating steps (i) to (iii).
40. In one embodiment, the method may further include performing negative SELEX or counter SELEX for another trait of the target.
39. In one embodiment, the SELEX may be at least one selected from the group consisting of classic SELEX, Advanced SELEX, IP-SELEX, Capture-SELEX, Cell-SELEX, CE-SELEX, M-SELEX, AFM-SELEX, AEGIS-SELEX, and Animal-SELEX.
41. In one embodiment, the method may further include evaluating specific binding affinities of the identified nucleic acid ligand candidate sequence for targets.
42. In the method according to one embodiment, the targets may be selected from the group consisting of cells, virus, and proteins.
43. In the method according to one embodiment, the targets may be two different targets.
44. In the method according to one embodiment, one of the targets may be a plasma protein and the other may be a therapeutic target.
45. In the method according to one embodiment, the non-natural nucleic acid ligand may consist of about 40 to about 100 nucleotides.
46. In one embodiment, the method may include removing nucleotides that are uninvolved in specific binding affinity for a target, from each of the identified nucleic acid ligand candidate sequences.
47. A method for detecting a target from a sample, according to one embodiment of the present disclosure, may include contacting a target from the sample with the nucleic acid ligand according to one embodiment.
48. A non-natural nucleic acid ligand according to one embodiment of the present disclosure may be prepared by the method according to one embodiment.

According to the present disclosure in some embodiments, the novel nucleic acid ligand is a single nucleic acid ligand and can exhibit excellent specific binding affinities for two or more different targets.

According to the present disclosure in some embodiments, the novel nucleic acid ligand, when administered as a drug into the body, specifically binds to a plasma protein and thus can exhibit an effect of excellent in vivo instability, slow renal clearance, and ability to deliver into cells via a pathway of endocytosis or the like. The novel nucleic acid ligand can exhibit an excellent therapeutic effect on diseases dependent on therapeutic targets and can be utilized as drugs actual applied to clinical trials. Furthermore, the novel nucleic acid ligand can be utilized in the fields of diagnosis, bio-imaging, drug-discovery tools, analytical reagents, biosensors, food inspection, target (e.g., protein) purification, or the like.

According to the present disclosure in some embodiments, the novel nucleic acid ligand does not need to be used together with other macromolecules (e.g., PEG) after preparation in order to attain the in vivo stability or slow renal clearance, and thus can exclude problems in that such molecules affect the structure of the nucleic acid ligand or reduce the affinity or specificity for a desired target. Therefore, the nucleic acid ligand can maintain excellent therapeutic efficacy intact that has been confirmed in tests, as a drug applied to clinical trials.

According to the present disclosure in some embodiments, the novel nucleic acid ligand has a relatively short sequence length (e.g., an about 20 to about 100 oligonucleotide sequence, and preferably an about 25 to about 65 oligonucleotide sequence) and does not need to be used together with other substances. Therefore, in a mass production process, the quality or performance of nucleic acid ligands is not varied from batch to batch, and easy quality control is attained, and the purity and synthesis yield for using the nucleic acid ligand as a drug can be satisfied. As a result, the novel nucleic acid ligand can be efficiently mass-produced using only an au-synthesizer.

According to the present disclosure in some embodiments, the novel nucleic acid ligand can bind to three-dimensional structures of cells, viruses, or proteins, and such binding can exhibit an excellent therapeutic effect to be attained.

### BRIEF DESCRIPTION OF DRAWINGS

FIG. 1 shows a schematic flowchart for obtaining single nucleic acid ligands by using a plasma protein as a first target protein and a cancer cell-specific protein as a second target protein according to one embodiment.
FIG. 2 shows one example of a schematic diagram of a first selection method as one of the methods for preparing a nucleic acid ligand according to one embodiment.
FIG. 3 shows one example of a schematic diagram of a second selection method as one of the methods for preparing a nucleic acid ligand according to one embodiment.
FIG. 4 shows another example of a schematic diagram of a second selection method as one of the methods for preparing a nucleic acid ligand according to one embodiment.
FIG. 5 shows still another example of a schematic diagram of a second selection method as one of the methods for preparing a nucleic acid ligand according to one embodiment.
FIG. 6A shows the results of bead-based ELISA binding assay in which the binding affinity of nucleic acid library pools obtained through primary SELEX for a plasma protein (human serum albumin) was analyzed.
FIG. 6B shows the results of bead-based ELISA binding assay in which the binding affinity of nucleic acid library pools obtained through primary SELEX for a plasma protein (immunoglobulin G) was analyzed.
FIG. 6C shows the results of bead-based ELISA binding assay in which the binding affinity of nucleic acid library pools obtained through primary SELEX for a plasma protein (transferrin) was analyzed.
FIG. 7 shows the results of filter binding assay in which the binding affinity of nucleic acid library pools obtained through primary SELEX for a plasma protein (albumin) was analyzed.
FIG. 8A shows the results of analyzing binding affinity of nucleic acid library pools obtained through secondary SELEX for KARS G12D mutant protein.
FIG. 8B shows the results of analyzing binding affinity of nucleic acid library pools obtained through secondary SELEX for KARS G12V mutant protein.
FIG. 8C shows the results of analyzing binding affinity of nucleic acid library pools obtained through secondary SELEX for KARS G12C mutant protein.
FIG. 8D shows the results of analyzing binding affinity of nucleic acid library pools obtained through secondary SELEX for BCL2.
FIG. 9A shows the results of analyzing binding affinity of clone nucleic acid ligands selected from the secondary SELEX nucleic acid library pools for human serum albumin (HSA).
FIG. 9B shows the results of analyzing binding affinity of clone nucleic acid ligands selected from the secondary SELEX nucleic acid library pools for PD-L1 protein.
FIG. 10A shows the results of analyzing binding affinity of clone nucleic acid ligands selected from the secondary SELEX nucleic acid library pools for human serum albumin (HSA).
FIG. 10B shows the results of analyzing binding affinity of clone nucleic acid ligands selected from the secondary SELEX nucleic acid library pools for KRAS G12D protein.

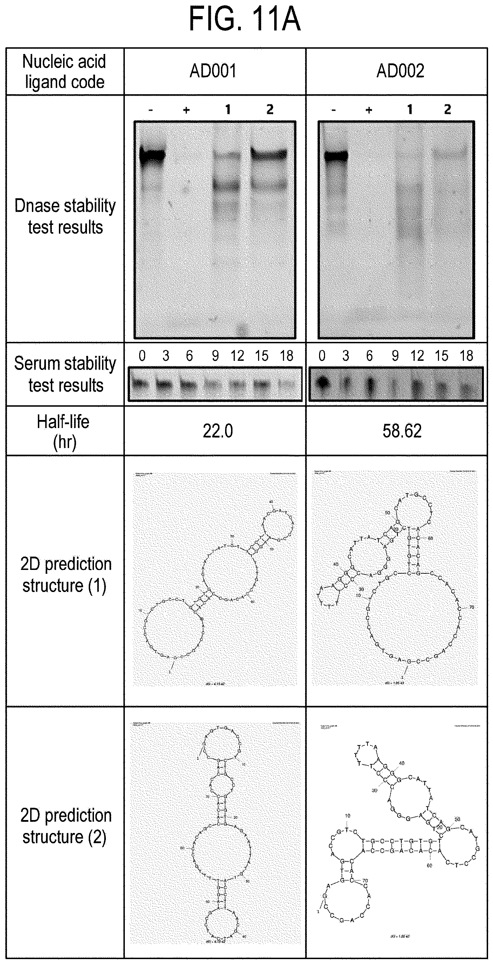

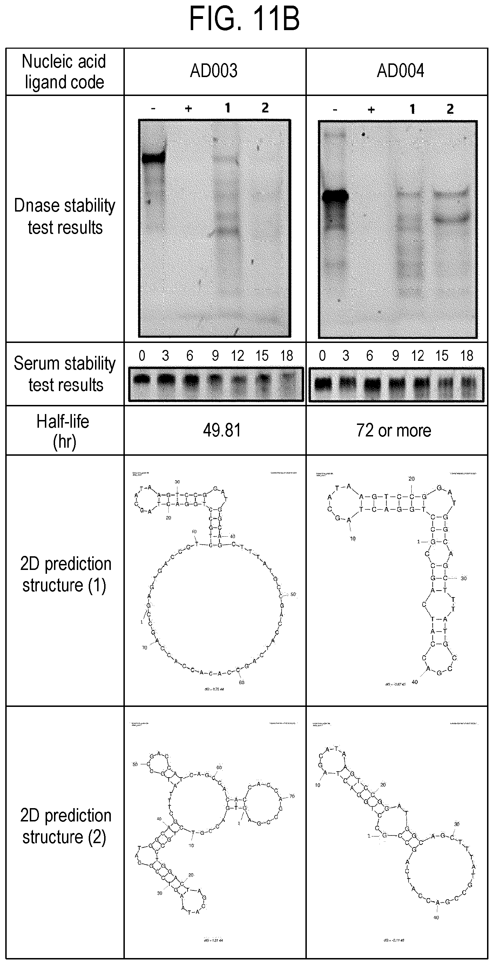

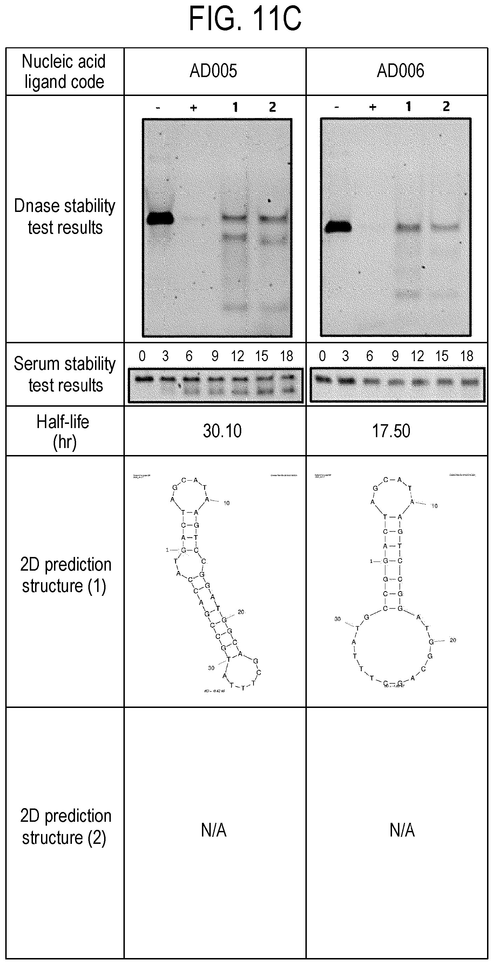

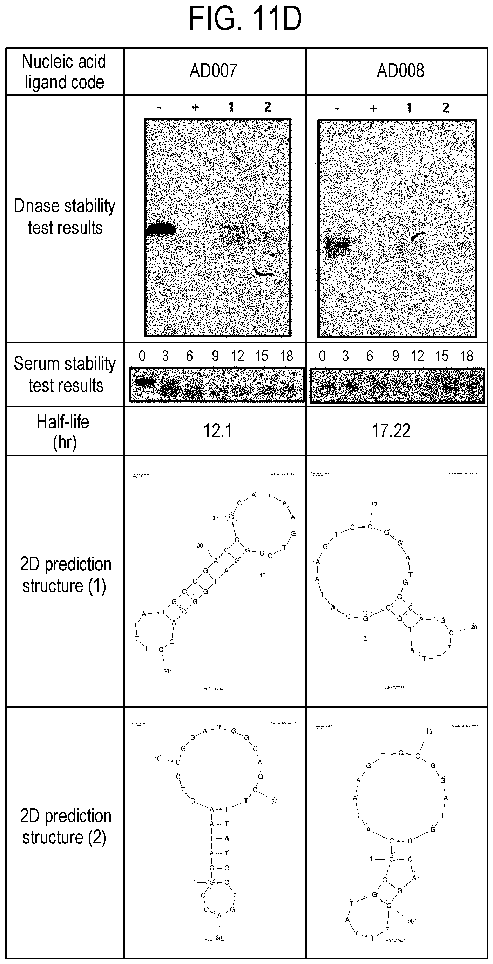

FIGS. 11A to 11D show Dnase stability results of human serum albumin (HSA)- and KRAS G12D-targeted nucleic acid ligands according to one embodiment against human serum albumin (HSA) and KRAS G12D protein and 2D prediction structures of the individual nucleic acid ligands. In the Dnase stability test results, the sign (-) indicates a negative control without the addition of target proteins and a nuclease; the sign (+) indicates a positive control with the addition of only a nuclease; the numeral 1 indicates a test group with human serum albumin and a nuclease, and the numeral 2 indicates a test group with KRAS G12D protein and a nuclease.

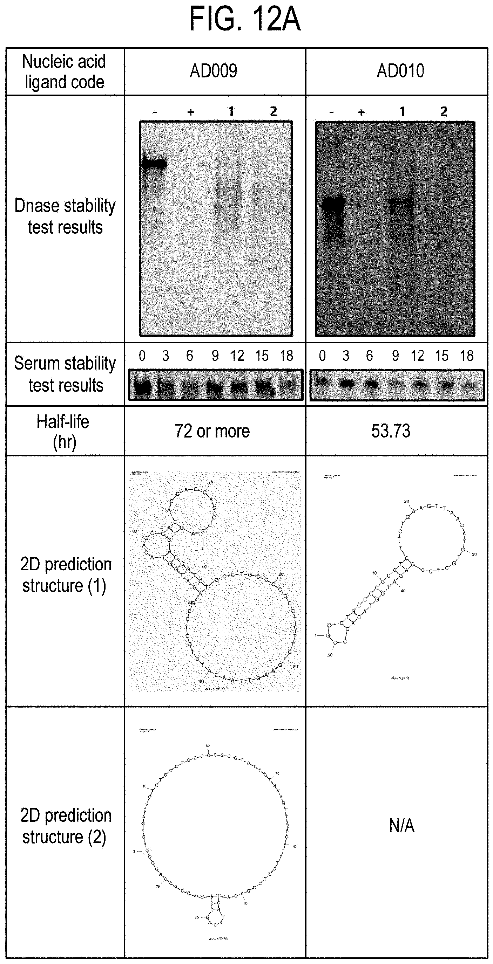

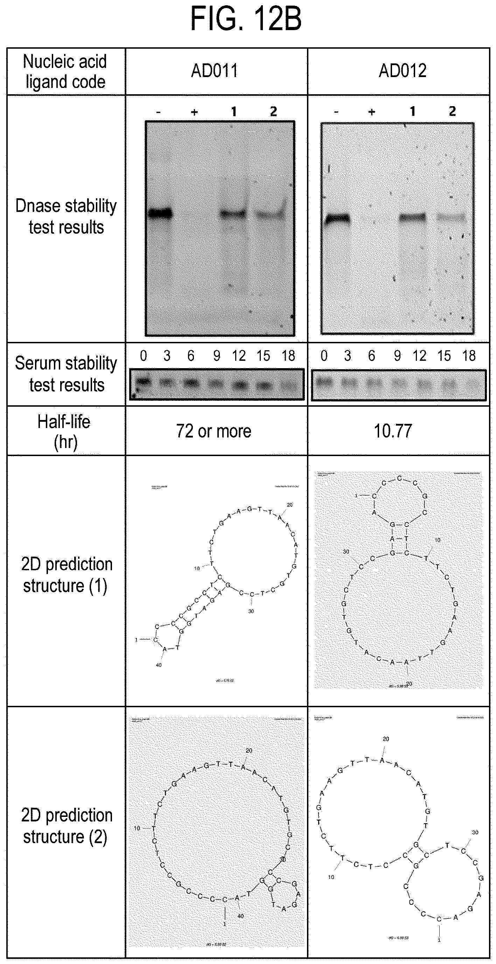

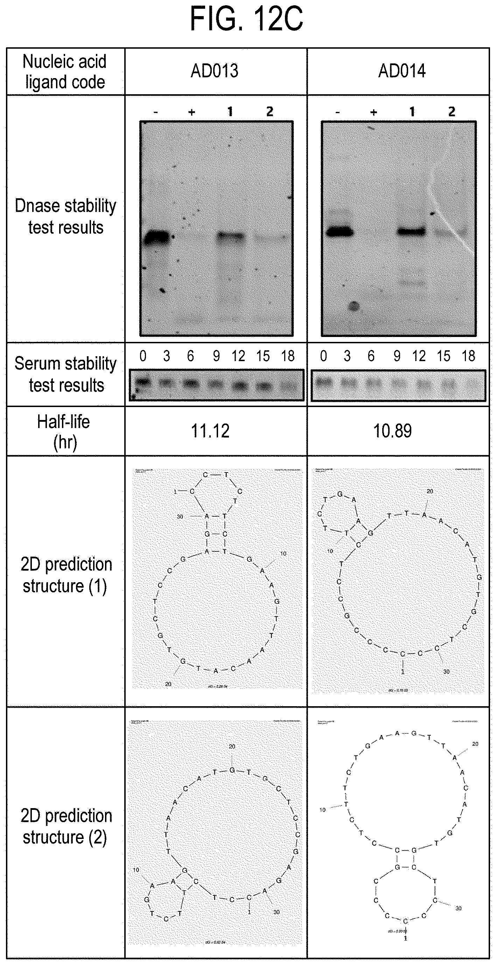

FIGS. 12A to 12C show Dnase stability results of human serum albumin (HSA)- and KRAS G12D-targeted nucleic acid ligands according to one embodiment against human serum albumin (HSA) and KRAS G12D protein and 2D prediction structures of the individual nucleic acid ligands. In the Dnase stability test results, the sign (-) indicates a negative control without the addition of a target protein and a nuclease; the sign (+) indicates a positive control with the addition of only a nuclease; the numeral 1 indicates a test group with human serum albumin and a nuclease, and the numeral 2 indicates a test group with KRAS G12D protein and a nuclease.
FIG. 13 shows the UPLC analysis results, before and after purification, of the nucleic acid ligand AD005 according to one embodiment.
FIG. 14 shows the mass spectrometry results, after purification, of the nucleic acid ligand AD005 according to one embodiment.
FIGS. 15A and 15B show the serum stability results of human serum albumin (HSA)- and KRAS G12D-targeted nucleic acid ligands according to one embodiment in various species.
FIG. 16 shows the results of confirming stability of human serum albumin (HSA)- and KRAS G12D-targeted nucleic acid ligands according to one embodiment after the reaction with a KRAS mutant or wild-type protein and treatment with DNase.
FIG. 17 shows the serum half-life test results of human serum albumin (HSA)- and KRAS G12D-targeted nucleic acid ligands according to one embodiment.
FIG. 18 shows the binding affinity analysis results of a nucleic acid ligand capable of specifically binding to human serum albumin (HSA) and KRAS G12D mutant protein according to one embodiment for KARS G12D.
FIGS. 19A to 19D show the microscopy results of the intracellular delivery effect of human serum albumin (HSA)- and KRAS G12D-targeted nucleic acid ligands according to one embodiment.
FIGS. 20A and 20B show the results of confirming through three-dimensional analysis that a human serum albumin (HSA)- and KRAS G12D-targeted nucleic acid ligand was colocalized with the macropinocytosis marker 70 KDa dextran-TMR.
FIG. 21 shows the cancer cell viability by the treatment with human serum albumin (HSA)- and KRAS G12D-targeted nucleic acid ligands according to one embodiment.
FIGS. 22A and 22B show the cancer cell viability by the treatment with human serum albumin (HSA)- and KRAS G12D-targeted nucleic acid ligands according to one embodiment.
FIG. 23 shows the cancer cell viability inhibitory effect of a human serum albumin (HSA)- and KRAS G12D-targeted nucleic acid ligand prepared according to one embodiment after the treatment with an endocytosis inhibitor.
FIGS. 24A and 24B show the effect of inhibiting the phosphorylation of ERK, a protein downstream of the KARS signaling pathway, by human serum albumin (HSA)- and KRAS G12D-targeted nucleic acid ligands according to one embodiment.
FIG. 25 shows a schematic diagram of intracellular delivery and action mechanisms of a human serum albumin (HSA)- and KRAS G12D-targeted nucleic acid ligands according to one embodiment.
FIGS. 26A to 26C show a tumor inhibitory effect of a human serum albumin (HSA)- and KRAS G12D-targeted nucleic acid ligand according to one embodiment in a MIA PaCa-2 pancreatic cancer cell line-injected xenograft mouse.
FIGS. 27A and 27C show the in vivo stability results with respect to changes in mouse organ weights by the administration of a human serum albumin (HSA)- and KRAS G12D-targeted nucleic acid ligand according to one embodiment.
FIGS. 28A and 28B show the tumor inhibitory effect depending on the dose of a serum albumin (HSA)- and KRAS G12D-targeted nucleic acid ligand according to one embodiment.
FIG. 29 shows the results of change in mouse organ weights and in vivo stability results over with changes in mouse organ weights depending on the dose of a human serum albumin (HSA)- and KRAS G12D-targeted nucleic acid ligand according to one embodiment.
FIG. 30 shows the measurement results of liver damage indexes depending on the dose of a human serum albumin (HSA)- and KRAS G12D-targeted nucleic acid ligand according to one embodiment.
FIG. 31 shows the results of measuring white blood cell, red blood cell, and platelet levels by a human serum albumin (HSA)- and KRAS G12D-targeted nucleic acid ligand according to one embodiment.
FIG. 32 shows a PD-1/PD-L1 binding inhibitory effect at the protein level by human serum albumin (HSA)- and PD-L1-targeted nucleic acid ligands according to one embodiment.
FIG. 33 shows a PD-1/PD-L1 binding inhibitory effect at the protein level by human serum albumin (HSA)- and PD-L1-targeted nucleic acid ligands according to one embodiment.
FIG. 34 shows a PD-1/PD-L1 binding inhibitory effect at the cell level by human serum albumin (HSA)- and PD-L1-targeted nucleic acid ligands according to one embodiment.

### DETAILED DESCRIPTION

Various embodiments described herein are illustrated for the purpose of clarifying the technical idea of the present disclosure, and are not intended to limit the present disclosure to any specific embodiment. The technical idea of the present disclosure includes various modifications, equivalents, alternatives and embodiments selectively combined from all or part of individual embodiments described in the present document. Further, the scope of the technical idea of the present disclosure is not limited to the various embodiments described below, and the detailed description thereof.

The terms used herein, including technical or scientific terms, may have meanings that are generally understood by a person having ordinary knowledge in the art to which the present disclosure pertains, unless otherwise specified. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

The singular of an expression used herein may include the meaning of the plural of the expression unless otherwise indicated in the context clearly dictates otherwise, and the same applies to singular forms of expressions as set forth in the claims.

The expressions "1st", "2nd", "first", "second", and the like used herein are used to distinguish one object from another in referring to plural same objects unless otherwise indicated in the context, and do not limit the order or importance of the objects.

The expressions used herein, "A, B and C," "A, B or C," "A, B and/or C," "at least one of A, B, and C," "at least one of A, B, or C," "at least one of A, B, and/or C," "at least one selected from A, B, and C", "at least one selected from A, B, or C", "at least one selected from A, B, and/C", and the like may be used to refer to each listed item or any possible combination of the listed items may be provided. For example, the expression "at least one selected from A and B" may refer to all of (1) A, (2) at least one of A, (3) B, (4) at least one of B, (5) at least one of A and at least one of B, (6) B and at least one of A, (7) A and at least one of B, and (8) both of A and B.

The term "about" or "approximately" used herein may refer to the usual error range for each value, as is widely known to a person skilled in the art. In the context of a numerical value or range described herein, the term may mean ±20%, ±15%, ±10%, ±9%, ±8%, ±7%, ±6%, ±5%, ±4%, ±3%, ±2%, or ±1% of a numerical value or range recited or claimed in one embodiment.

As used herein, the term "aptamer" refers to an oligonucleotide that has specific binding affinity for only one target.

As used herein, the terms "SELEX", "SELEX technology", "SELEX method", "SELEX process" may be used interchangeably with each other, and may refer to a combined method of a process of selecting a nucleic acid interacting with a target in a preferred manner (e.g., binding to a protein) and a process of amplifying the selected nucleic acid. This SELEX method is an in vitro method for selecting a nucleic acid molecule capable of binding to a target molecule with high specificity, which is disclosed in U.S. Patent No. 5,475,096 ("Nucleic Acid Ligands") and U.S. Patent No. 5,270,163 (WO 91/19813, "Nucleic Acid Ligands").

### 1. Novel nucleic acid ligand

The present disclosure in one embodiment relates to a novel non-natural nucleic acid ligand, which is a new class of nucleic acid compound that was previously considered impossible to exist.

As used herein, the terms "novel nucleic acid ligand", "nucleic acid ligand", "non-natural nucleic acid ligand", and "novel non-natural nucleic acid ligand" may be used interchangeably with one another. These terms may refer to a nucleic acid molecule having specific binding affinities for two or more different targets.

As used herein, the term "non-natural" refers to non-occurring in nature, and in this aspect, the novel nucleic acid ligand according to one embodiment of the present disclosure may not be a nucleic acid with a known physiological function binding to a target.

In one embodiment, the novel nucleic acid ligand may have specific binding affinities for two or more different targets. For example, the novel nucleic acid ligand may have specific binding affinities for two, three, four, five, six, or more targets.

In one embodiment, the novel nucleic acid ligand may have specific binding affinities for two different targets.

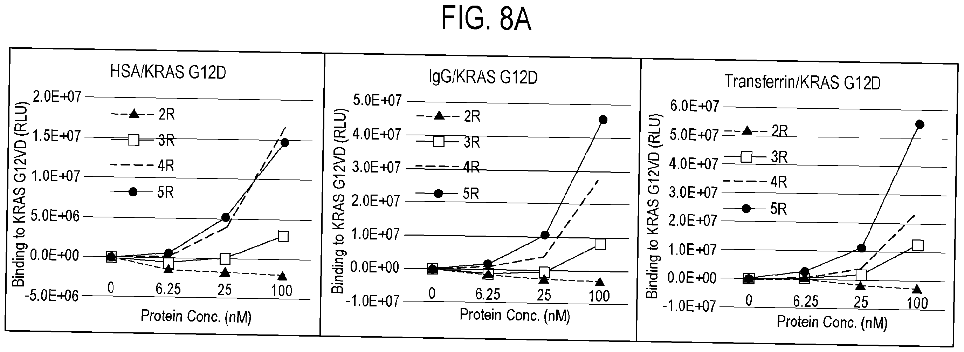

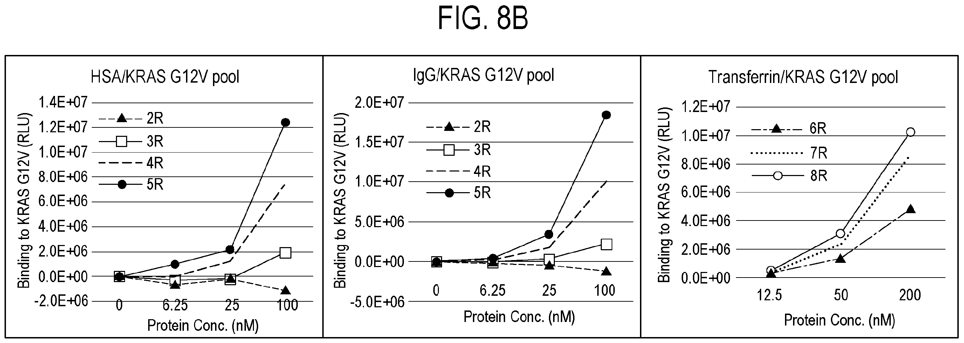

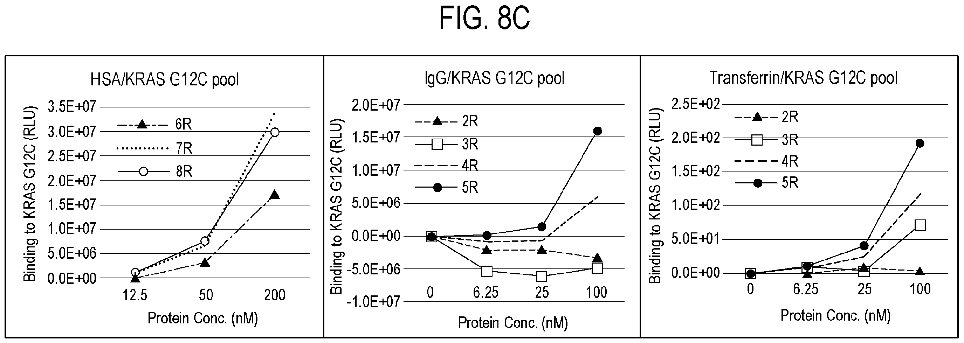

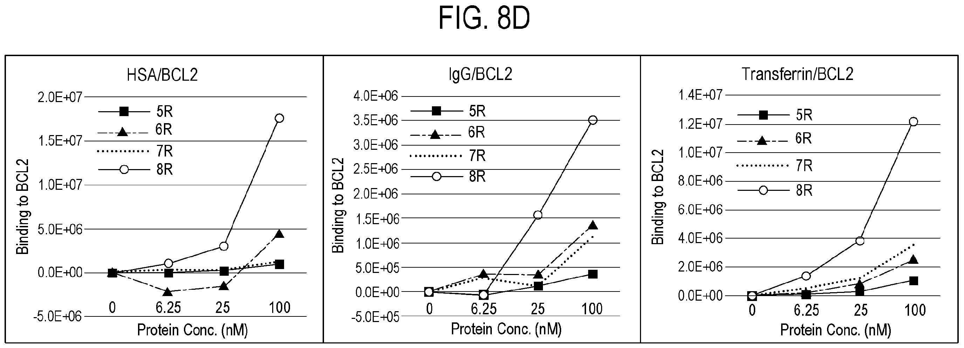

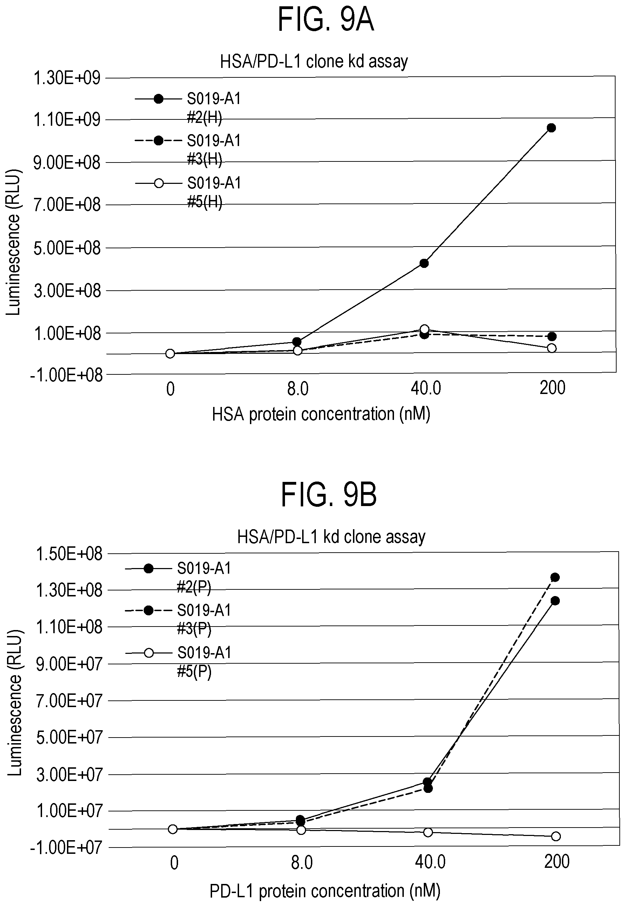

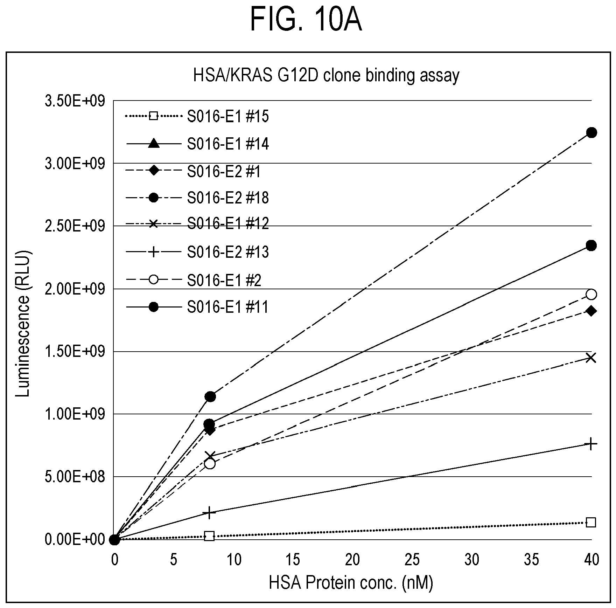

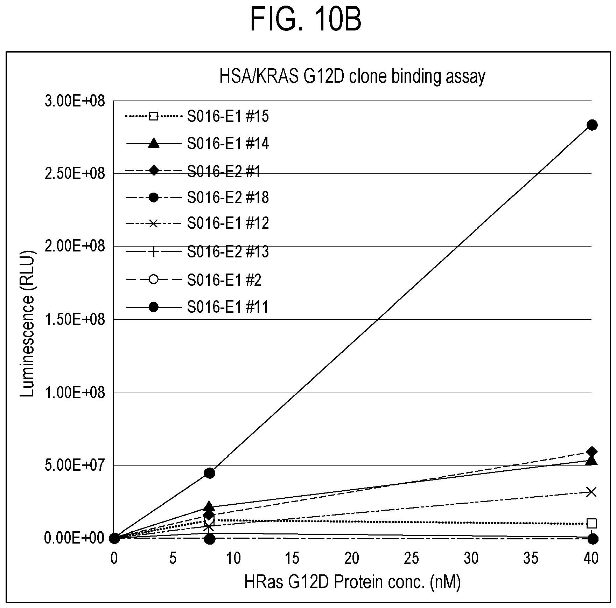

As used herein, the term "specific binding affinity" may mean that the nucleic acid ligand binds to its target with generally much higher affinity than binding to other non-target components or ingredients present in a mixture or sample. In one embodiment, a plurality of nucleic acid ligand candidate sequences may be selected from a nucleic acid library or a nucleic acid mixture having specific binding affinities for two or more different targets (see FIGS. 8A to 8D), and the novel nucleic acid ligand according to one embodiment may be selected by confirmation to have specific binding affinities for two or more different targets from among the selected plurality of nucleic acid ligand candidate sequences (see FIGS. 9A, 9B, 10A, and FIG. 10B).

In one embodiment, the targets may have a three-dimensional structure. In one embodiment, the targets do not include a polynucleotide that binds to a nucleic acid through a mechanism which predominantly depends on Watson/Crick base pairing or triple helix binding.

In one embodiment, the novel nucleic acid ligand may interact with the targets by three-dimensionally binding to the targets, recognizing three-dimensional structures of the targets, or forming three-dimensional structures by itself. The novel nucleic acid ligand may regulate characteristics of respective targets by binding to the targets, recognizing three-dimensional structures of the targets, or interacting with the targets. For example, a novel nucleic acid ligand according to an example can bind to a target protein to inhibit the activity of a protein, block signaling pathways, or inhibit activity of proteins downstream of signaling pathways.

As used herein, the term "binding" or "interacting" may refer to the association between two molecules (e.g., a stable association between a nucleic acid ligand and a target) due to, for example, pi-stacking, electrostatic interactions, hydrophobic interactions, ionic interactions, and/or hydrogen bonding interactions, under physiological conditions.

As used herein, the term "regulating" may refer to changing functional characteristics, biological activity, and/or biological mechanisms (or pathways) of targets. For example, the term may refer to upregulating (e.g., activating or stimulating), downregulating (e.g., inhibiting or suppressing), or changing characteristics, activity, and/or mechanisms of targets. For example, when the target is a cell, the characteristics of the cell to be regulated may be cell viability, cell proliferation, gene expression, cell morphology, cell activation, phosphorylation, calcium mobilization, degranulation, cell migration, and/or cell differentiation.

In one embodiment, the novel nucleic acid ligand is neither a conventional aptamer that binds to only one specific target, nor a bi-specific aptamer obtained by coupling or connecting a plurality of different aptamers to each other.

In one embodiment, the novel nucleic acid ligand is not in the form of a coupled body of a plurality of aptamers (non-coupling).

As used herein, the term "coupled body" may refer to a body obtained by coupling, fusing, linking, or conjugating two or more separate aptamers. For example, the coupled body may mean a dimer, a trimer, a tetramer, or a higher-order multimer of any monomeric aptamers, or may mean a bi-specific aptamer obtained by coupling or linking two or more different aptamers, or may mean a complex/conjugate/linkage composed of a plurality of aptamers. The coupled body may or may not contain a linker.

In one embodiment, in the novel nucleic acid ligand, all or a part of a nucleic acid sequence forming a binding site to one target may form all or a part of a nucleic acid sequence forming a binding site to another target. In one embodiment, the nucleic acid sequence forming a binding site to a target may include a sequence of a 5' or 3' primer for amplification of a nucleic acid ligand or a part of the sequence.

In one embodiment, the part of the nucleic acid sequence forming a binding site to a target may be about 1% to about 99%, about 5% to about 95%, about 10% to about 90%, about 20% to about 80%, about 30% to about 70%, or about 40% to about 60% of the entire sequence of the nucleic acid ligand, but is not limited thereto. In one embodiment, the part of the nucleic acid sequence forming a binding site to a target may contain one or more nucleotides constituting the nucleic acid ligand, but is not limited thereto.

In one embodiment, the novel nucleic acid ligand may be a nucleic acid ligand in which binding sites to two or more targets are not present separately from each other. For example, a nucleic acid ligand in which the binding sites to targets are present separately from each other may mean an aptamer multimer, a bi-specific aptamer, or a complex/conjugate/linkage composed of a plurality of aptamers.

In one embodiment, in the novel nucleic acid ligand, binding sites to two or more targets may be formed in one single nucleic acid ligand or from one single nucleic acid ligand. This may mean that tertiary structures of the nucleic acid ligand, which are formed to bind to the targets, are not formed from separate sequences, respectively, or formed by coupling ones formed from separate sequences, but formed from one nucleic acid ligand or the sequence of a single nucleic acid ligand. Therefore, the novel nucleic acid ligand corresponds to a substance that is different or distinguished from a bi-specific aptamer.

In one embodiment, the novel nucleic acid ligand may consist of about 100 or less nucleotides. Specifically, the novel nucleic acid ligand may consist of 100 or less, 90 or less, 80 or less, 70 or less, 60 or less, 50 or less, or 40 or less nucleotides. In one embodiment, the novel nucleic acid ligand may consist of 10 to 105, 15 to 100, 20 to 95, 25 to 90, 30 to 85, 35 to 80, 40 to 75, 45 to 70, 50 to 65, or 55 to 60 nucleotides.

In one embodiment, the nucleotides may be selected from the group consisting of DNA nucleotides, RNA nucleotides, modified nucleotides thereof, and combinations thereof.

As used herein, the term "modified nucleotide" may refer to an analog, substituent, or ester of a naturally occurring nucleotide (A, G, T/U, and C), and may mean a broad concept encompassing modified nucleotides easily available by a person skilled in the art. For example, the modified nucleotide may refer to a nucleotide having a substitution at the C-5 position (e.g., a C-5 modified pyrimidine), a nucleotide having a modified sugar (ribose or deoxyribose) constituting the nucleotide, or a nucleotide having all of such modifications.

In one embodiment, the C-5 modified pyrimidine may refer to a C-5 modified amino carbonyl pyrimidine, and examples thereof may include 5-(N-benzylcarboxyamide)-2'-deoxyuridine (Bz-dU), 5-(N-isobutylcarboxyamide)-2'-deoxyuridine (iBu-dU), 5-(N-phenethylcarboxyamide)-2'-deoxyuridine (Pe-dU), 5- (N-thiophenylmethylcarboxyamide)-2'-deoxyuridine (Th-dU), 5-(N-[2-(1H-indol-3yl)ethyl]carboxyamide)-2'-deoxyuridine (Trp-dU), 5-(N-[1-(3-trimethylammonium)propyl]carboxyamide)-2'-deoxyuridine chloride, 5-(N-naphthylmethylcarboxyamide)-2'-deoxyuridine (Nap-dU), 5-(N-[1-(2,3-dihydroxypropyl)]carboxyamide)-2'-deoxyuridine), 5-(N-benzylcarboxyamide)-2'-deoxycytidine (Bz-dC), 5-(N-isobutylcarboxyamide)-2'-deoxycytidine (iBu-dC), 5-(N-phenylcarboxyamide)-2'-deoxycytidine (Pe-dC), 5-(N-thiophenylmethylcarboxyamide)-2'-deoxycytidine (Th-dC), 5-(N - [2-(1H-indol-3yl)ethyl]carboxyamide)-2'-deoxycytidine (Trp-dC), 5-(N-[1-(3-trimethylammonium)propyl]carboxyamide)-2'-deoxycytidine chloride, 5-(N-naphthylmethylcarboxyamide)-2'-deoxycytidine (Nap-dC), and/or 5-(N-[1-(2,3-dihydroxypropyl)]carboxyamide)-2'-deoxycytidine, but is not limited thereto.

In one embodiment, a modified nucleotide having a 2'-position modification of the ribose sugar may include 2'-deoxy-2'-fluorouridine (U_{f}), 2'-deoxy-2'-fluorocytidine (C_{f}), 2'-hydroxyadenosine (Aᵣ), 2'-methoxyadenosine (Aₘ), and 2'-methoxyguanosine (Gₘ), but is not limited thereto.

In one embodiment, a modified nucleotide having both a C-5 modification and a 2' position modification of the ribose sugar may include 5-(N-benzylcarboxyamide)-2'-O-methyluridine, 5-(N-benzylcarboxyamide)-2'-fluorouridine, 5-(N-isobutylcarboxyamide)-2'-O-methyluridine, 5-(N-isobutylcarboxyamide)-2'-fluorouridine, 5-(N-[2-(1H-indole-3yl)ethyl]carboxyamide)-2'-O-methyluridine, 5-(N-[2-(1H-indole-3yl)ethyl]carboxyamide)-2'-O-fluorouridine, 5-(N-naphthylmethylcarboxyamide)-2'-O-methyluridine, 5-(N-naphthylmethylcarboxyamide)-2'-fluorouridine, 5-(N-[1-(2,3-dihydroxypropyl)]carboxyamide)-2'-O-methyluridine, and/or 5-(N-[1-(2,3-dihydroxypropyl)]carboxyamide)-2'-O-fluorouridine, but is not limited thereto.

In one embodiment, the modified nucleotide having both a C-5 modification and a 2' position modification of the ribose sugar may include 5-(N-benzylcarboxyamide)-2'-O-methylcytidine, 5-(N-benzylcarboxyamide)-2'-fluorocytidine, 5-(N-isobutylcarboxyamide)-2' -O-methylcytidine, 5-(N-isobutylcarboxyamide)-2'-fluorocytidine, 5-(N-[2-(1H-indole-3yl)ethyl]carboxyamide)-2'-O-methylcytidine, 5-(N-[2-(1H-indole-3yl)ethyl]carboxyamide)-2'-O-fluorocytidine, 5-(N-naphthylmethylcarboxyamide)-2'-O-methylcytidine, 5-(N-naphthylmethylcarboxyamide)-2'-fluorocytidine, 5-(N-[1-(2,3-dihydroxypropyl)]carboxyamide)-2'-O-methylcytidine, and/or 5-(N-[1-(2,3-dihydroxypropyl)]carboxyamide)-2'-O-fluorocytidine, but is not limited thereto.

In one embodiment, the modified nucleotide may be 5-(N-benzylcarboxyamide)-2'-deoxyuridine (Bz-dU) or 5-(N-naphthylmethylcarboxyamide)-2'-deoxyuridine (Nap-dU), and therefore, at least one of the nucleotides constituting the novel nucleic acid ligand may be such a modified nucleotide.

In one embodiment, the novel nucleic acid ligand may consist of one nucleic acid sequence selected from the group consisting of SEQ ID NO: 6 to SEQ ID NO: 183.

In one embodiment, the novel nucleic acid ligand may be selected from the group consisting of single-stranded RNA, double-stranded RNA, single-stranded DNA, and double-stranded DNA.

In one embodiment, the novel nucleic acid ligand may be used by conjugating biotin in front of the 5'-end or the back of the 3'-end as needed.

In one embodiment, the novel nucleic acid ligand may be used by conjugating a fluorescent substance (e.g., FAM, VIC, HEX, BHQ-1, Cy5, Cy3, rhodamine, Texas Red, etc.) in front of the 5'-end or the back of the 3'-end as needed.

The present disclosure in one embodiment may relate to a non-natural nucleic acid ligand prepared by the method including a method according to one embodiment.

### 2. Use of novel nucleic acid ligand

The present disclosure in one embodiment may relate to a pharmaceutical composition for prevention or treatment of cancer, the pharmaceutical composition containing the novel nucleic acid ligand according to one embodiment.

In a pharmaceutical composition according to an example of the present disclosure, a novel nucleic acid may be delivered into cancer cells having a therapeutic target via a pathway of endocytosis or the like. The novel nucleic acid ligand may bind to a plasma protein present in the body of a subject to which the pharmaceutical composition is administered. Therefore, the pharmaceutical composition containing the novel nucleic acid ligand can exhibit excellent in vivo stability and slow renal clearance (excellent residence time in the body), and the novel nucleic acid ligand can enter cancer cells via a pathway of endocytosis or the like after reaching the cancer tissue.

As used herein, the term "subj ect" refers to a mammal, such as a horse, sheep, a pig, a goat, or a dog, including a human, that has or may develop a disease or a disorder caused by a target or its action, and preferably refers to a human.

As used herein, the term "endocytosis" refers to a cellular action by which a cell transports a substance outside the cell membrane into the cell membrane. By endocytosis, a foreign substance is sent into the cell while enclosed in the cell membrane, and the absorbed substance is degraded by vesicle formation in the cell and then recycled or discharged to the outside. Endocytosis may be divided into macropinocytosis, receptor-mediated endocytosis involving clathrin protein, caveolae endocytosis, and the like, depending on the method of activation or the type of protein involved.

In one embodiment, the pharmaceutical composition may further contain a plasma protein. In such a case, the pharmaceutical composition may be in the form in which the plasma protein is bound to the novel nucleic acid ligand or the form of the novel nucleic acid ligand alone, and the plasma protein may be a protein that is present outside the body of a subject to be treated.

In a pharmaceutical composition according to an example of the present disclosure, a novel nucleic acid ligand may be delivered into cancer cells having a therapeutic target via a pathway of macropinocytosis or the like.

As used herein, the term "macropinocytosis" is one type of endocytosis, wherein a pocket may be formed while the cellular membrane protrudes out of a cell and then contracts again by the cytoskeletal protein actin and the formed pocket may be separated while entering the cell, thereby forming vesicles (macropinosomes) with a diameter of about 0.2 µm to 5 µm.

Macropinocytosis plays an important role in tumor metabolism, which recently attracts attention. Through macropinocytosis, nutrients (e.g., glutamine, albumin, etc.) necessary for rapid growth of tumor cells may be supplied into cancer cells and used for metabolism. That is, extracellular fluid, extracellular molecules, and the like may be contained inside the vesicles formed by macropinocytosis. Macropinocytosis may be activated by mutation of a tumor gene or protein of cancer cells. For example, the tumor gene may be SRC gene or KRAS gene. For example, macropinocytosis may be activated by mutation of KRAS gene or protein of cancer cells. The mutation of KRAS protein may be caused by mutations of the 12th, 13th, and 61th amino acids of the wild-type KRAS protein, and for example, may be KRAS G12D, KRAS G12V, KRAS G12C, KRAS G12A, KRAS G12S, KRAS G12R, KRAS G13D, or KRAS Q61H.

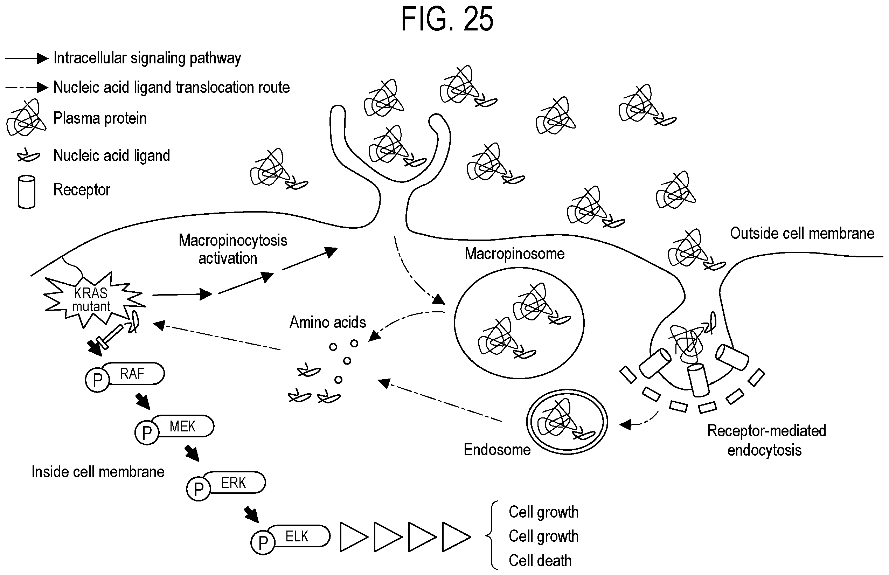

In one embodiment, the efficiency of delivery of the nucleic acid ligand into the cell by macropinocytosis can be increased by binding of the nucleic acid ligand and the plasma protein. That is, the nucleic acid ligand binding to the plasma protein may be excellent in the delivery into cells compared with nucleic acid ligands not binding to the plasma protein (see FIG. 25).

As shown in FIG. 25, a nucleic acid ligand according to one embodiment of the present disclosure can be delivered into the cytoplasm through various mechanisms of endocytosis. Macropinocytosis, which has attracted attention as a main pathway for supplying essential nutrients necessary for rapid proliferation of cancer cells, as well as other endocytosis pathways may be involved in the delivery of the nucleic acid ligand. The nucleic acid ligand delivered into the cytoplasm can inhibit the proliferation of cancer cells by binding to a therapeutic target protein to disturb signaling pathways.

In one embodiment, the nucleic acid ligand delivered into cells can bind to a KRAS mutant protein (e.g., KRAS G12D) inside cancer cells to inhibit the phosphorylation of proteins (ERK, etc.) downstream of the KRAS signaling pathways, thereby inhibiting excessive cell growth, proliferation, division, and the like. Therefore, the nucleic acid ligand according to the embodiment of the present disclosure can an effect of preventing or treating KRAS mutant protein-expressing cancer (e.g., lung cancer, colorectal cancer, pancreatic cancer, breast cancer, ovarian cancer, endometrial cancer, cervical cancer, bladder cancer, gallbladder biliary tract cancer, skin cancer, stomach cancer, brain cancer, kidney cancer, acute myeloid leukemia, etc.).

As used herein, the term "treatment" may refer to any action that alleviates or advantageously changes symptoms of a subject with diseases.

As used herein, the term "prevention" may refer to any action that inhibits or delays the disease.

In one embodiment, the novel nucleic acid ligand may be administered at a pharmaceutically effective amount.

In one embodiment, the novel nucleic acid ligand may be administered at a dose of about 1 µg to about 100 µg, about 5 µg to about 80 µg, about 10 µg to about 70 µg, about 15 µg to about 60 µg, about 20 µg to about 50 µg, or about 30 µg to about 40µg, and the administration may be performed once a day or divided into several times.

As used herein, the term "administration" refers to the introduction of the pharmaceutical composition according to one embodiment to a subject by any suitable method. The route of administration may be oral or parenteral through any general route as long as the pharmaceutical composition can reach a target tissue.

As used herein, the term "pharmaceutically effective amount" refers to an amount sufficient to treat a disease at a reasonable benefit/risk ratio applicable to medical treatment, and the level of the effective dose may be determined depending on factors including the patient's body weight, sex, age, health condition, severity, or indication, activity of a drug, sensitivity to a drug, time of administration, route of administration, excretion rate, duration of treatment, and drugs that are simultaneously used, and other factors well known in the medical field.

In one embodiment, the cancer may be a solid cancer.

In one embodiment, the cancer may express PD-L1 protein on the surface of cancer cells. Specifically, the cancer may be at least one selected from the group consisting of brain cancer, lung cancer, colorectal cancer, small intestine cancer, stomach cancer, testicular cancer, thyroid cancer, cervical cancer, skin cancer, bladder cancer, ovarian cancer, kidney cancer, liver cancer, pancreatic cancer, urothelial cell cancer, and breast cancer.

In one embodiment, the pharmaceutical composition may be an immune anticancer agent.

In one embodiment, the novel nucleic acid ligand contained in the immune anticancer agent can exhibit an anticancer immune effect by binding to CD3d/e or 4-1BB on the surface of cytotoxic T cells to activate cytotoxic T cells.

In one embodiment, the cancer may be expressed by BCL-2 protein.

In one embodiment, the cancer may be expressed by a KRAS mutant protein. Specifically, the cancer may be at least one selected from the group consisting of lung cancer, colorectal cancer, pancreatic cancer, breast cancer, ovarian cancer, endometrial cancer, cervical cancer, bladder cancer, gallbladder cancer, biliary tract cancer, skin cancer, stomach cancer, brain cancer, kidney cancer, and acute myeloid leukemia.

In one embodiment, the pharmaceutical composition for prevention or treatment of cancer can inhibit the above-described division or proliferation of cancer cells or induce death of cancer cells.

In one embodiment, the pharmaceutical composition for prevention or treatment of cancer may contain as an active ingredient a novel nucleic acid ligand consisting of one nucleic acid sequence selected from the group consisting of SEQ ID NO: 24, SEQ ID NO: 27, SEQ ID NO: 30, SEQ ID NO: 84, and SEQ ID NO: 100.

In one embodiment, the pharmaceutical composition may be administered orally or parenterally depending on a desired method, and examples of parenteral administration routes may include intravenous, intradermal, intrathoracic, intramuscular, subcutaneous, topical, transdermal, transmucosal, intraperitoneal, and rectal administration.

In one embodiment, the pharmaceutical composition may be administered by inhalation. The administration by inhalation may be an administration using a pharmaceutical preparation that contains respirable particles or droplets containing the nucleic acid ligand and can be inhaled through the respiratory tract, nasal passages, or the like, but is not limited thereto. For example, a dry powder inhaler device (DPI), a pressurized metered dose inhaler (pMDI), or the like may be used for the administration by inhalation, but is not limited thereto.

In one embodiment, the pharmaceutical composition may be formulated for oral or parenteral use. For example, the pharmaceutical compositions may be formulated into solid preparations for oral administration (e.g., a tablet, a pill, a powder, granules, a capsules, etc.), liquid preparations for oral administration (e.g., a suspension, a liquid preparation for internal use, an emulsion, a liposome formulations, a syrups, etc.), parenteral preparations (e.g., an injection, a non-aqueous solution, a suspension, an emulsion, a nasal spray, a transdermal absorption formulation, a lyophilized preparation, a suppository, etc.), or the like.

In one embodiment, the pharmaceutical composition may further contain a pharmaceutically acceptable salt, diluent, excipient, carrier, buffer, adjuvant, or surfactant that can be typically used in pharmaceutical compositions at a level that does not affect the action of the novel nucleic acid ligand, but is not limited thereto. These ingredients may be helpful in the formulation of pharmaceutical compositions.

As used herein, the term "pharmaceutically acceptable" may mean that a certain substance is non-toxic and does not interact with the action of the active ingredient of the pharmaceutical composition, and for example, may mean those approved by the management agency of each country or listed in the pharmacopoeia of each country. As used herein, the term "pharmaceutically acceptable salt" may refer to a product that contains an ionic bond and is conventionally produced by reacting a compound with an acid or base suitable for administration of a compound to a subject.

In one embodiment, examples of the adjuvant may include citric acid, hydrochloric acid, tartaric acid, stearic acid, polyethylene glycol, polypropylene glycol, ethanol, sodium bicarbonate, distilled water, hyaluronic acid, cationic lipids such as Lipofectamine, starch, gelatin, talc, ascorbic acid, olive oil, palm oil, methyl cellulose, titanium oxide, sodium-carboxymethyl cellulose, a sweetener, a preservative, and the like, but are not limited thereto.

The present disclosure in one embodiment may relate to a method for prevention or treatment of cancer, the method including administering the novel nucleic acid ligand according to one embodiment to a subject in needed thereof at a pharmaceutically acceptable amount.

The present disclosure in one embodiment may relate to use of the novel nucleic acid ligand according to one embodiment in the manufacture of a medicine for preventing or treating cancer.

The present disclosure in one embodiment may relate to a novel nucleic acid ligand according to one embodiment for use in the prevention or treatment of cancer.

The present disclosure in one embodiment may relate to a composition for cancer diagnosis, the composition containing the novel nucleic acid ligand according to one embodiment.

The present disclosure in one embodiment may relate to a kit for cancer diagnosis, the kit containing the novel nucleic acid ligand according to one embodiment.

The present disclosure in one embodiment may relate to a method for diagnosing cancer, the method including administering the novel nucleic acid ligand according to one embodiment to a subject in need thereof.

The present disclosure in one embodiment may relate to use of the novel nucleic acid ligand according to one embodiment in the manufacture of a medicine for diagnosing cancer.

The present disclosure in one embodiment may relate to a novel nucleic acid ligand according to one embodiment for use in the diagnosis of cancer.

The present disclosure in one embodiment may relate to a diagnostic composition containing the novel nucleic acid ligand according to one embodiment.

In the present disclosure in one embodiment, the novel nucleic acid ligand according to one embodiment can be used in the detection of diagnostic reagents, analytical reagents, harmful substances, and the like. The present disclosure in one embodiment may relate a method for detecting a target in a sample by using the novel nucleic acid ligand according to one embodiment, the method including (a) contacting the target from the sample with the novel nucleic acid ligand according to the embodiment. In one embodiment, the detection method may further include, after step (a), (b) determining whether the binding of the target and the nucleic acid ligand is formed, to detect the presence or absence of the target in the sample.

The present disclosure in one embodiment may relate to a contrast medium or diagnostic radiopharmaceutical containing a novel nucleic acid ligand according to one example. The novel nucleic acid ligand may bind to a substance that is commonly used for acting as a contrast medium or a diagnostic radiopharmaceutical, and for example, may bind to a radioactive isotope.

The description of the pharmaceutical compositions, uses, and treatment methods of the present disclosure may be equally applied unless they are not consistent with one another.

### 3. Novel screening method for selecting novel nucleic acid ligands

The present disclosure in one embodiment may relate to a method for identifying a novel non-natural nucleic acid ligand having specific binding affinities for two or more different targets.

In one embodiment, the method may include contacting one or more nucleic acid ligand candidate sequences with each of two or more different targets in a sequential manner. The step may include identifying one or more nucleic acid ligand candidate sequences having specific binding affinities for the targets.

In one embodiment, the method may include: (a) contacting one or more first nucleic acid ligand candidate sequences with a target and identifying one or more second nucleic acid ligand candidate sequences specifically binding to the target; and (b) contacting (i) the one or more second nucleic acid ligand candidate sequences obtained in step (a) or (ii) one or more third nucleic acid ligand candidate sequences prepared using the one or more second nucleic acid ligand candidate sequences obtained in step (a) with a target different from the target in step (a) and identifying one or more fourth nucleic acid ligand candidate sequences specifically binding to the different target.

In one embodiment, the method may further include, after the step of conducting a contact with each target, separating sequences specifically binding to the target. The sequences thus separated may be identified as sequences specifically binding to the target by passing through a step of evaluating specific binding affinity for the target.

In one embodiment, the nucleic acid ligand candidate sequences may refer to nucleic acid molecules included in nucleic acid libraries or a nucleic acid molecule mixture.

In one embodiment, the nucleic acid ligand candidate sequences may refer to consecutive nucleotide molecules consisting of DNA nucleotides, RNA nucleotides, modified nucleotides thereof, and combinations thereof.

In one embodiment, the first nucleic acid ligand candidate sequences may consist of a 5'-primer binding sequence, a random sequence, and a 3'-primer binding sequence. The 5'-primer binding sequence or the 3'-primer binding sequence may be appropriately selected depending on the type of nucleic acid ligand or target to be selected, and is not limited to the primer binding sequences or primer sequences described in the present disclosure. For example, the first nucleic acid ligand candidate sequences may consist of the nucleic acid sequence of SEQ ID NO: 1 (GCTGGTGGTGTGGCTG-N(40)-CAGGCAGACGGTCACTCA).

In one embodiment, the number of first nucleic acid ligand candidate sequences may be 10¹⁴ to 10³⁰, 10¹⁶ to 10²⁸, 10²⁰ to 10²⁶, or 10²² to 10²⁵. The first nucleic acid ligand candidate sequences may be included in the SELEX nucleic acid library pool.

In one embodiment, the nucleic acid ligand candidate sequences obtained in step (a) may be used intact as the second nucleic acid ligand candidate sequences.

In one embodiment, the third nucleic acid ligand candidate sequences may be obtained by linking a random sequence consisting of a plurality of nucleotides to at least one of both ends of the second nucleic acid ligand candidate sequences. The random sequence linked to the second nucleic acid ligand candidate sequences may consist of about 10 to about 50, about 15 to about 45, about 20 to about 40, or about 25 to about 35 consecutive nucleotides.

In one embodiment, step (b) may further include linking a random sequence consisting of a plurality of nucleotides to at least one of both ends of the one or more second nucleic acid ligand candidate sequences obtained in step (a) to prepare one or more third nucleic acid ligand candidate sequences. In one embodiment, the random sequence linked to the second nucleic acid ligand candidate sequences may consist of about 10 to about 50, about 15 to about 45, about 20 to about 40, or about 25 to about 35 consecutive nucleotides.

In one embodiment, the random sequence may be linked to any one of both ends of the second nucleic acid ligand candidate sequences, and in the preparation of the third nucleic acid ligand candidate sequences, the second nucleic acid ligand candidate sequence or a part thereof may be used as a primer sequence for nucleic acid amplification. Through this, excessive lengthening of the nucleic acid ligand candidate sequences can be prevented, and novel nucleic acid ligands capable of achieving the purposes and effects of the present disclosure can be obtained.

As used herein, the term "random sequence" or "any sequence" may be used interchangeably with each other, and may refer to a sequence prepared such that a nucleic acid sequence with a desired length is randomly constructed.

In one embodiment, the third nucleic acid ligand candidate sequences may consist of about 40 to about 100, about 50 to about 90, about 55 to about 85, about 60 to about 80, or about 65 to about 75 consecutive nucleotides.

In one embodiment, the method may be performed by systematic evolution of ligands by exponential enrichment (SELEX).

The present disclosure in one embodiment may relate to a method for identifying a non-natural nucleic acid ligand having specific binding affinities for two or more different targets, the nucleic acid ligand being identified by systematic evolution of ligands by exponential enrichment (SELEX) starting from one nucleic acid library, the method may include: selecting one target from the group consisting of two or more different targets each having a three-dimensional structure in each SELEX round; and selecting a nucleic acid ligand having specific binding affinity for the selected target in each SELEX round.

In one embodiment, after SELEX performed in one or more rounds for one target is completed, SELEX performed in one or more rounds for another target may be performed. In such a case, SELEX for one target may be performed in about 4 rounds to about 10 rounds or about 5 rounds to about 8 rounds, but is not limited thereto.

In one embodiment, the method may include: (i) performing one or more SELEX rounds on one target; (ii) performing one or more SELEX rounds on a target different from the target in step (i) by using a nucleic acid library obtained in step (i); (iii) performing step (i) again by using a nucleic acid library obtained in step (ii); and (iv) repeating steps (i) to (iii). In such a case, SELEX for one target may be performed in 1 round, 2 rounds, 3 rounds, 4 rounds, 5 rounds, 6 rounds, 7 rounds, 8 rounds, 9 rounds, 10 rounds, or more.

The method according to one embodiment may further include a negative selection step (e.g., negative SELEX) or a counter selection step (e.g., counter SELEX) to select sequences having excellent binding affinity for one trait of a target. In one embodiment, the negative selection step is a method of removing non-specific binding for a target, and the counter selection step is a method of increasing specificity for a target through binding to another trait (e.g., a mutant) of the target but not the target.

In one embodiment, in the counter selection step, a sequence binding specifically to one trait of the target may be separately selected from another trait of the target.

For example, in the counter selection step, in order to separate sequences specifically binding to a KRAS mutant protein, sequences having affinity for the wild-type KRAS protein are first separately removed from library sequences, and sequences specifically binding to only the KRAS mutant protein (e.g., KRAS G12D) as a target can be obtained by using the remaining library sequences.

In the counter selection step, sequences specifically binding to one protein for a therapeutic target may be separately selected from similar family proteins including the therapeutic target. For example, in the counter selection step, in order to separate sequences specifically binding to BCL2 protein, sequences having affinity for BCL2L1, which is a family protein having a similar structure, is first separately removed from library sequences, and sequences specifically binding to only the BCL2 protein as a target can be obtained by using the remaining library sequences.

The method according to one embodiment may further include performing negative SELEX or counter SELEX on another trait of the target.

In one embodiment, the SELEX may be at least one selected from the group consisting of classic SELEX, Advanced SELEX, IP-SELEX, Capture-SELEX, Cell-SELEX, CE-SELEX, M-SELEX, AFM-SELEX, AEGIS-SELEX, and Animal-SELEX, but is not limited thereto.

The method according to one embodiment may further include evaluating specific binding affinity of the identified nucleic acid ligand candidate sequences for target. For example, the step of evaluating specific binding affinity may be performed by at least one of bead-based ELISA binding assay, filter binding assay, evaluation of Dnase stability against target, ELISA-based determination of binding affinity (K_{d}), and evaluation of stability or half-life in serum, but is not limited thereto.

In a method according to one embodiment, the target may correspond to two different targets.

The method according to one embodiment may include removing nucleotides not involved in specific binding affinity for target from the identified nucleic acid ligand candidate sequences.

The present disclosure in one embodiment may relate to a method for identifying a non-natural nucleic acid ligand having specific binding affinities for two or more different targets, the method including performing systematic evolution of ligands by exponential enrichment (SELEX) on each of two or more different targets in a sequential manner, wherein the SELEX in each case is performed to identify one or more nucleic acid ligands having specific binding affinity for each target.

In one embodiment, the method may be a method for identifying a non-natural nucleic acid ligand, the method including: (a) performing SELEX on one target; and (b) performing SELEX on a target different from the target in step (a) by using a nucleic acid library obtained in step (a), wherein the targets are two different targets having a three-dimensional structure and SELEX in each case is performed to select a nucleic acid ligand having specific binding affinity for each target.

In one embodiment, the method may be a method for identifying a non-natural nucleic acid ligand, the method including: (a) contacting a first nucleic acid library with one target to primarily select a plurality of nucleic acid candidate sequences binding to the target; and (b) contacting (i) the plurality of primarily selected nucleic acid ligand candidate sequences with another target to secondarily select a plurality of nucleic acid candidate sequences binding to the another target, wherein the targets are two or more different targets.

The present disclosure in one embodiment may relate to a method for detecting a target in a sample, the method including contacting a target from the sample with the nucleic acid ligand according to one embodiment.

### 4. Targets

As used herein, the term "target" may refer to any compound on which a nucleic acid can act in a preferable manner. For example, the target may include a protein, peptide, nucleic acid, carbohydrate, lipid, polysaccharide, glycoprotein, hormone, receptor, antigen, antibody, virus, pathogen, toxic substance, substrate, metabolite, transition state analog, cofactor, inhibitor, drug, dye, nutrient, growth factor, cell, tissue, and/or a fragment, component, or part of any of the foregoing, but is not limited thereto. The target may be modified within a range that does not substantially change its identity. For example, if the target is a protein, the modification may include a minor modification or alternation in an amino acid sequence, disulfide bond formation, glycosylation, lipidation, acetylation, phosphorylation, or any other manipulation or alteration (e.g., conjugation with an indication component that does not substantially change the nature of the target).

In one embodiment, the targets may have a three-dimensional structure. The three-dimensional structure may refer to a three-dimensional biological structure.

In one embodiment, the targets may be selected from the group consisting of cells, viruses, and proteins.

In one embodiment, the cells may be prokaryotic cells (e.g., bacteria), eukaryotic cells (e.g., mammalian cells), parasitic cells (e.g., malaria cells, Leishmania cells, Cryptosporidium cells, or amoeba cells), or fungal cells, but are not limited thereto.

In one embodiment, the cells may be cancer cells. Such cells may be originated from any cancerous or precancerous tumor.

In one embodiment, one of the targets may be a plasma protein and the other may be a therapeutic target. The therapeutic target may be a therapeutic target protein.

In one embodiment, the plasma protein is not limited as long as the protein is present in the plasma of an organism, and for example, the plasma protein may be selected from the group consisting of albumin, alpha1 globulin, alpha2 globulin, beta globulin, gamma globulin, immunoglobulin A, immunoglobulin G, lipoproteins, fibrinogen, transferrin, and transthyretin, but is not limited thereto.

The novel nucleic acid ligand according to one embodiment of the present disclosure, by binding to a plasma protein, can exhibit excellent in vivo stability and slow renal clearance (that is, increased residence time in the body), and additionally, may be delivered into cells via a pathway of endocytosis or the like. In one embodiment, the novel nucleic acid ligand specifically binds to a plasma protein to exhibit resistance to a nuclease such as Dnase in serum conditions and thus can show an excellent effect on in vivo stability and half-life. Furthermore, the novel nucleic acid ligand specifically binds to a plasma protein to delay renal filtration and thus can reduce the clearance rate and increase the retention time in the body. Furthermore, the novel nucleic acid ligand specifically binds to a plasma protein to enter the cells, for example, cancer cells and thus can exhibit an excellent therapeutic effect.

As used herein, the term "therapeutic target" refers to a target for treating a disease or disorder, and the type of therapeutic target is not limited as long as the therapeutic target can exhibit a therapeutic effect by interacting with the novel nucleic acid ligand according to one embodiment of the present disclosure. For example, the therapeutic target may be a protein that is involved in intracellular signaling.

In one embodiment, the therapeutic target may be a protein present in cells, a protein present in the cell membrane, or mutants thereof.

In one embodiment, the novel nucleic acid ligand, after specifically binding to a plasma protein, can reach a target tissue while avoiding the degradation by nucleases in the body or renal excretion, and thereafter may enter the target cell via a pathway of endocytosis or the like and bind to a therapeutic target (e.g., a protein in the cell) present in the cell, thereby exhibiting an intended therapeutic effect. The novel nucleic acid ligand entering the cell can be separated from the plasma protein by intracellular digestion and thereafter can exhibit an intended therapeutic effect by binding to the therapeutic target.

In one embodiment, the novel nucleic acid ligand, after specifically binding to a plasma protein, can reach a target tissue while avoiding the degradation by nucleases in the body or renal excretion, and thereafter may bind to or interact with a therapeutic target (e.g., cell membrane protein) present in the cell membrane of the target tissue, or may be dissociated from the plasma protein to bind to or interact with a therapeutic target present in the cell membrane.

In one embodiment, the therapeutic target may be selected from the group consisting of membrane proteins, transmembrane proteins, glycoproteins, immune antibodies, viruses, viral envelope glycoproteins, viral enzymes, secretory proteins, serine proteases, peptide hormones, neurotransmitters, hormones, dehydrogenases, cytokines, E3 ubiquitin ligases, neuropeptides, hydrolases, serine protease inhibitors, phosphatase, chemokine proteins, methyl transferases, oxidases, growth factors, bacteria, bacterial proteins, intracellular proteins, extracellular matrices, receptors, transcription factors, and tumor proteins, but is not limited thereto.

In one embodiment, the therapeutic target may be selected from the group consisting of 4-1BB, acetylcholine receptors, alpha thrombin, amylin, angiopoietin 1, angiopoietin 2, AXL, BCL-2, BMPR-1R, BRD1, BRD2, BRD3, BRD4, BRDT, BTLA, calcitonin gene-related peptides, CREB-binding protein (CPB), CCK4/PTK7, CD16a, CD16b, CD19, CD20, CD200, CD200R, CD27, CD28, CD3, CD30, CD32A, CD32B, CD33, CD4, CD40L, CD52, CD80, CD94, CSF1R, CTLA-4, DDR1, DDR2, E2F1, EGFR, EPH, ERBB2, FGF, FGFR, ghrelin, GITR, glypican 3, gonadotropin-releasing hormone 1, HIV gp120, HIV-1 integrase, HIV-1 reverse transcriptase, HRAS, HVEM, ICOS, IDH1, IGF1R, immunoglobulin E, interferon-gamma, KIT, KRAS, LAG3, LFA, L-selectin, LTK, MDM2, MDMX, mucin 1, MYC, neurotensin 1, neutrophil elastase, NF-Kb, NKG2D, nociceptin, NTRK1, NTRK2, OX-40, PD-1, PDGF, PDGFRfamily, PD-L1, PD-L2, phospholipase A2, plasminogen activation inhibitor 1, PP2A, PPM1D, PPP2CA, protein tyrosine phosphatase, P-selectin, PSMA, respiratory syncytial virus, RET, SDF1b, SetDB1, SLAMF7, Staphylococcus enterotoxin B, STAT3, tenascin, TGFBR, TIGIT, TIM3, TNFSF7, tyrosinase, VCAM-1, VEGF, VEGFR family, αᵥβ₃ integrin, and mutants thereof, but is not limited thereto.

In one embodiment, the therapeutic target may be at least one of proteins present in the cell or mutants thereof. For example, the therapeutic target present in the cell may be at least one selected from the group consisting of KRAS, BCL2, MYC, PP2A, BRD1, BRD2, BRD3, BRD4, BRDT, CBP, E2F1, MDM2, MDMX, PPP2CA, PPM1D, STAT3, and IDH1.

In one embodiment, the therapeutic target may be a KRAS mutant protein. The KRAS mutant may be caused by a mutation of the 12th, 13th, or 61st amino acid of the wild-type KRAS protein. For example, the KRAS mutant may be KRAS G12D, KRAS G12V, KRAS G12C, KRAS G12A, KRAS G12S, KRAS G12R, KRAS G13D, KRAS Q61H, or combinations thereof, and preferably KRAS G12D, KRAS G12C, KRAS G12V, or combinations thereof.

When a KRAS mutation occurs, the KRAS protein activated by a growth factor is not inactivated and always retains GTP in an activated state, and thus the signaling pathway is continuously stimulated. Therefore, cell division protein, cell growth proteins, cyclins, or CDK proteins may be continuously produced to continuously induce cell growth, proliferation, division, and the like, resulting in tumor formation.

In one embodiment, the novel nucleic acid ligand specifically or selectively binds to a KRAS mutant protein (e.g., KRAS G12D) inside the cancer cell to inhibit the phosphorylation of proteins (ERK, etc.) downstream of the KRAS signaling pathway, thereby inhibiting excessive cell growth, proliferation, division, and the like. Therefore, the nucleic acid ligand can exhibit an effect of preventing or treating KRAS mutant protein-expressing cancer (e.g., lung cancer, colorectal cancer, pancreatic cancer, breast cancer, ovarian cancer, endometrial cancer, cervical cancer, bladder cancer, gallbladder biliary tract cancer, acute myeloid leukemia, etc.).

In one embodiment, the therapeutic target may be a protein present in the cell membrane or a mutant thereof. For example, the therapeutic target present in the cell membrane may be at least one selected from the group consisting of PD-L1, PD-L2, CD40L, LAG3, TIM3, TIGIT, BTLA, CD52, SLAMF7, 4-1BB, OX-40, ICOS, GITR, CD27, CD28, CD16, CD3, CD20, EGFR family, AXL, CSF 1R, DDR1, DDR2, EPH receptor family, FGFR family, VEGFR family, IGF1R, LTK, PDGFR family, RET, KIT, NTRK1, and NTRK2.

In one embodiment, the novel nucleic acid ligand may bind to PD-L1 on the surface of the cancer cell to inhibit the binding of the T cell surface protein PD-1 to PD-L1, thereby blocking the inhibition of T cells, and thus allows T cells to attack the cancer cells. Therefore, the nucleic acid ligand can exhibit an effect of preventing or treating cancer (e.g., bladder cancer, lung cancer, breast cancer, ovarian cancer, urothelial cell cancer, skin cancer, etc.) expressing PD-L1 on the surface of cancer cells.

In one embodiment, the novel nucleic acid ligand can exhibit an anticancer immune effect by binding to CD3d/e or 4-1BB on the surface of cytotoxic T cells to activate cytotoxic T cells. Therefore, the novel nucleic acid ligand can exhibit an effect in cancer immunotherapy (e.g., the treatment of multiple myeloma, solid cancer, metastatic non-small cell carcinoma, kidney cancer, lung cancer, head and neck cancer, liver cancer, breast cancer, stomach cancer, esophageal cancer, ovarian cancer, Hodgkin's lymphoma, bladder cancer, melanoma, or the like) that can be applied by activation of cytotoxic T cells.

The present disclosure will be clearly understood from the above-described embodiments and the below-described examples. Hereinafter, the present disclosure will be explained in detail such that a person skilled in the art can easily understand and implement the disclosure by way of the examples described in reference to accompanying tables. However, these examples are given for illustrating the present disclosure, and the scope of the present disclosure is not limited to these examples.

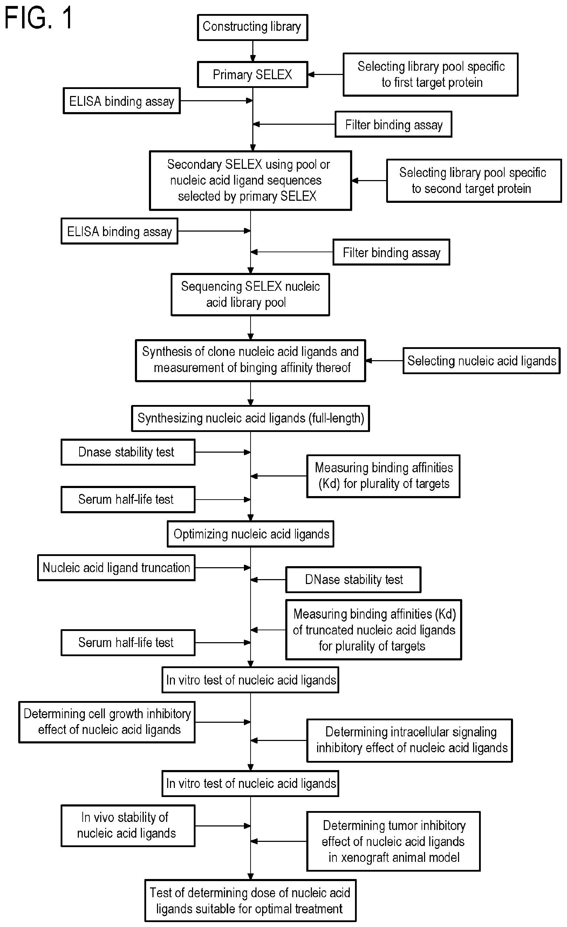

As shown in FIG. 1, the examples below were carried out to obtain nucleic acid ligands according to one embodiment of the present disclosure.

### [Example 1] Selection of novel nucleic acid ligands by first selection method

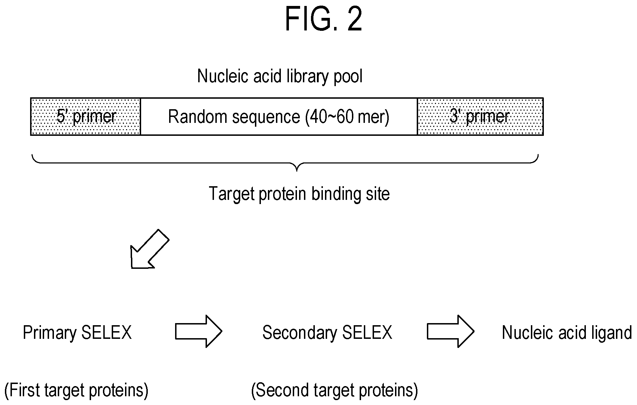

A nucleic acid library pool having binding affinity for a first target was selected from a nucleic acid library pool by using the SELEX technology (primary SELEX), and then secondary SELEX for a second target was immediately performed using the selected nucleic acid library pool, thereby ultimately obtaining nucleic acid ligands capable of binding to a plurality of targets (see FIG. 2). The selection method of novel nucleic acid ligands was specifically described below.

### 1. Construction of modified nucleic acid-containing libraries for SELEX

A single-stranded nucleic acid library template (Biotin-GCTGGTGGTGTGGCTG-N(40)-CAGGCAGACGGTCACTCA (SEQ ID NO: 1)) was prepared with any sequence of 40 consecutive nucleotides at the center, a primer binding site for nucleic acid amplification at each of the 5'-end and 3'-end of the any sequence, and biotin conjugated in front of the primer binding site of the 5'-end. The prepared nucleic acid library template is theoretically a mixture of 1 × 10²⁴ DNA with different nucleotide sequences. This single-stranded nucleic acid library template was synthesized using a DNA auto-synthesizer and used in the test after PAGE purification (Trilink, USA).

To construct a nucleic acid library containing the modified nucleic acid 5-(N-benzylcarboxamide)-2'-deoxyuridine (Bz-dU) or 5-(N-(1-naphthylmethyl)carboxamide)-2'-deoxyuridine (Nap-dU), the biotin-conjugated library template was fixed to a resin that was surface-modified with the streptavidin protein. Specifically, for the fixation, the library template and 500 µL of a resin (Thermo Scientific, USA) were mixed in a 5 M NaCl solution and reacted at room temperature for 1 hour at room temperature. In addition, 20 µM 5' primer (GAGTGACCGTCTGCCTG (SEQ ID NO: 2)), 0.5 mM dNTP (dATP, dGTP, dCTP, Nap-dUTP or Bz-dUTP), 0.25 U/µL KOD XL DNA polymerase (Merck, USA), 120 mM Tris-HCl pH 7.8, and 1X KOD buffer (Merck, USA) were added to the resin washed with NaCl, followed by an enzymatic reaction at 70°C for 2 hours, thereby preparing double-stranded DNA containing modified nucleic acids. The double-stranded DNA containing modified nucleic acids was denatured by 20 mM NaOH to separate the resin and single-stranded DNA, and then the separate supernatant was neutralized with a neutralization buffer (700 mM HCl, 180 mM HEPES, 0.45%(v/v) Tween 20). The libraries that had been separated were concentrated using Amicon ultra-15 (Merck Millipore, USA) and then quantified with a UV spectrophotometer, thereby finally constructing modified nucleic acid-containing single-stranded libraries.

### 2. Selection of nucleic acid library pool specific for first target protein (plasma protein)

### 2-1. Selection of nucleic acid library pools specific for plasma proteins (primary SELEX)

As a target protein for SELEX, a protein containing a histidine tag (6xHis-tag) was used among recombinant proteins. The target protein was immobilized on beads by using Dynabeads (Thermo Scientific, USA), which were magnetic bead that were surface-modified with cobalt and binds to the His-tag of the protein. Specifically, the target protein dissolved in buffer SB18 (40 mM HEPES, 102 mM NaCl, 5 mM KCl, 5 mM MgCl2, and 0.05% (v/v) Tween 20; the same hereinafter) and 50 µl of his-tag magnetic beads were reacted at 25° C in a thermomixer (Eppendorf, Germany) at 1200 rpm for 30 minutes and washed three times with buffer SB18 using a magnet, thereby preparing a first target protein immobilized on the beads. The first target protein is human serum albumin (HSA; Abeam, UK, product name: Recombinant human serum albumin Protein (His tag), catalog number: ab217817), immunoglobulin G (IgG; Abeam, UK, product name: Recombinant Human IgG1 protein (His tag), catalog number: ab219660), or transferrin (TF; Abeam, UK, product name: Recombinant human Transferrin protein (Active), catalog number: ab155698), corresponding to plasma proteins. All of the above plasma proteins were used in the examples below.

The synthesized nucleic acid library pool was placed in buffer SB18, followed by reaction from 95°C to 37°C, gradually lowered by 1°C, for 10 seconds at each temperature, thereby inducing the tertiary structure of the nucleic acid libraries. For non-specific negative selection, a protein competition buffer (1 µM prothrombin and 1 µM casein) was mixed with the above reaction solution, and then added to 10 µg of magnetic beads, followed by reaction at 37°C using a thermomixer at 1200 rpm for 10 minutes. Thereafter, the supernatant was transferred to a plasma protein-immobilized Dynabeads to select a nucleic acid library pool binding to the plasma protein. Specifically, the modified nucleic acid libraries that had been subjected to non-specific negative selection were reacted with the plasma protein-immobilized magnetic beads at 37°C for 1 hour in a thermomixer at 1200 rpm. The nucleic acid libraries and the target protein-magnetic bead complex were washed five times with buffer SB18, and then a nucleic acid library pool binding to the target protein was collected by addition of a 2 mM NaOH solution, and then neutralized with an 8 mM HCl solution.

The libraries binding to the plasma protein were amplified using quantitative PCR (QPCR, CFX real time PCR detection system; Bio-Rad, USA) for use in the next round of SELEX. The nucleic acid libraries collected from the above SELEX process was mixed with QPCR buffer (5' primer (GAGTGACCGTCTGCCTG (SEQ ID NO: 2)), 3' primer (Biotin-GCTGGTGGTGTGGCTG (SEQ ID NO: 3)), 1X KOD buffer, KOD XL DNA polymerase, 1 and mM dNTP), followed by one cycle under the conditions of 96°C for 15 seconds, 55°C for 10 seconds, and 68°C for 30 minutes and 30 cycles repeated under the conditions of 96°C for 15 seconds and 72°C for 1 minute, thereby amplifying nucleic acid libraries binding to plasma proteins to prepare double-stranded oligo DNA.

The double-stranded oligo DNA prepared through QPCR was immobilized by mixing with 25 µl of Myone^{™} streptavidin magnetic beads (Thermo Scientific, USA; the same hereinafter) at room temperature for 10 minutes, and anti-sense strands were removed by addition of a 20 mM NaOH solution. Thereafter, a nucleic acid library pool containing modified nucleic acids was synthesized using an enzyme by the same method as in the construction of the modified nucleic acid-containing libraries, and then used in the next round. The SELEX round was repeatedly performed a total of eight times.

In the 4th to 8th SELEX rounds, a kinetic challenge for reaction with 10 mM dextran sulfate was performed after the binding of the nucleic acid library pool and the target proteins, and subsequent processes were the same as the qPCR amplification process and the anti-sense collection process in the first round.

### 2-2. Determination of binding affinities of primary SELEX nucleic acid pools for plasma proteins

### 2-2-1. Bead-based ELISA binding assay: albumin, immunoglobulin G, and transferrin

To determine the binding affinity of the nucleic acid library pool subjected to the SELEX rounds for plasma proteins, bead-based ELISA binding assay was performed.

The nucleic acid library pools in the 5th to 7th rounds performed in advance were amplified. The nucleic acid library pool in each round was mixed with a buffer ((5' primer (GAGTGACCGTCTGCCTG (SEQ ID NO: 2), 3' primer (Biotin-GCTGGTGGTGTGGCTG (SEQ ID NO: 3)), 1X KOD buffer, KOD XL DNA polymerase, and 0.2mM dNTP), followed by 20 cycles repeated under the conditions of 96°C for 15 seconds and 72°C for 1 minute, thereby amplifying the nucleic acid library pool to prepare double-stranded oligo DNA. The double-stranded oligo DNA was immobilized by mixing with 25 µl of Myone^{™} streptavidin magnetic beads at room temperature for 10 minutes, and sense strands in the solution were removed by addition of a 20 mM NaOH solution. Thereafter, the magnetic bead-anti-sense complex was washed two times with buffer SB17 (40 mM HEPES, 102 mM NaCl, 5 mM KCl, 5 mM MgCl₂, 1 mM EDTA, and 0.05 %(v/v) Tween 20; the same hereinafter), and further washed with 16 mM NaCl. The magnetic bead-anti-sense complex that had been washed was mixed with 2.5 µM 5' primer (Biotin-GAGTGACCGTCTGCCTG (SEQ ID NO: 4)), 0.5 mM dNTP (dATP, dGTP, dCTP, Nap-dUTP or Bz-dUTP), 0.25 U/µL KOD XL DNA polymerase, 120mM Tris-HCl pH7.8, and 1X KOD buffer, followed by an enzymatic reaction at 68°C for 1 hour, thereby preparing a nucleic acid library pool containing modified nucleic acids and biotin. The supernatant was removed using a magnet and washed three times with buffer SB17. To collect newly synthesized biotin-anti-sense strands, 20 mM NaOH was added, followed by neutralization with 80 mM HCl.

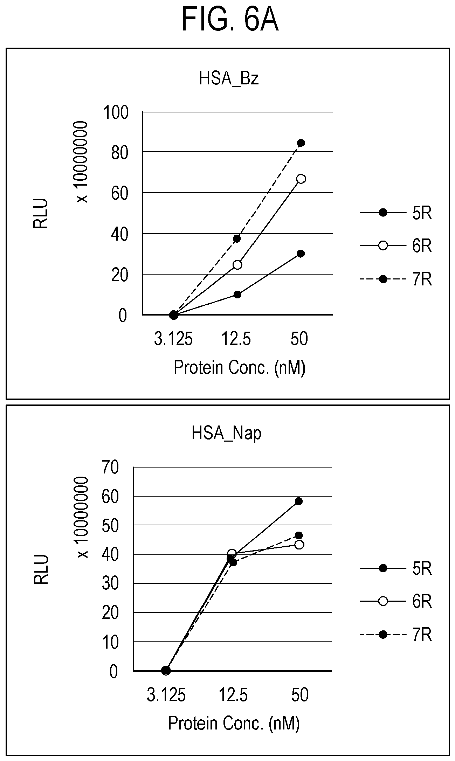

One pmol of the prepared biotin-conjugated nucleic acid library pool was placed in buffer SB18 and reacted from 95°C to 37°C, gradually lowered by 1°C, for 10 seconds at each temperature, thereby inducing the tertiary structure of DNA. The plasma proteins were prepared with 200 nM to 4-fold dilutions, which were then bound to a structure-stabilized nucleic acid library pool at 37°C for 30 minutes, and then 10 µl of histidine pull-down beads (Thermo Scientific, USA) were placed, followed by binding at 25°C for 30 minutes. The biotin nucleic acid library pool-plasma protein-bead complex was washed three times with buffer SB18 by using a magnet, and then 100 µl of a streptavidin-horseradish peroxidase (HRP) reagent diluted at a ratio of 1 :3,000 was added to the sample, followed by reaction at 25°C for 30 minutes. Again, the HRP-nucleic acid library pool-plasma protein-bead complex was washed three times with buffer SB18, and then 100 µl of a chemiluminescence solution was added to separate only the supernatant by using a magnet. The supernatant was transferred to a white plate, and the fluorescence value was measured using the luminescence protocol of the GloMax plate meter (Promega, USA). Thereafter, the results are analyzed after the subtraction of a resulting value in a negative control having no protein from a resulting value for each test group (see FIGS. 6A to 6C).

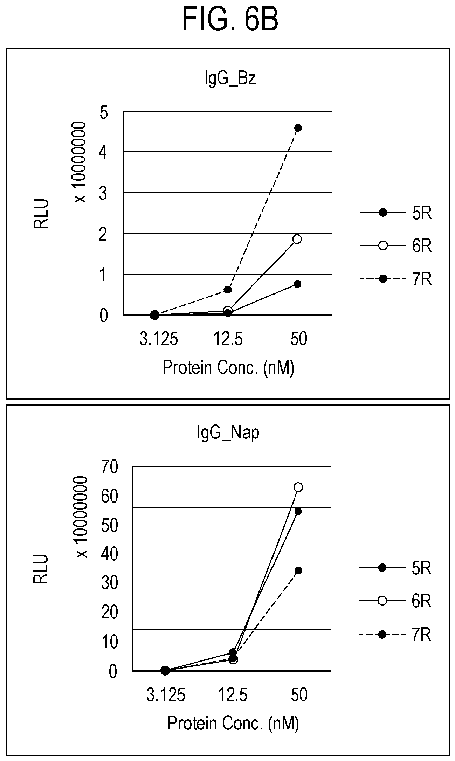

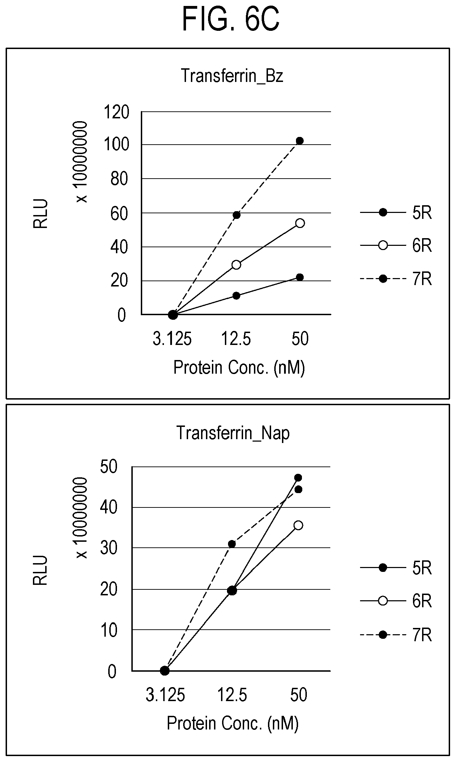

FIG. 6A shows the target binding affinity results of the nucleic acid library pools containing Bz-dU (top panel) and Nap-dU (bottom panel), respectively, for the first target protein albumin; FIG. 6B shows the target binding affinity results of the nucleic acid library pools containing Bz-dU and Nap-dU, respectively, for immunoglobulin G; and FIG. 6C shows the target binding affinity results of nucleic acid library pools containing Bz-dU and Nap-dU, respectively, for transferrin. FIGS. 6A to 6C confirmed the presence of nucleic acid library pools binding to target proteins in all of the primary SELEX steps, regardless of the type of modified nucleic acid or target protein used in the construction of libraries.

### 2-2-2. Filter binding assay: human serum albumin

Filter binding assay was performed as another method for determining the binding affinity of the nucleic acid library pool subjected to the SELEX rounds for the plasma protein.

The nucleic acid library pools in the 6th to 8th rounds performed in advance were subjected to end labeling with α-³²P ATP (Perkin Elmer, USA) and terminal deoxynucleotidyl transferase (TdT, New England Biolabs, USA).

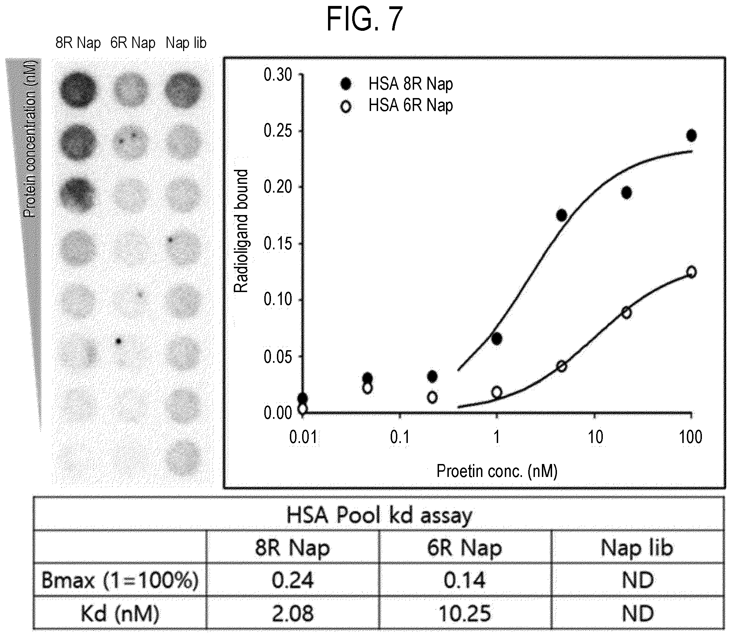

Specifically, the nucleic acid library pool obtained through the SELEX process was amplified by the same method as in **2-2-1** above, and then a sense single-stranded nucleic acid library pool was prepared. In addition, 1 pmol of the nucleic acid library pool, 0.25 µL of α-³²P ATP, 0.25 µL of TdT, and NEBuffer^{™} 4 (New England Biolabs, USA) were mixed and reacted at 37°C for 30 minutes, and then the TdT enzyme was inactivated by treatment at 70°C for 10 minutes. The labeled nucleic acid library pool was purified using a Micro spin G50 column (GE Healthcare, USA) and then quantified using a beta counter instrument. The nucleic acid library pool (20,000 cpm) was placed in 100µL of buffer SB18 and reacted from 95°C to 37°C, gradually lowered by 1°C, for 10 seconds at each temperature, thereby inducing the tertiary structure of the nucleic acid library pool. The target protein was subjected to serial dilution with buffer SB18, and then 30 µL of the structure-stabilized labeled nucleic acid library pool was added, followed by reaction at 37°C for 30 minutes. To the mixtures of the nucleic acid library pool and the target protein was added 5.5 µL of Zorbax resin (Agilent, USA), followed by reaction for 5 minutes. The samples that had been reacted was transferred to a MultiScreen-HV Filter Plate (Merck Millipore, USA) previously wetted with 50 µL of buffer SB18, and then the solution was removed by vacuum, and the membrane filter was washed with 100 µL of buffer SB18. The filter plate was exposed to an image plate for 16 hours, and then the image was quantified with FLA-5100 (Fujifilm, Japan). The binding affinity was calculated from the values obtained through the filter binding assay using SigmaPlot 11 (Systat Software, USA). BMax represents the ratio of bound oligo DNA to input; Kd (dissociation constant) represents affinity; and Nap lib indicates a nucleic acid library pool that has not been subjected to SELEX and was used as a negative control (see FIG. 7).

In addition, SELEX for selecting a nucleic acid library pool specifically binding to HSA, another first target protein, was performed, and then the target binding affinities of the nucleic acid library pools in the 6th and 8th rounds were determined by the above filter binding assay, and are shown in FIG. 7. FIG. 7 confirmed the presence of nucleic acid library pools showing binding affinity for the albumin protein in both of the two rounds. However, the nucleic acid library pool not subjected to SELEX showed no binding affinity for the albumin protein.

### 3. Selection of nucleic acid library pools specific to second target proteins (therapeutic target proteins)

Secondary SELEX was performed on therapeutic target proteins by using nucleic acid library pools in rounds in which the binding affinities for plasma proteins were confirmed through the above-described primary SELEX and binding assay. The secondary SELEX method is the same as the first SELEX method, but includes a counter selection process added, if necessary, to increase binding specificity to therapeutic target proteins.

The therapeutic target proteins used in the following examples are as below.
Wild-type KRAS protein: Abeam, product name: Recombinant Human KRAS protein (His tag), Catalog number: ab96817
KRAS G12D protein: Signal Chem, product name: KRAS (G12D) Protein(R06-32BH), Catalog number: R06-32BH
KRAS G12C protein: Signal Chem, product name: KRAS (G12C) Protein (R06-32DH), Catalog number: R06-32DH
KRASG12V protein: Signal Chem, product name: KRAS (G12V) Protein (R06-32CH), Catalog number: R06-32CH
PD-L1 protein: R&D SYSTEMS, product name: Recombinant Human PD-L1B7-H1 His-tag Protein, Catalog number: 9049-B7-100
BCL-2 protein: R&D SYSTEMS, product name: Recombinant Human Bcl-2 (minus C-Terminus) Protein, CF, Catalog number: 827-BC-050
4-1BB protein: R&D SYSTEMS, product name: Recombinant Human 4-1BB/TNFRSF9 Fc Chimera Protein, CF, Catalog number: 838-4B-100
CD3d/e protein: Prosci, product name: CD3 delta/epsilon Heterodimer Recombinant Protein (Fc Chimera), Catalog number: 96-164

To perform secondary SELEX, the nucleic acid library pool in a specific round, which had been confirmed to have binding affinity, was needed at a sufficient amount and thus was amplified using qPCR. The process after qPCR was the same as in primary SELEX. To select a nucleic acid library pool binding to a therapeutic target KRAS mutant protein, counter selection was performed on the wild-type KRAS protein. Specifically, the wild-type KRAS protein was added to 20 µg of magnetic beads and reacted at 37°C by using a thermomixer at 1200 rpm for 10 minutes, and then the wild-type protein-magnetic bead complex was washed three times using a magnet and buffer SB18. To the complex thus prepared were added a mixture of the prepared nucleic acid library pool and a protein competition buffer, followed by reaction at 37°C and 1200 rpm for 10 minutes, and then a nucleic acid library pool of the supernatant, which did not bind to the wild-type protein, was transferred to the mutant target protein-immobilized beads to select a nucleic acid library pool binding to the therapeutic target protein. Secondary SELEX for the therapeutic target protein was also performed in 5 to 8 rounds by the same method, and a kinetic challenge using dextran sulfate was not performed.

The binding affinity for therapeutic target proteins was determined using the nucleic acid library pool sample in each round by the same method as in the above-described ELISA binding assay (see FIGS. 8A to 8D).

For example, PD-L1, KRAS G12D, KRAS G12V, KRAS G12C, BCL-2, 4-1BB, and CD3d/e were used as the second target proteins. Of these, the results for PD-L1, KRAS G12D, KRAS G12V, KRAS G12C, and BCL-2 are shown in FIGS. 8A to 8D. FIGS. 8A to 8D confirmed the presence of nucleic acid library pools showing binding affinity for the second target proteins, regardless of the type of first target protein and the type of second target protein.

### 4. Nucleotide sequencing of SELEX nucleic acid library pools

The SELEX round nucleic acid library pool samples confirmed to have binding affinity were amplified through PCR using the 5' primer (GAGTGACCGTCTGCCTG (SEQ ID NO: 2)) and the 3' primer (GCTGGTGGTGTGGCTG (SEQ ID NO: 5)), cloned using the TOPO TA Cloning kit (Thermo Scientific, USA), and transformed into *E*. *coli* TOP10 cells. The plasmid nucleic acid of a single colony grown on an agar medium was amplified using a rolling circle amplification (RCA) kit (Enzynomics, Korea), and it was determined whether the cloned PCR product was inserted. Thereafter, nucleotide sequencing was performed on the RCA sample of the single sequence-inserted clones by the BigDye terminator cycle sequencing kit (Thermo Scientific, USA) and capillary electrophoresis (SolGent, Korea). For example, at least about 50 clones for each sample were sequenced to ultimately identify each nucleic acid sequence present in the nucleic acid library pools selected through SELEX.

The nucleic acid sequences that have been analyzed were analyzed by the Multalin website or our software to select sequence with the highest expression frequency (e.g., approximately 5 to approximately 10 sequences).

### 5. Synthesis of clone nucleic acid ligands through DNA polymerase and determination of binding affinity

The selected nucleic acid sequences were synthesized by using a double-stranded DNA used for nucleotide sequencing as a template, along with a 5' primer (GAGTGACCGTCTGCCTG (SEQ ID NO: 2)), a biotin-conjugated primer 3' primer (Biotin-GCTGGTGGTGTGGCTG (SEQ ID NO: 3)), and DNA polymerase, and corresponded to clone nucleic acid ligands. The binding affinities of the clone nucleic acid ligands for target proteins were determined by the same method as the bead-based ELISA binding assay as in **2-2-1** of Example 1 described above (e.g., see FIGS. 9A, 9B, 10A, and 10B).

For example, the binding affinities of the clone nucleic acid ligands, which had been sequenced and specifically bound to HSA and PD-L1, for respective target proteins were determined (see FIGS. 9A and 9B). As a result, 5019-A1 #2 clone and 5019-A1 #3 clone confirmed to have binding affinity were selected. These clones were used to synthesize nucleic acid ligands of SEQ ID NO: 99 and SEQ ID NO: 100, which were then used in the examples below (see FIGS. 9A and 9B).

For example, as a result of analyzing the binding affinities of the clone nucleic acid ligands to the HSA protein and the KRAS protein, respectively, S016-E1 #11 clone, S016-E1 #14 clone, S016-E2 #1 clone, and S016-E2 #12 clone confirmed to have binding affinity were selected. These clones were used to synthesize nucleic acid ligands of SEQ ID NO: 20, SEQ ID NO: 21, SEQ ID NO: 22, and SEQ ID NO: 28, which were then used in the examples below (see FIGS. 10A and 10B).

The sequences confirmed to have binding affinities for first and second target proteins were selected, and the full-length nucleic acid ligands thus selected were synthesized in the form of containing modified nucleic acids by using an DNA auto-synthesizer

For example, the full-length nucleic acid ligands selected by the method as in Example 1 were AP013, AP027, AP014, AA001, AB001, AD001, AD002, AD003, AD009, AV001, AV006, AX001, ID001, IC001, IC007, IV001, TD001, TD005, TD009, and TC001, which were nucleic acid ligands shown in Table 1.

### [Example 2] Preparation of novel nucleic acid ligands by the second selection method

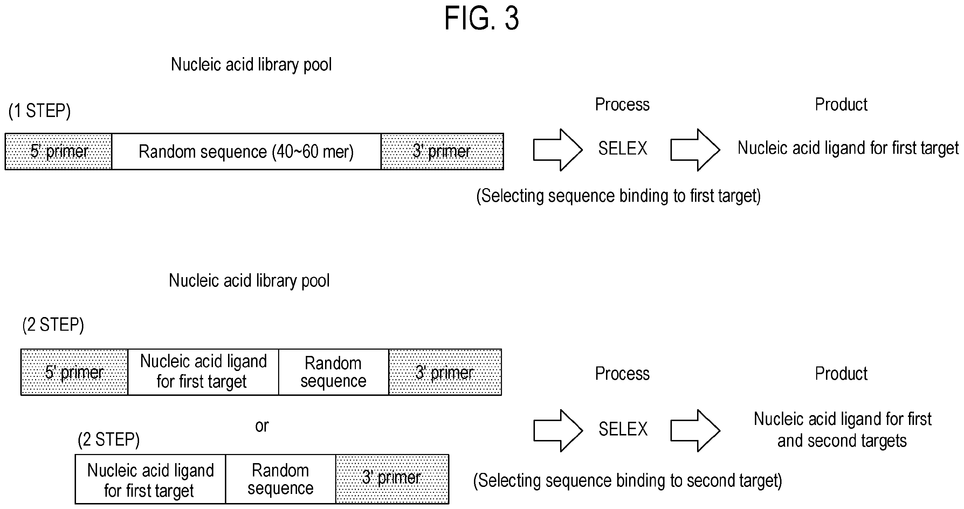

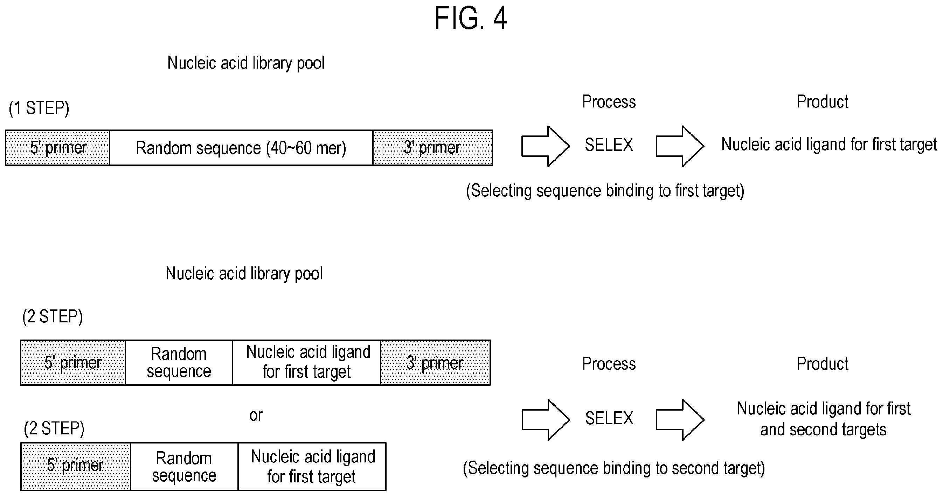

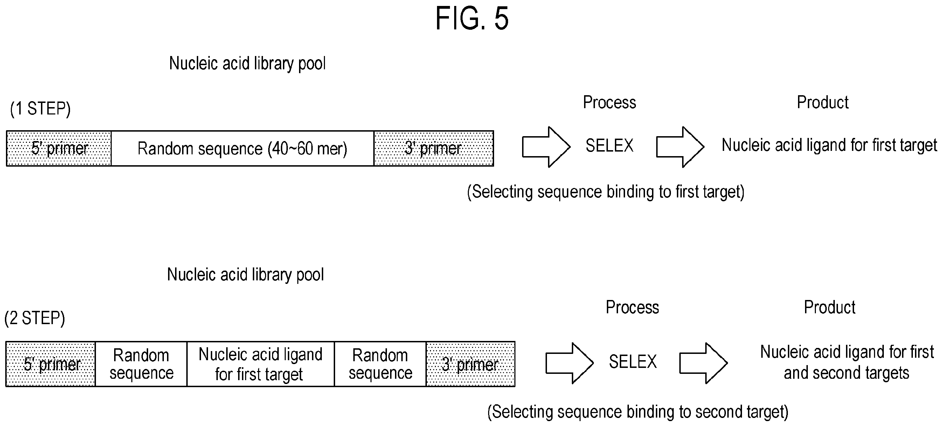

Sequences having binding affinity for the first target were selected from the first nucleic acid library pool by using SELEX technology (Primary SELEX), and a random sequence was linked to the selected sequences to construct the second nucleic acid library pool. Then, sequences having binding affinity for the second target were selected by performing secondary SELEX using the second nucleic acid library pool, and ultimately, nucleic acid ligands capable of binding to a plurality of targets were prepared (see FIGS. 3 to 5).

The specific preparation method of nucleic acid ligands was the same as the method as in Example 1, but different in view of the selection of the nucleic acid library pool specific to the second target protein as follows.

Specifically, the sequences confirmed to have binding affinity for a plasma protein were selected in Sections **1** and **2** of Example 1. Any consecutive sequence of a length of 25 to 35 was further linked to each of the selected nucleic acid sequences to prepare a consecutive nucleic acid sequence of a total length of 64 to 74, and a primer binding site for nucleic acid amplification was linked at 3'-end of the sequence and biotin was conjugated in front of the 5'-end thereof, thereby preparing a single-stranded nucleic acid library template (biotin-selected sequence-N(25 to 35)-CAGGCAGACGGTCACTCA). The prepared nucleic acid library template was theoretically a mixture of 1 × 10²⁴ nucleic acids having different nucleotide sequences from one another. To prevent the lengthening of the nucleic acid library, the primer binding site at the 5'-end was omitted, and the 5'-primer was substituted with the selected sequence. The template of such a single-stranded nucleic acid library was synthesized using a DNA auto-synthesizer and was used for tests after PAGE purification (Trilink, USA).

The newly prepared nucleic acid library was used to perform secondary SELEX on a therapeutic target protein by the same method as the method with respect to the first target protein.

For example, the full-length nucleic acid ligands selected by the method as in Example 2 were AD032, AD034, AP030, and AP031, which were nucleic acid ligands shown in Table 1.

For example, the full-length nucleic acid ligands selected by the methods of Examples 1 and 2 are shown in Table 1 below. The nucleic acid ligands thus prepared were analyzed and purified by a reverse-phase C18 column on Waters Prep 150 (Waters, USA) and Waters ACQUITY UPLC H-Class Bio PLUS System (Waters, USA). In addition, the mass spectrometry of the purified nucleic acid ligands was performed by Waters Xevo G2-XS Q-TOF System (Waters, USA). The mass spectrometry results are shown in Table 2.

In the nucleic acid sequence shown in the table below, each part designated as 6 indicates 5-(N-(1-naphthylmethyl)carboxamide)-2'-deoxyuridine (Nap-dU). In this regard, in SEQ ID NO: 6 to SEQ ID NO: 183 of the attached sequence listing, each part designated as n indicates 5-(N-(1-naphthylmethyl)carboxamide)-2'-deoxyuridine (Nap-dU).

**TABLE 1**

| Nucleic acid ligand code | First target | Second target | Sequence number | Nucleic acid sequence |
|---|---|---|---|---|
| AP013 | HSA | PD-L1 | SEQ ID NO: 84 | GAGTGACCGTCTGCCTGACAACAACGAG6GCGAAC6G6 AC6AC66GA6AGAGACCC6CAGCCACACCACCAGCC |
| AP027 | HSA | PD-L1 | SEQ ID NO: 98 | GAGTGACCGTCTGCCTG6CAA6CAC6GACGAAA6G6AC6 AC66GA6AGAGC6AACC6CAGCCACACCACCAGCC |
| AP014 | HSA | PD-L1 | SEQ ID NO: 85 | GAGTGACCGTCTGCCTGGAGCA6GG6CACA6G6AC6AC66 GA6AGGAGCCCG6GCC6CAGCCACACCACCAGCC |
| AA001 | HSA | CD3d/e | SEQ ID NO: 6 | GAGTGACCGTCTGCC6GGA6CC6GCCC6666AA6CCGAC66 6AA6GGCCACGC6CACCAGCCACACCACCAGCC |
| AB001 | HSA | 4-1BB | SEQ ID NO: 13 | GAGTGACCGTCTGCC6G6C6CCAACC6CGCC666ACAAC6 A6A6C6GACGAAC6AACACAGCCACACCACCAGCC |
| AD001 | HSA | KRAS G12D | SEQ ID NO: 20 | GAGTGACCGTCTGCCTG6G6GAGAA66A6G6ACC66AACG A6CA6CCGAAGG6666GACCACAGCCACACCACCAGCC |
| AD002 | HSA | KRAS G12D | SEQ ID NO: 21 | GAGTGACCGTCTGCCTG6G6C6GAGGGACCC66666AAGG GCA66A6CAGCA6GCC6CACACAGCCACACCACCAGCC |
| AD003 | HSA | KRAS G12D | SEQ ID NO: 22 | GAGTGACCGTC6GCCTGGAC6AGCA6AAG6CCGGA6GGC AGC666A6GCCGACCA6CAGCCACACCACCAGCC |
| AD009 | HSA | KRAS G12D | SEQ ID NO: 28 | GAGTGACCGTCTGCC6GCCCCGCC6C66C6GAAG66AACA 6G6GC6CCGAGA6GG6ACAGCCACACCACCAGCC |
| AD032 | HSA | KRAS G12D | SEQ ID NO: 42 | CAGG6CGAGC6G6ACGCG6G6G6GACC6GAAGACG6A6A AC6666ACAGAA6C6ACA |
| AD034 | HSA | KRAS G12D | SEQ ID NO: 43 | CAGG6CGAGC6G6ACGCG6G6G6GACC6GCCCCAC66C6G ACA66AGAC6G6G6AGA |
| AV001 | HSA | KRAS G12V | SEQ ID NO: 119 | GAGTGACCGTCTGCCTGAGCAGGAGGA6G6GACACAAC 666GCA666CAAGCC6GA6CAGCCACACCACCAGCC |
| AV006 | HSA | KRAS G12V | SEQ ID NO: 124 | GAGTGACCGTCTGCCTG6GAGAA66A6G6ACC66AACGA6 CA6CCGAAGG6666GACCAGCCACACCACCAGCC |
| AX001 | HSA | BCL-2 | SEQ ID NO: 173 | GAGTGACCGTCTGCCTG6ACGA6AAC6GAG6G6A6GCA66 AGACC6666G6GACCG6CAGCCACACCACCAGCC |
| ID001 | IgG | KRAS G12D | SEQ ID NO: 139 | GAGTGACCGTCTGCCTGGAC6AGCA6AAG6CCGGA6GGC AGC666A6GCCGACCA6CAGCCACACCACCAGCC |
| IC001 | IgG | KRAS G12C | SEQ ID NO: 128 | GAGTGACCGTCTGCCTGAAAG6CCGGA6GGCAGC666AC A6GCAGAAC6AACG6ACACAGCCACACCACCAGCC |
| IC007 | IgG | KRAS G12C | SEQ ID NO: 134 | GAGTGACCGTCTGCCTGGACCAGCA6AAG6CCGGA6GG CAGC666A6GCCGACCA6CAGCCACACCACCAGCC |
| IV001 | IgG | KRAS G12V | SEQ ID NO: 178 | GAGTGACCGTCTGCCTG6G6GAGAA66A6G6ACCC66AAC GA6CA6CCGAAGG6666GACCACAGCCACACCACCAGCC |
| TD001 | TF | KRAS G12D | SEQ ID NO: 149 | GAGTGACCGTCTGCCTGGGAC6AGCA6AAG6CCGGA6GG CAGC666A6GCCGACCA6CAGCCACACCACCAGCC |
| TD005 | TF | KRAS G12D | SEQ ID NO: 153 | GAGTGACCGTCTGCCTGAAAG6CCGGA6GGCAGC666AC AGGCAGAAC6AACG6ACACAGCCACACCACCAGCC |
| TD009 | TF | KRAS G12D | SEQ ID NO: 157 | GAGTGACCGTCTGCCTGGA6A6GCC6C66C6GCAG66ACG G6G6GA6CGCCCAA6AGCAGCCACACCACCAGCC |
| TC001 | TF | KRAS G12C | SEQ ID NO: 144 | GAGTGACCGTCTGCCTGGAC6AGCATAAG6CCGGA6GG CAGC666A6GCCGACCA6CAGCCACACCACCAGCC |
| AP030 | HSA | PD-L1 | SEQ ID NO: 99 | 6CCCAGG6CGAGC6G6ACGCG6G6G6GACCAC6G6AC6AC 66GA6AGAGGCAC6GCC6 |
| AP031 | HSA | PD-L1 | SEQ ID NO: 100 | 6CCCAGG6CGAGC6G6ACGCG6G6G6GACC66AC6GA6AA 6CACC66GAA6AG6G6AC |

### [Example 3] Optimization (truncation) and preparation of full-length nucleic acid ligands

A truncation process for optimization was performed using the full-length nucleic acid ligands selected by the methods as in Examples 1 and 2.

For optimization, the 2D structures of the full-length nucleic acid ligands were predicted using the Mfold web server (http://www.unafold.org/mfold/applications/dna-folding-form.php), and the length was reduced from both ends of the full-length nucleic acid ligands selected on the basis of the structures, to synthesize optimized nucleic acid ligand candidates with minimum lengths similar to the clone sequence (the full-length nucleic acid ligand serving as the parent). For example, the 2D prediction structures of the nucleic acid ligands AD001 to AD015 are shown in FIGS. 11A to 11D and 12A to 12C.

The nucleic acid ligands were synthesized by a general DNA auto-synthesis method using Mermade 12 or Mermade 48 (Bioautomation, USA). The nucleic acid ligand was sequentially synthesized from the 3'-end to the 5'-end by four steps (deblocking -> coupling -> capping -> oxidation) as a single cycle for each 1-mer (each one nucleotide). If necessary, biotin or a fluorescent substance (FAM or Cy5) was conjugated in front of the 5'-end of the nucleic acid ligand. The biotin and FAM were synthesized in an auto-synthesizer, and Cy5 was synthesized by amine coupling after 5'-amine nucleic acid ligand synthesis. The synthesized nucleic acid ligand or the biotin or fluorescent substance (FAM or Cy5)-conjugated nucleic acid ligand was purified by HPLC and used in the following example.

The nucleic acid ligands prepared in Example 3, together with full-length nucleic acid ligands (indicated in bold and underlined), are shown in Table 2. All of optimized nucleic acid ligands before a next full-length nucleic acid ligand used the same full-length nucleic acid ligand (e.g., AP001 to AP006 and AP021 to AP026 being obtained by using the AP013 full-length nucleic acid ligand).

The nucleic acid ligands thus prepared were analyzed and purified by a reverse-phase C18 column on Waters Prep150 (Waters, USA) and Waters ACQUITY UPLC H-Class Bio PLUS System (Waters, USA). In addition, the mass spectrometry of the purified nucleic acid ligands was performed by Waters Xevo G2-XS Q-TOF System (Waters, USA). The mass spectrometry results are shown in Table 2.

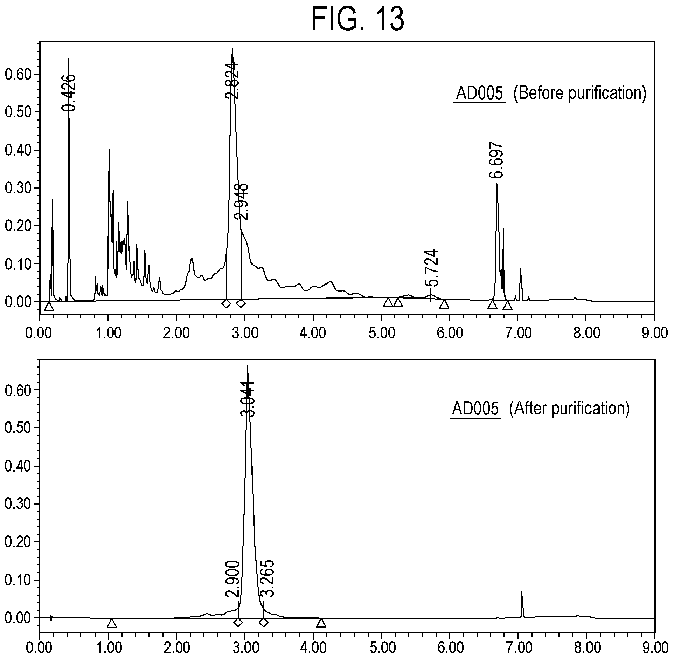

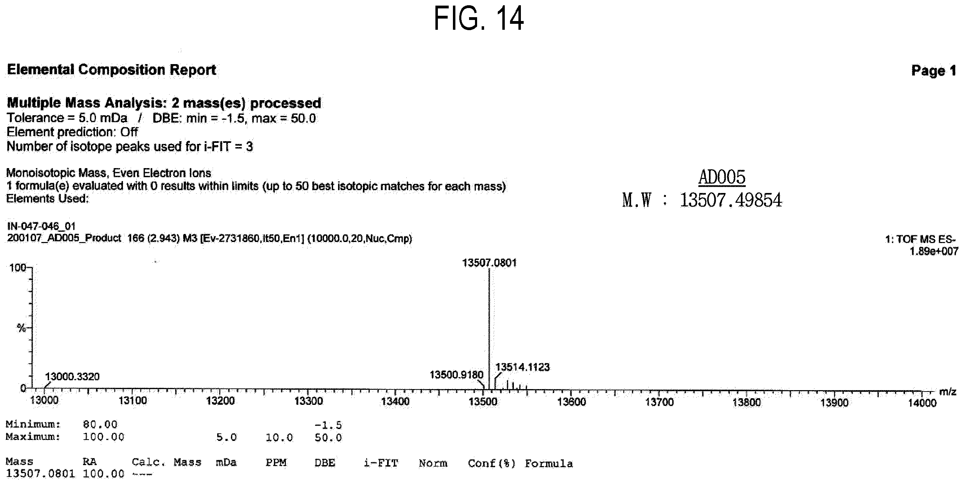

For example, the UPLC analysis results, before and after purification, of the nucleic acid ligand AD005 according to one embodiment of the present disclosure are shown in FIG. 13 and the mass spectrometry results thereof are shown in FIG. 14.

**TABLE 2**

| Nucleic acid ligand code | First target | Second target | Sequence number | Nucleic acid sequence | Calculated molecular weight (Da) | Measured molecular weight (Da) |
|---|---|---|---|---|---|---|
| **AP013** | HSA | PD-L1 | SEQ ID NO: 84 | | 24409.37708 | 24408.9492 |
| AP001 | HSA | PD-L1 | SEQ ID NO: 72 | | 17252.21971 | 17252.0977 |
| AP002 | HSA | PD-L1 | SEQ ID NO: 73 | | 14033.67385 | 14033.1553 |
| AP003 | HSA | PD-L1 | SEQ ID NO: 74 | | 12805.45465 | 12804.9521 |
| AP004 | HSA | PD-L1 | SEQ ID NO: 75 | | 11465.21672 | 11464.7471 |
| AP005 | HSA | PD-L1 | SEQ ID NO: 76 | | 10550.05513 | 10549.5020 |
| AP006 | HSA | PD-L1 | SEQ ID NO: 77 | | 9594.887391 | 9593.9219 |
| AP021 | HSA | PD-L1 | SEQ ID NO: 92 | | 22805.11586 | 22805.3711 |
| AP022 | HSA | PD-L1 | SEQ ID NO: 93 | | 21913.9655 | 21913.3750 |
| AP023 | HSA | PD-L1 | SEQ ID NO: 94 | | 19584.56662 | 19584.5176 |
| AP024 | HSA | PD-L1 | SEQ ID NO: 95 | | 17089.15504 | 17089.0684 |
| AP025 | HSA | PD-L1 | SEQ ID NO: 96 | | 16800.10867 | 16800.0156 |
| AP026 | HSA | PD-L1 | SEQ ID NO: 97 | | 15146.81313 | 15146.5127 |
| **AP027** | HSA | PD-L1 | SEQ ID NO: 98 | | 24881.52207 | 24881.1738 |
| AP015 | HSA | PD-L1 | SEQ ID NO: 86 | | 17555.31194 | 17555.2031 |
| AP016 | HSA | PD-L1 | SEQ ID NO: 87 | | 14505.81884 | 14505.3877 |
| AP017 | HSA | PD-L1 | SEQ ID NO: 88 | | 12644.45964 | 12644.9463 |
| AP018 | HSA | PD-L1 | SEQ ID NO: 89 | | 10967.15287 | 10966.6768 |
| AP019 | HSA | PD-L1 | SEQ ID NO: 90 | | 10076.00251 | 10075.2451 |
| AP020 | HSA | PD-L1 | SEQ ID NO: 91 | | 8511.706006 | 8511.6914 |
| **AP014** | HSA | PD-L1 | SEQ ID NO: 85 | | 24777.44422 | 24776.7734 |
| AP007 | HSA | PD-L1 | SEQ ID NO: 78 | | 17620.28685 | 17620.1641 |
| AP008 | HSA | PD-L1 | SEQ ID NO: 79 | | 14401.74099 | 14401.2188 |
| AP009 | HSA | PD-L1 | SEQ ID NO: 80 | | 13430.57834 | 13430.0586 |
| AP010 | HSA | PD-L1 | SEQ ID NO: 81 | | 11577.23545 | 11577.7646 |
| AP011 | HSA | PD-L1 | SEQ ID NO: 82 | | 10670.09018 | 10669.5986 |
| AP012 | HSA | PD-L1 | SEQ ID NO: 83 | | 9409.88115 | 9409.8633 |
| AP045 | HSA | PD-L1 | SEQ ID NO: 109 | | 14939.82759 | 14939.0322 |
| AP046 | HSA | PD-L1 | SEQ ID NO: 110 | | 13864.6248 | 13863.8174 |
| AP047 | HSA | PD-L1 | SEQ ID NO: 111 | | 13535.57228 | 13535.0977 |
| AP048 | HSA | PD-L1 | SEQ ID NO: 112 | | 13206.51976 | 13205.7412 |
| AP050 | HSA | PD-L1 | SEQ ID NO: 113 | | 10253.96285 | 10253.0781 |
| AP051 | HSA | PD-L1 | SEQ ID NO: 114 | | 9940.905245 | 9940.8955 |
| AP052 | HSA | PD-L1 | SEQ ID NO: 115 | GAGCCCG6GCC6CAGCC | 5873.139405 | 5872.1543 |
| AP053 | HSA | PD-L1 | SEQ ID NO: 116 | AGCCCG6GCC6CAGCC | 5544.086882 | 5543.0952 |
| AP054 | HSA | PD-L1 | SEQ ID NO: 117 | | 16713.14157 | 16713.1035 |
| AP055 | HSA | PD-L1 | SEQ ID NO: 118 | | 16400.08397 | 16400.043 |
| **AA001** | HSA | CD3d/e | SEQ ID NO: 6 | | 24777.44422 | 24777.6914 |
| AA002 | HSA | CD3d/e | SEQ ID NO: 7 | | 17595.29697 | 17595.1602 |
| AA003 | HSA | CD3d/e | SEQ ID | GA6CC6GCCC6666AA6CCGAC | 14545.80388 | 14545.2637 |
| | | | NO: 8 | 666AA6GGCCACGC6CAC | | |
| AA004 | HSA | CD3d/e | SEQ ID NO: 9 | | 12852.50219 | 12851.9795 |
| AA005 | HSA | CD3d/e | SEQ ID NO: 10 | | 11199.20665 | 11198.7344 |
| AA006 | HSA | CD3d/e | SEQ ID NO: 11 | | 9979.003771 | 9979.1611 |
| AA007 | HSA | CD3d/c | SEQ ID NO: 12 | GCCC6666AA6CCGAC666AA6 | 8558.753545 | 8558.7832 |
| **AB001** | HSA | 4-1BB | SEQ ID NO: 13 | | 25195.59154 | 25195.1484 |
| AB002 | HSA | 4-1BB | SEQ ID NO: 14 | | 17700.32864 | 17700.1992 |
| AB003 | HSA | 4-1BB | SEQ ID NO: 15 | | 14650.83554 | 14650.4082 |
| AB004 | HSA | 4-1BB | SEQ ID NO: 16 | | 12837.49881 | 12836.9912 |
| AB005 | HSA | 4-1BB | SEQ ID NO: 17 | | 11136.1808 | 11135.7344 |
| AB006 | HSA | 4-1BB | SEQ ID NO: 18 | | 9931.972837 | 9931.1016 |
| AB007 | HSA | 4-1BB | SEQ ID NO: 19 | | 9329.868854 | 9329.8477 |
| **AD001** | HSA | KRAS G12D | SEQ ID NO: 20 | | 26685.87207 | 26685.7910 |
| **AD002** | HSA | KRAS G12D | SEQ ID NO: 21 | | 25867.68811 | 25867.6348 |
| **AD003** | HSA | KRAS G12D | SEQ ID NO: 22 | | 24457.40326 | 24457.0605 |
| AD004 | HSA | KRAS G12D | SEQ ID NO: 23 | | 16962.14036 | 16962.0449 |
| AD005 | HSA | KRAS G12D | SEQ ID NO: 24 | | 13912.64727 | 13912.1592 |
| AD006 | HSA | KRAS G12D | SEQ ID NO: 25 | | 12235.3405 | 12234.8760 |
| AD007 | HSA | KRAS G12D | SEQ ID NO: 26 | | 11409.17794 | 11408.7393 |
| AD008 | HSA | KRAS G12D | SEQ ID NO: 27 | | 9899.928681 | 9899.9736 |
| AD016 | HSA | KRAS G12D | SEQ ID NO: 35 | | 10558.03373 | 10557.3926 |
| AD017 | HSA | KRAS G12D | SEQ ID NO: 36 | | 8536.633329 | 8535.7422 |
| AD018 | HSA | KRAS G12D | SEQ ID NO: 37 | GAC6AGCA6AAG6CCGGA6 | 6947.371777 | 6946.603 |
| AD020 | HSA | KRAS G12D | SEQ ID NO: 38 | | 10510.01126 | 10509.3818 |
| AD024 | HSA | KRAS G12D | SEQ ID NO: 39 | | 9610.882306 | 9609.9199 |
| AD025 | HSA | KRAS | SEQ ID | AG6CCGGA6GGCAGC666A6G | 8182.615762 | 8181.6958 |
| | | G12D | NO: 40 | C | | |
| AD026 | HSA | KRAS G12D | SEQ ID NO: 41 | | 10686.08509 | 10685.0693 |
| AD059 | HSA | KRAS G12D | SEQ ID NO: 50 | | 13439.54846 | 13438.7393 |
| AD060 | HSA | KRAS G12D | SEQ ID NO: 51 | | 13126.49086 | 13125.6611 |
| AD061 | HSA | KRAS G12D | SEQ ID NO: 52 | | 11906.28798 | 11905.8672 |
| AD063 | HSA | KRAS G12D | SEQ ID NO: 53 | | 11328.19523 | 11327.3516 |
| AD064 | HSA | KRAS G12D | SEQ ID NO: 54 | | 10999.1427 | 10998.335 |
| AD065 | HSA | KRAS G12D | SEQ ID NO: 55 | | 8793.689874 | 8792.627 |
| AD066 | HSA | KRAS G12D | SEQ ID NO: 56 | | 9281.829783 | 9281.8711 |
| AD067 | HSA | KRAS G12D | SEQ ID NO: 57 | | 8663.730885 | 8662.6592 |
| AD068 | HSA | KRAS G12D | SEQ ID NO: 58 | AAG6CCGGA6GGCAGC666 | 7091.418059 | 7090.4351 |
| AD069 | HSA | KRAS G12D | SEQ ID NO: 59 | AG6CCGGA6GGCAGC666 | 6778.360451 | 6777.4009 |
| AD070 | HSA | KRAS G12D | SEQ ID NO: 60 | AAG6CCGGA6GGCAGC66 | 6618.319254 | 6617.3169 |
| AD071 | HSA | KRAS G12D | SEQ ID NO: 61 | GAC6AGCA6AAG6CC | 5471.120488 | 5470.1323 |
| AD072 | HSA | KRAS G12D | SEQ ID NO: 62 | GAC6AGCA6AAG6C | 5182.074113 | 5181.0767 |
| AD073 | HSA | KRAS G12D | SEQ ID NO: 63 | GAC6AGCA6AAG6 | 4893.027738 | 4892.04 |
| AD078 | HSA | KRAS G12D | SEQ ID NO: 64 | AGC666A6GCCGACCA6 | 6393.306865 | 6393.3403 |
| AD079 | HSA | KRAS G12D | SEQ ID NO: 65 | GC666A6GCCGACCA6 | 6080.249257 | 6079.2754 |
| **AD009** | HSA | KRAS G12D | SEQ ID NO: 28 | | 25026.52588 | 25025.8418 |
| AD010 | HSA | KRAS G12D | SEQ ID NO: 29 | | 17531.26298 | 17531.1484 |
| AD011 | HSA | KRAS G12D | SEQ ID NO: 30 | | 14481.76989 | 14481.2471 |
| AD012 | HSA | KRAS G12D | SEQ ID NO: 31 | | 12564.40963 | 12563.9199 |
| AD013 | HSA | KRAS G12D | SEQ ID NO: 32 | | 11079.1716 | 11078.6982 |
| AD014 | HSA | KRAS G12D | SEQ ID NO: 33 | | 11280.18936 | 11279.7344 |
| AD015 | HSA | KRAS G12D | SEQ ID NO: 34 | | 9794.951341 | 9794.9541 |
| AD080 | HSA | KRAS G12D | SEQ ID NO: 66 | | 12418.43912 | 12417.5723 |
| AD081 | HSA | KRAS G12D | SEQ ID NO: 67 | | 12105.38151 | 12104.5459 |
| AD083 | HSA | KRAS G12D | SEQ ID NO: 68 | | 11945.34031 | 11945.0078 |
| AD084 | HSA | KRAS G12D | SEQ ID NO: 69 | 6CCGAGA6GG6A | 4595.965045 | 4594.9746 |
| AD085 | HSA | KRAS G12D | SEQ ID NO: 70 | | 14168.71228 | 14167.8818 |
| AD086 | HSA | KRAS G12D | SEQ ID NO: 71 | | 13695.61348 | 13694.8086 |
| **AD032** | HSA | KRAS G12D | SEQ ID NO: 42 | | 20523.88859 | 20523.3672 |
| AD043 | HSA | KRAS G12D | SEQ ID NO: 162 | | 12107.28913 | 12106.8447 |
| AD044 | HSA | KRAS G12D | SEQ ID NO: 163 | | 14008.65448 | 14008.1396 |
| AD045 | HSA | KRAS G12D | SEQ ID NO: 164 | | 16030.05488 | 16029.8789 |
| AD046 | HSA | KRAS G12D | SEQ ID NO: 165 | | 17747.3678 | 17747.3984 |
| AD047 | HSA | KRAS G12D | SEQ ID NO: 166 | | 19608.727 | 19608.5332 |
| AD048 | HSA | KRAS G12D | SEQ ID NO: 167 | | 10317.06414 | 10316.5586 |
| AD049 | HSA | KRAS G12D | SEQ ID NO: 168 | | 12010.36583 | 12009.8408 |
| AD050 | HSA | KRAS G12D | SEQ ID NO: 169 | | 13943.72101 | 13943.1904 |
| AD051 | HSA | KRAS G12D | SEQ ID NO: 170 | | 15653.01761 | 15652.9365 |
| AD052 | HSA | KRAS G12D | SEQ ID NO: 171 | | 17530.37173 | 17530.3730 |
| AD053 | HSA | KRAS G12D | SEQ ID NO: 172 | | 19263.67956 | 19263.7363 |
| **AD034** | HSA | KRAS G12D | SEQ ID NO: 43 | | 20483.84472 | 20483.1777 |
| AD036 | HSA | KRAS G12D | SEQ ID NO: 44 | | 19055.57818 | 19054.5293 |
| AD037 | HSA | KRAS G12D | SEQ ID NO: 45 | | 18726.52565 | 18725.4512 |
| AD038 | HSA | KRAS G12D | SEQ ID NO: 46 | | 18253.42685 | 18253.7031 |
| AD039 | HSA | KRAS G12D | SEQ ID NO: 47 | | 17162.22915 | 17161.3594 |
| AD040 | HSA | KRAS G12D | SEQ ID NO: 48 | | 16849.17154 | 16848.0254 |
| AD041 | HSA | KRAS G12D | SEQ ID NO: 49 | | 12764.45697 | 12763.2266 |
| **AV001** | HSA | KRAS G12V | SEQ ID NO: 119 | | 24809.47177 | 24809.8281 |
| AV002 | HSA | KRAS G12V | SEQ ID NO: 120 | | 20469.77032 | 20469.3262 |
| | | | | 6CAGCCACACCACCAGCC | | |
| AV003 | HSA | KRAS G12V | SEQ ID NO: 121 | | 16287.06999 | 16287.0889 |
| AV004 | HSA | KRAS G12V | SEQ ID NO: 122 | | 14433.76854 | 14433.3174 |
| AV005 | HSA | KRAS G12V | SEQ ID NO: 123 | | 11071.03097 | 11070.5186 |
| **AV006** | HSA | KRAS G12V | SEQ ID NO: 124 | | 25281.61676 | 25281.3516 |
| AV007 | HSA | KRAS G12V | SEQ ID NO: 125 | | 17961.39473 | 17961.2266 |
| AV008 | HSA | KRAS G12V | SEQ ID NO: 126 | | 15508.01752 | 15507.9736 |
| AV009 | HSA | KRAS G12V | SEQ ID NO: 127 | | 13437.64084 | 13437.1006 |
| **AX001** | HSA | BCL-2 | SEQ ID NO: 173 | | 25457.65288 | 25457.5684 |
| AX002 | HSA | BCL-2 | SEQ ID NO: 174 | | 18730.52325 | 18730.3633 |
| AX003 | HSA | BCL-2 | SEQ ID NO: 175 | | 15107.88511 | 15107.7080 |
| AX004 | HSA | BCL-2 | SEQ ID NO: 176 | | 12388.37351 | 12388.8965 |
| AX005 | HSA | BCL-2 | SEQ ID NO: 177 | | 11655.34013 | 11654.8096 |
| **ID001** | IgG | KRAS G12D | SEQ ID NO: 139 | | 24288.3505 | 24287.6738 |
| ID002 | IgG | KRAS G12D | SEQ ID NO: 140 | | 18157.33676 | 18157.2598 |
| ID003 | IgG | KRAS G12D | SEQ ID NO: 141 | | 14039.62423 | 14039.1328 |
| ID004 | IgG | KRAS G12D | SEQ ID NO: 142 | | 12828.43291 | 12827.9570 |
| ID005 | IgG | KRAS G12D | SEQ ID NO: 143 | | 19948.64904 | 19948.5723 |
| **IC001** | IgG | KRAS G12C | SEQ ID NO: 128 | | 24449.38323 | 24448.9531 |
| IC002 | IgG | KRAS G12C | SEQ ID NO: 129 | | 19335.52639 | 19335.3242 |
| IC003 | IgG | KRAS G12C | SEQ ID NO: 130 | | 14985.73896 | 14985.2871 |
| IC004 | IgG | KRAS G12C | SEQ ID NO: 131 | | 14073.68 | 14073.1436 |
| IC005 | IgG | KRAS G12C | SEQ ID NO: 132 | | 13381.47774 | 13381.0195 |
| IC006 | IgG | KRAS G12C | SEQ ID NO: 133 | | 11857.22882 | 11856.7051 |
| **IC007** | IgG | KRAS G12C | SEQ ID NO: 134 | | 24104.29807 | 24103.6348 |
| IC008 | IgG | KRAS G12C | SEQ ID NO: 135 | | 16793.0876 | 16793.0137 |
| IC009 | IgG | KRAS G12C | SEQ ID NO: 136 | | 15388.83229 | 15388.7598 |
| IC010 | IgG | KRAS G12C | SEQ ID NO: 137 | | 13855.5718 | 13855.0996 |
| IC011 | IgG | KRAS G12C | SEQ ID NO: 138 | | 12644.38048 | 12643.9121 |
| **IV001** | IgG | KRAS G12V | SEQ ID NO: 178 | | 26974.91845 | 26974.8516 |
| IV002 | IgG | KRAS G12V | SEQ ID NO: 179 | | 22635.217 | 22634.6035 |
| IV003 | IgG | KRAS G12V | SEQ ID NO: 180 | | 17108.19003 | 17108.0723 |
| IV004 | IgG | KRAS G12V | SEQ ID NO: 181 | | 15125.84197 | 15125.4365 |
| IV005 | IgG | KRAS G12V | SEQ ID NO: 182 | | 13616.59271 | 13616.0742 |
| IV006 | IgG | KRAS G12V | SEQ ID NO: 183 | | 16599.21522 | 16599.1641 |
| **TD001** | TF | KRAS G12D | SEQ ID NO: 149 | | 24617.40302 | 24616.8574 |
| TD002 | TF | KRAS G12D | SEQ ID NO: 150 | | 16375.03605 | 16374.9404 |
| TD003 | TF | KRAS G12D | SEQ ID NO: 151 | | 14241.69979 | 14241.1777 |
| TD004 | TF | KRAS G12D | SEQ ID NO: 152 | | 10967.03228 | 10966.5381 |
| **TD005** | TF | KRAS G12D | SEQ ID NO: 153 | | 24305.33695 | 24304.8672 |
| TD006 | TF | KRAS G12D | SEQ ID NO: 154 | | 17803.21971 | 17803.1973 |
| TD007 | TF | KRAS G12D | SEQ ID NO: 155 | | 16198.9585 | 16198.8730 |
| TD008 | TF | KRAS G12D | SEQ ID NO: 156 | | 12042.23506 | 12041.7080 |
| **TD009** | TF | KRAS G12D | SEQ ID NO: 157 | | 25081.5317 | 25080.6152 |
| | | | | G6GA6CGCCCAA6AGCAGCCA CACCACCAGCC | | |
| TD010 | TF | KRAS G12D | SEQ ID NO: 158 | | 19841.66832 | 19841.5605 |
| TD011 | TF | KRAS G12D | SEQ ID NO: 159 | | 16593.12313 | 16593.0039 |
| TD012 | TF | KRAS G12D | SEQ ID NO: 160 | | 15701.97277 | 15701.9824 |
| TD013 | TF | KRAS G12D | SEQ ID NO: 161 | | 19355.55663 | 19355.4766 |
| **TC001** | TF | KRAS G12C | SEQ ID NO: 144 | | 24119.29773 | 24118.6152 |
| TC002 | TF | KRAS G12C | SEQ ID NO: 145 | | 17988.284 | 17988.2266 |
| TC003 | TF | KRAS G12C | SEQ ID NO: 146 | | 14665.73944 | 14665.2207 |
| TC004 | TF | KRAS G12C | SEQ ID NO: 147 | | 13870.57146 | 13869.9961 |
| TC005 | TF | KRAS G12C | SEQ ID NO: 148 | | 12659.38015 | 12658.9131 |
| **AP030** | HSA | PD-L1 | SEQ ID NO: 99 | | 20367.74236 | 20366.1543 |
| **AP031** | HSA | PD-L1 | SEQ ID NO: 100 | | 20703.85739 | 20702.7285 |
| AP033 | HSA | PD-L1 | SEQ ID NO: 101 | | 20089.8044 | 20088.1133 |
| AP034 | HSA | PD-L1 | SEQ ID NO: 102 | | 17738.39767 | 17737.1855 |
| AP035 | HSA | PD-L1 | SEQ ID NO: 103 | | 16005.08983 | 16005.373 |
| AP036 | HSA | PD-L1 | SEQ ID NO: 104 | | 14311.78815 | 14311.8096 |
| AP037 | HSA | PD-L1 | SEQ ID NO: 105 | | 11576.28164 | 11576.7568 |
| AP038 | HSA | PD-L1 | SEQ ID NO: 106 | | 13967.6568 | 13967.8672 |
| AP039 | HSA | PD-L1 | SEQ ID NO: 107 | | 15668.9748 | 15668.9502 |
| AP040 | HSA | PD-L1 | SEQ ID NO: 108 | | 18934.58932 | 18934.2793 |

| | | | | | | |
|---|---|---|---|---|---|---|
| *In the mass spectrometry results of Table 2, the nucleic acid ligands having biotin conjugated to the 5' end thereof were used except for the nucleic acid ligands AP030 and AP031. | | | | | | |

Hereinafter, a procedure for selecting nucleic acid ligands capable of exhibiting efficacy when actually used as a drug was performed on the nucleic acid ligands prepared in Examples 1 to 3.

### [Example 4] Dnase stability of selected full-length nucleic acid ligands against plurality of targets

There are several types of nucleases in serum, and it is well known that the reaction of oligonucleic acid materials with serum results in the degradation of oligonucleic acid molecules within several hours. However, it has been reported that a nucleic acid ligand conjugated to a plasma protein such as albumin is not degraded by exhibiting resistance to the degradation action of nucleases. The nucleic acid ligands, even when conjugated to target proteins other than plasma proteins, can exhibit resistance to the degradation action of nucleases. Therefore, the selected full-length nucleic acid ligands were reacted under conditions of exposure to a nuclease to checking the degradation degree of the nucleic acid ligands, thereby evaluating the presence or absence of the binding with first and second target proteins and furthermore evaluating the stability in serum. These evaluations can determine in advance whether a nucleic acid ligand can exhibit valid efficacy by binding to a target protein in the blood to maintain its structure or its target binding site, which exhibits the desired effect.

First, 0.5 pmol of a nucleic acid ligand (selected by the method as in Example 1 and 2) dissolved in buffer SB18, DMEM, a buffer solution for nuclease reaction (DNaseI RNase-free, Thermo scientific, USA), and sterile DW were mixed. The nucleic acid ligand mixture thus prepared was reacted from 95°C to 37°C, gradually lowered by 1°C, for 10 seconds at each temperature, thereby inducing the tertiary structure of the nucleic acid ligand. To the stabilized nucleic acid ligand was added 20 pmol of a first target protein (a plasma protein) or a second target protein (a therapeutic target protein), followed by reaction at 4°C for 30 minutes, thereby conjugating the target protein to the nucleic acid ligand. The target protein was not added for a positive control and a negative control. To this reaction solution was added 1 unit of a nuclease (DNaseI, Thermo Scientific, USA), thereby activating the action of the nuclease at 37°C for 16 hours. The nuclease was not added for the negative control. After the 16-hour reaction, the nuclease was inactivated by heating at 85°C, and electrophoresis was performed on a 10% urea-polyacrylamide gel (Urea-Polyacrylamide gel). The stability of the nucleic acid ligand against the nuclease was determined by comparing the band intensities of the respective controls and respective test groups. From the above results, whether the nucleic acid ligand binds to the target protein with high affinity could be confirmed, and nucleic acid ligand candidates exhibiting excellent stability against the nuclease could be selected.

The above test was conducted using the full-length nucleic acid ligands selected by the methods as in Examples 1 and 2. The nucleic acid ligands having significant band intensities for both the plasma protein and the therapeutic target protein would have excellent binding characteristics to both the plasma protein and therapeutic target protein. The binding to each target protein was evaluated to be significant when the band intensity was 10% or more in comparison with the band of the negative control without the addition of the nuclease, in the electrophoresis results. Typical full-length nucleic acid ligands showing binding to both the plasma protein and the therapeutic target protein are shown in Table 3. Even though the Dnase electrophoresis results for the full-length nucleic acid ligands are not significant, the optimized sequences thereof might show significant electrophoresis results and confirmed binding affinity (Kd) (e.g., TD001, TD005, and TD009).

For example, the electrophoresis results with respect to Dnase stability for AD001, AD002, AD003, and AD009 nucleic acid ligands are shown in FIGS. 11A to 11D and 12A to 12C.

To further determine the binding affinity, for a target, of the nucleic acid ligands showing binding to each target protein, the biding affinity (K_{d}) was measured.

### [Example 5] Binding affinity (K_{d}) of full-length nucleic acid ligands for plurality of targets

The binding affinity of the nucleic acid ligands were measured using enzyme linked immunosorbent assay (ELISA). First, the 5' biotin-conjugated nucleic acid ligand was reacted in buffer SB18 from 95°C to 37°C, gradually lowered by 1°C, for 10 seconds at each temperature, thereby inducing the tertiary structure of the nucleic acid ligand.

The test method was composed of the following steps.
1) A target protein was coated on a microplate at a low temperature for 16 hours.
2) The surface of the microplate was blocked by reaction at room temperature for 1 hour using Blocking Buffer (Pierce^{™} Protein-Free (PBS) Blocking Buffer, Thermo Scientific, USA).
3) The microplate was treated with a nucleic acid ligand, followed by reaction at 37°C for 1 hour.
4) For enzyme conjugation, the microplate was treated with a streptavidin-horseradish peroxidase (HRP) conjugate (Thermo Scientific, USA), followed by reaction at room temperature for 1 hour.
5) The microplate was treated with TMB substrate solution (Thermo Scientific, USA), followed by reaction at room temperature for 30 minutes.
6) The microplate was treated with a reaction termination solution (2 M sulfuric acid).
7) The absorbance was measured at 450 nm using GloMax plate meter (Promega, USA).

The target protein was used with 500 nM, and the nucleic acid ligand was used with 500 nM to 4-fold dilutions. The measured binding affinities of the nucleic acid ligands for respective target proteins are shown in Table 3. It could be confirmed that the nucleic acid ligands showing the binding to all the target proteins in the electrophoresis results exhibited binding affinity for each target protein.

In Table 3, "No binding" indicates that the binding affinity of a nucleic acid ligand for a target was predicted to be low due to the absence of a significant absorbance value, and "N/A" indicates that the present test was not conducted.

### [Example 6] Serum half-life of full-length nucleic acid ligands

First, 2 pmol of the nucleic acid ligand dissolved in buffer SB18 was reacted from 95°C to 37°C, gradually lowered by 1°C, for 10 seconds at each temperature, thereby inducing the tertiary structure of the nucleic acid ligand. The stabilized nucleic acid ligand was added to the 96% human serum and then reacted at 37°C for 0, 3, 6, 9, 12, 15, and 18 hours, thereby inducing the degradation of the nucleic acid ligand. After each of the reaction times, the nuclease was inactivated by heating at 85°C, and electrophoresis was performed on a 10% urea-polyacrylamide gel. A control without the addition of human serum was included as a loading control, and the band intensities of the control and the test groups were measured. The intensity trend line over time was determined using the measurement values, and the intensity value was inserted into the trend equation to obtain the half-life. If the intensity value deviates from the trend line, an additional test was conducted under conditions of 0, 24, 48, 72, and 96 hours.

For example, the electrophoresis results over time for the AD001 to AD015 nucleic acid ligands are shown in FIGS. 11A to 11D and 12A to 12C. In addition, the serum half-life (hr) thus measured for each target protein is shown in Table 3. Table 3 confirmed that the nucleic acid ligands according to one embodiment of the present disclosure showed high serum half-life, indicating that the nucleic acid ligands according to one embodiment of the present disclosure can have excellent binding affinity for the plasma proteins and thus show excellent in vivo stability.

### [Example 7] Binding affinity (Kd), serum half-life, and Dnase stability of optimized nucleic acid ligands

Some of the optimized nucleic acid ligands prepared in Example 3 were tested for Dnase stability for target proteins by the same method as in Example 4. The results for typical optimized nucleic acid ligands are shown in Table 3.

The optimized nucleic acid ligands confirmed to have binding to target proteins according to the Dnase stability test were measured for binding affinities (K_{d}) for target proteins by the same method as in Example 5 and determined for the serum half-life by the same method as in Example 6. The results thus obtained are shown in Table 3.

The nucleic acid ligands indicated in bold and underlined on Table 3 were the original full-length nucleic acid ligands (full length sequences), which were basis for optimization, and the nucleic acid ligands therebelow corresponded to the optimized truncated nucleic acid ligands (truncated sequences).

Based on the results of Table 3, candidate nucleic acid ligands for in vitro and in vivo tests for a desired therapeutic target could be selected.

**TABLE 3**

| Nucleic acid ligand code | First target | Second target | Sequence number | Serum half-life t_{1/2} (hr) | Biding affinity for first target binding affinity | | Binding affinity for second target | |
|---|---|---|---|---|---|---|---|---|
| | | | | | K_{d}(nM) | R² | K_{d}(nM) | R² |
| **AP013** | HSA | PD-L1 | SEQ ID NO: 84 | 72 or longer | 19.05 | 92 | 73.97 | 99.6 |
| AP001 | HSA | PD-L1 | SEQ ID NO: 72 | 72 or longer | 20.88 | 95.3 | 42.74 | 98.1 |
| AP021 | HSA | PD-L1 | SEQ ID NO: 92 | 72 or longer | 15.57 | 91.7 | 129.53 | 98.9 |
| AP022 | HSA | PD-L1 | SEQ ID NO: 93 | 72 or longer | 16.61 | 90.0 | 66.56 | 99.3 |
| AP023 | HSA | PD-L1 | SEQ ID NO: 94 | 72 or longer | 51.72 | 95.2 | 93.21 | 95.7 |
| AP024 | HSA | PD-L1 | SEQ ID NO: 95 | 72 or longer | 22.29 | 95.0 | 33.26 | 99.8 |
| **AP027** | HSA | PD-L1 | SEQ ID NO: 98 | 72 or longer | 2.17 | 83.9 | 353.64 | 94.70 |
| **AA001** | HSA | CD3d/e | SEQ ID NO: 6 | 6.42 | 9.62 | 89.6 | 3.17 | 74.5 |
| AA003 | HSA | CD3d/e | SEQ ID NO: 8 | 72 or longer | 4.32 | 97.5 | 7.63 | 65 |
| AA004 | HSA | CD3d/e | SEQ ID NO: 9 | 11.7 | 9.54 | 98.1 | 10.21 | 96.4 |
| AA005 | HSA | CD3d/e | SEQ ID NO: 10 | 15.6 | 8.27 | 97.0 | 39.42 | 93.0 |
| AA006 | HSA | CD3d/e | SEQ ID NO: 11 | 4.1 | 15.54 | 93 | 14.28 | 97.0 |
| AA007 | HSA | CD3d/e | SEQ ID NO: 12 | 13.5 | 2.03 | 96 | 15.05 | 96.0 |
| **AB001** | HSA | 4-1BB | SEQ ID NO: 13 | 72 or longer | 8.39 | 97.7 | 0.04 | 96.4 |
| AB002 | HSA | 4-1BB | SEQ ID NO: 14 | 47.01 | 2.92 | 98.4 | 0.48 | 99.1 |
| AB003 | HSA | 4-1BB | SEQ ID NO: 15 | 8.3 | 19.77 | 87.3 | 0.87 | 99.4 |
| AB004 | HSA | 4-1BB | SEQ ID NO: 16 | 6.4 | 101.51 | 87.3 | 0.05 | 98.9 |
| AB005 | HSA | 4-1BB | SEQ ID NO: 17 | 5.3 | 53.52 | 84.1 | 0.12 | 99.0 |
| AB006 | HSA | 4-1BB | SEQ ID NO: 18 | 6.7 | 277.62 | 98.8 | 0.44 | 94.6 |
| AB007 | HSA | 4-1BB | SEQ ID NO: 19 | 2.5 | 185.52 | 80.7 | 0.49 | 93.0 |
| **AD001** | HSA | KRAS G12D | SEQ ID NO: 20 | 22.0 | 8.40 | 94.1 | 8.12 | 97.3 |
| **AD002** | HSA | KRAS G12D | SEQ ID NO: 21 | 58.62 | 1.30 | 96.7 | 0.55 | 93.6 |
| **AD003** | HSA | KRAS G12D | SEQ ID NO: 22 | 49.81 | 13.01 | 91.2 | 13.79 | 91.3 |
| AD004 | HSA | KRAS G12D | SEQ ID NO: 23 | 72 or longer | 190.44 | 89.7 | 16.59 | 97.4 |
| AD005 | HSA | KRAS G12D | SEQ ID NO: 24 | 30.1 | 121.90 | 94.9 | 22.92 | 92.8 |
| AD006 | HSA | KRAS G12D | SEQ ID NO: 25 | 17.4 | 938.84 | 75.9 | 77.56 | 93.3 |
| AD007 | HSA | KRAS G12D | SEQ ID NO: 26 | 12.1 | 535.83 | 97.0 | 151.68 | 98.4 |
| AD008 | HSA | KRAS G12D | SEQ ID NO: 27 | 17.2 | 66.86 | 96.6 | 286.65 | 97.1 |
| **AD009** | HSA | KRAS G12D | SEQ ID NO: 28 | 72 or longer | 0.65 | 82.6 | 0.51 | 82.7 |
| AD010 | HSA | KRAS G12D | SEQ ID NO: 29 | 53.73 | 4.49 | 99.0 | 8.18 | 97.7 |
| AD011 | HSA | KRAS G12D | SEQ ID NO: 30 | 72 or longer | 5.46 | 99.0 | 9.46 | 97.6 |
| AD012 | HSA | KRAS G12D | SEQ ID NO: 31 | 10.8 | 28.74 | 98.9 | 23.09 | 99.2 |
| AD013 | HSA | KRAS G12D | SEQ ID NO: 32 | 11.12 | 47.02 | 99.7 | 3.99 | 98 |
| AD014 | HSA | KRAS G12D | SEQ ID NO: 33 | 10.89 | 28.78 | 98.6 | 1.95 | 95 |
| AD015 | HSA | KRAS G12D | SEQ ID NO: 34 | 10.65 | 36.84 | 98.6 | N/A | N/A |
| **AD032** | HSA | KRAS G12D | SEQ ID NO: 42 | 72 or longer | 0.68 | 96.3 | 15.51 | 95.0 |
| AD043 | HSA | KRAS G12D | SEQ ID NO: 162 | 72 or longer | 4.18 | 98.3 | 263.23 | 79.0 |
| AD044 | HSA | KRAS G12D | SEQ ID NO: 163 | 48.7 | 2.93 | 98.8 | 1577.76 | 80.5 |
| AD045 | HSA | KRAS G12D | SEQ ID NO: 164 | 45.53 | 0.73 | 99.6 | 20.03 | 97.0 |
| AD046 | HSA | KRAS G12D | SEQ ID NO: 165 | 18.26 | 0.65 | 94.9 | 5.38 | 88.0 |
| AD050 | HSA | KRAS G12D | SEQ ID NO: 169 | 72 or longer | 5.55 | 92.1 | 454.57 | 95.0 |
| AD051 | HSA | KRAS G12D | SEQ ID NO: 170 | 34.0 | 5.95 | 97.4 | 514.12 | 94.5 |
| AD052 | HSA | KRAS G12D | SEQ ID NO: 171 | 14.29 | 3.06 | 97.7 | 4.93 | 95.0 |
| AD053 | HSA | KRAS G12D | SEQ ID NO: 172 | 72.00 | 1.37 | 99.4 | 1.03 | 93.7 |
| **AD034** | HSA | KRAS G12D | SEQ ID NO: 43 | 9.3 | 0.59 | 96.9 | 472.54 | 91.1 |
| **AV001** | HSA | KRAS G12V | SEQ ID NO: 119 | 72 or longer | 11.21 | 80.8 | 58.01 | 93.3 |
| **TD001** | TF | KRAS G12D | SEQ ID NO: 149 | 72 or longer | No binding | No binding | N/A | N/A |
| TD003 | TF | KRAS G12D | SEQ ID NO: 151 | 13.30 | 141.29 | 78.5 | 2720.49 | 94.2 |
| **TD005** | TF | KRAS G12D | SEQ ID NO: 153 | 72.00 | No binding | No binding | No binding | No binding |
| TD006 | TF | KRAS G12D | SEQ ID NO: 154 | 72.00 | 967.50 | 82.3 | 628.34 | 91.2 |
| TD007 | TF | KRAS G12D | SEQ ID NO: 155 | 7.5 | 431.36 | 84.0 | 2118.95 | 84.3 |
| **TD009** | TF | KRAS G12D | SEQ ID NO: 157 | 11.745 | No binding | No binding | 2481.58 | 92.6 |
| TD012 | TF | KRAS G12D | SEQ ID NO: 160 | 18.10 | 14.92 | 84.0 | 1311.19 | 96.1 |
| TD013 | TF | KRAS G12D | SEQ ID NO: 161 | 14.0 | 645.60 | 92 | 1545.48 | 94.0 |

| | | | | | | | | |
|---|---|---|---|---|---|---|---|---|
| *In the test shown in Table 3, nucleic acid ligands having biotin conjugated to the 5'-end thereof were used. | | | | | | | | |

### [Example 8] Additional tests on nucleic acid ligands targeting KRAS proteins

### 1. Serum stability and Dnase stability against target proteins

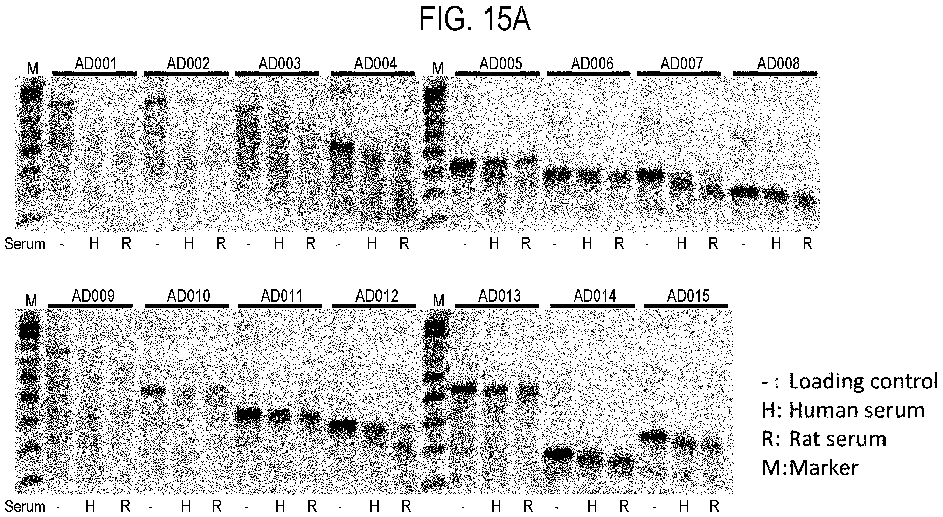

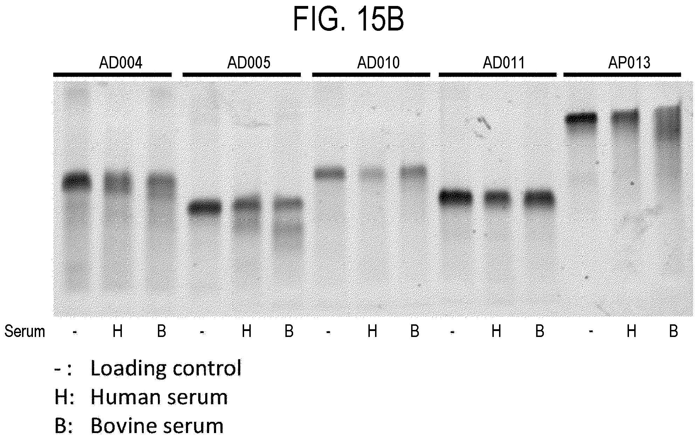

1-1. The same method as in Example 6 was conducted, wherein the nucleic acid ligands were reacted with sera for 24 hours and then subjected to electrophoresis. The stability of the nucleic acid ligands in various animal-derived sera was tested. The reason was that nucleic acid ligands showing stability in both animal and human sera need to be selected in order to expect the effects in animal tests to be also exhibited in humans. The HSA/KRAS-targeted nucleic acid ligands AD001 to AD015 were tested for stability, and the electrophoresis results are shown in FIGS. 15A and 15B. Additionally, AP013, an HSA/PD-L1-targeted nucleic acid ligand, was also tested.

As shown in FIGS. 15A and 15B, the nucleic acid ligands according to one embodiment of the present disclosure were stably maintained without being degraded in the human-derived serum, and particularly, the nucleic acid ligands, such as AD005, AD008 and AD011, showed excellent stability in various serum conditions, such as human serum, bovine serum, and rat serum. The above results confirmed that the nucleic acid ligands according to one embodiment of the present disclosure bound to plasma proteins and were stably maintained in sera.

1-2. The nucleic acid ligand AD005 having the excellent effect was tested for Dnase stability against human serum albumin and various KRAS mutant proteins, by the same method as in Example 4. KRAS G12D, KRAS G12C, KRAS G12V, and wild-type KRAS were used as KRAS proteins. In the test, the molar ratios of a nucleic acid ligand and a target protein were 1:1 and 1:5, respectively.

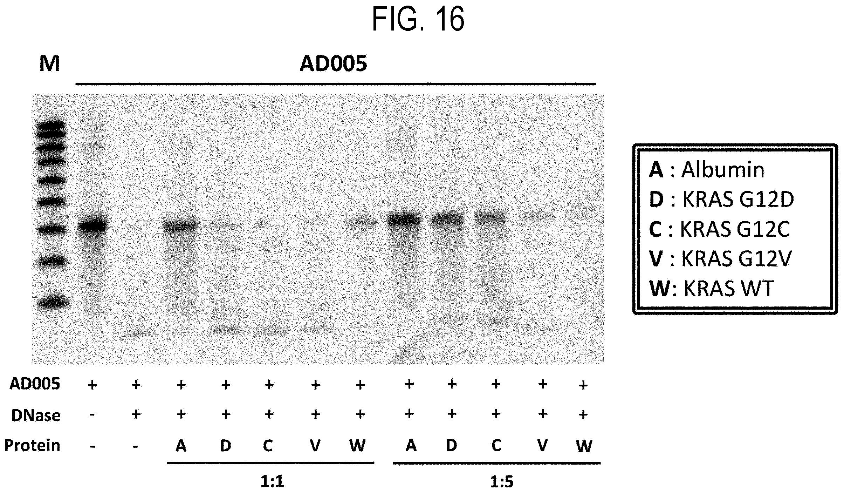

As a result, the nucleic acid ligand AD005 specifically binding to HSA/KRAS G12D according to one embodiment of the present disclosure, when incubated with KRAS G12D or KRAS G12C, showed resistance to a nuclease even by treatment with DNase (see FIG. 16). The reason why AD005 also bound to KRAS G12C is that G12D and G12C have very similar structures due to a mutation at one position. AD005 showed excellent resistance to a nuclease even when reacted with human serum albumin as one of targets. In addition, AD005 showed increased resistance to a nuclease as the molar proportion of a target protein increased, and the reason was that as the amount of the target protein increased, the nucleic acid ligand can bind more to the target protein, thereby avoiding the degradation by the nuclease.

However, the nucleic acid ligand was all degraded by DNase action when reacted with KRAS G12V as another mutant or the wild-type KRAS protein. This indicates that a nucleic acid ligand according to one embodiment of the present disclosure exhibits strong binding affinity to only some KRAS mutants (G12D and G12C), and as a result, showed increased stability against the nuclease. Furthermore, the nucleic acid ligands according to one embodiment of the present disclosure showed selective binding to KRAS proteins depending on the presence or absence of the mutation of KRAS protein.

### 2. Serum half-life test

To determine the half-life of nucleic acid ligands in the human serum, a test was conducted. In the test, two types of nucleic acid ligands (AD005 and AD011) that were evaluated to have excellent Dnase stability results and one type of aptamer (KD003) not binding to plasma proteins as a control were used.

The nucleic acid sequence of the aptamer KD003 was ACCSGSSGCA GCACCSASSCASACGGACGCSCSCS (SEQ ID NO: 184). Each part denoted as 5 indicates 5-(N-benzylcarboxamide)-2'-deoxyuridine (Bz-dU).

First, 10 µl of the nucleic acid ligand or the aptamer KD003 at a concentration of 500 µg/ml was mixed with 90 µl of the human serum and reacted at 37°C for 0.5, 1, 2, 4, 6, 10, and 24 hours. Upon the completion of the reaction, 50 µl of HFIP (1,1,1,3,3,3-hexafluoro-2-propanol; Sigma-Aldrich, Germany) was added to each reaction solution, stirred, and centrifuged at 13,000 rpm for 5 minutes to obtain a supernatant. Until the 24-hour-treated sample was completed, the other samples were stored at 4°C, and when all the reactions were completed, the samples were loaded on an ultra-performance liquid chromatography (UPLC) instrument and analyzed by injection of 5 µL. The Waters ACQUITY UPLC H-Class Bio System (Water Corporation, USA) was used as the UPLC instrument, and equipped with ZORBAX RR Eclipse Plus C18, 95 Å, 3.5 µm, 4.6 × 100 mm (Agilent Technologies, USA) as an analytical column. The flow rate was 1.7 mL/min, and analysis was performed by a gradient method using 15 mM TEA and 38 mM HFIP in water as mobile phase A and acetonitrile as mobile phase B. The nucleic acid ligands or aptamer was detected by a PDA (UV 260 nm) detector.

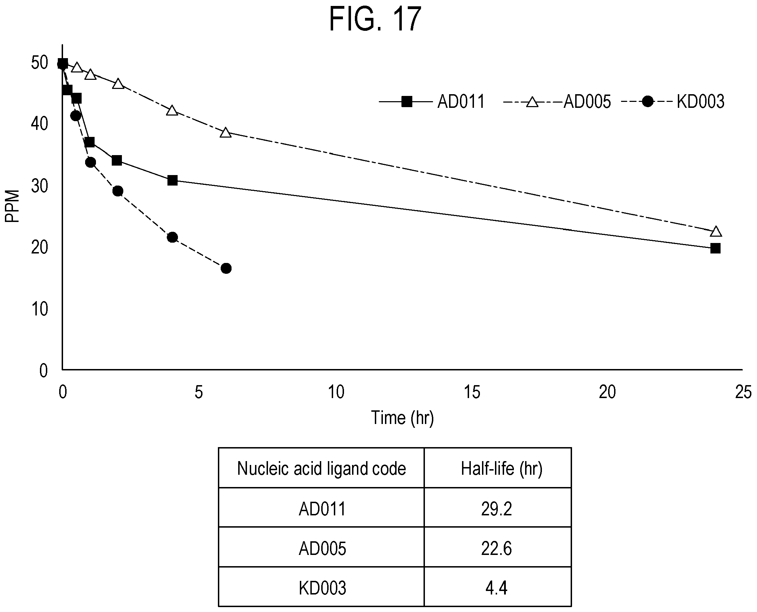

As a result, the nucleic acid ligands according to one embodiment of the present disclosure had a half-life that was at least about 5-fold longer than that of the control nucleic acid aptamer (KD003) not binding to the plasma protein (see FIG. 17). These results indicate that the nucleic acid ligands according to one embodiment of the present disclosure show excellent binding affinity for plasma proteins and thus actually have excellent stability in the serum.

### 3. Target binding affinity

The specific binding affinities of the nucleic acid ligands for the KRAS mutant proteins were determined by the following method (Test 1) and the method in Example 5 (Test 2).

3-1. [Test 1] The binding affinities of the nucleic acid ligands were measured using a bio-layer interferometry (BLI) method. BLI is a test method of monitoring the binding action between molecules by measuring the interference of light caused by the thickness change of a protein layer adsorbed to the end of a biosensor, and the test was conducted using Octet Red 96e equipment (ForteBio, USA). First, a nucleic acid ligand having biotin conjugated to the 5'-end thereof was reacted in buffer SB18 from 95°C to 37°C, gradually lowered by 1°C, for 10 seconds at each temperature, thereby inducing the tertiary structure of the nucleic acid ligand. The binding affinity was measured by the following five steps of: (1) measuring a baseline in buffer SB18 by using a streptavidin-coated biosensor (60 seconds); (2) binding the nucleic acid ligand prepared by the above-described method to the streptavidin biosensor (180 seconds); (3) measuring a second baseline in buffer SB 18 (120 seconds); (4) preparing a target protein in buffer SB18 and allow the target protein to interact with the nucleic acid ligand bound to the biosensor (300 seconds); and (5) finally, inducing the dissociation of the target protein in buffer SB18 (600 seconds). All the binding affinity measurement steps were performed at 37°C, and the nucleic acid ligands were used with 500 nm and the target proteins were used with 500 nM to 2-fold dilutions. Specifically, the binding affinities of the nucleic acid ligand AD005 (SEQ ID NO: 24), showing excellent results in the above test, for the KRAS proteins (KRAS G12D, KRAS G12V, KRAS G12C, and wild-type KRAS) were analyzed to determine the specific binding affinities of the nucleic acid ligands for KRAS of various traits.

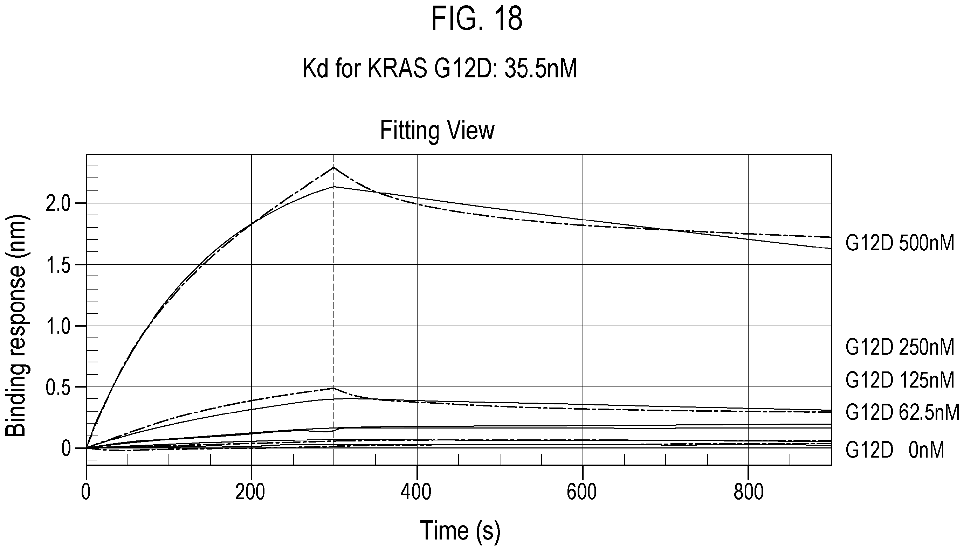

As a result, the dissociation constant value of the nucleic acid ligand AD005 was measured to be 35.5 nM for KRAS G12D, but not significant for the wild-type KRAS (KRAS WT), and another type of mutant protein (KRAS G12V) within the test concentration range, and thus the binding affinity of AD005 is predicted to be low (see FIG. 18).

3-2. [Test 2] The binding affinities (K_{d}) of the nucleic acid ligand AD005 for the KRAS proteins (KRAS G12D, KRAS G12V, KRAS G12C, and wild-type KRAS) were determined by the same method as in Example 5. As a result, the dissociation constant value of the nucleic acid ligand AD005 was measured to be 22.92 nM for KRAS G12D and 2.50 M for KRAS S12C, but not significant for the wild-type KRAS (KRAS WT) and another type of mutant protein (KRAS G12V) within the test concentration range, and thus the binding affinity of AD005 was predicted to be low.

The results of Tests 1 and 2 are summarized in Table 4 below.

**TABLE 4**

| | KRAS WT | KRAS G12D | KRAS G12C | KRAS G12V |
|---|---|---|---|---|
| Test 1 | No Binding | 35.50 nM | - | No Binding |
| Test 2 | No Binding | 22.92 nM | 2.50 nM | No Binding |

According to the results of Test 1 and 2, the nucleic acid ligand AD005 according to one embodiment of the present disclosure exhibited relatively strong binding affinities for KRAS G12D and KRAS G12C, but showed no or very weak binding affinities for wild-type KRAS (KRAS WT) and the other type of mutant protein (KRAS G12V). These results are consistent with the Dnase stability test results in Section 1.

### [Example 9] In vitro efficacy of KRAS G12D-targeted nucleic acid ligands

AD005 and AD011 were finally selected as nucleic acid ligands targeting KRAS G12D protein present in cancer cells through the above test, and the in vitro efficacy test was conducted below.

### 1. Cell culture

All cell lines were cultured at a 37°C in a 5% CO₂ incubator. The AsPC-1 pancreatic cancer cell line was purchased from ATCC (USA) and used for tests. The cell line was cultured using a medium containing 10% FBS and a 1% antibiotic in ATCC modified RPMI-1640 (Thermo Scientific, USA). The medium was exchanged once every 2-3 days, and for subculture, the cells were washed with PBS, and then cultured at 37°C for 1 minute after addition of 0.05% trypsin-EDTA, and thereafter, trypsin was inactivated by addition of a fresh medium, and cultured after passage at a ratio of 1:5 in a new culture vessel. The MIA PaCa-2 pancreatic cancer cell line was also purchased from ATCC and used for tests. The cell line was cultured using a medium containing 10% FBS and a 1% antibiotic in DMEM. The medium exchange and subculture were performed by the same method as in AsPC-1. All the cells were periodically checked for the contamination with mycoplasma by PCR using a kit (Biomax, Korea), and in vitro tests were conducted using only cells with no contamination detected.

### 2. Entry of nucleic acid ligands into cytoplasm

The KRAS G12D protein was present in cancer cells, and thus a nucleic acid ligand needs to enter the cytoplasm to act on this protein. The delivery of the prepared nucleic acid ligands into cells was determined by treating the cells with the nucleic acid ligands covalently linked to a fluorescent substance and checking the presence and location of fluorescence expression. For the fluorescent substance, tetramethylrhodamine (TMR), carboxyfluorescein (FAM), and fluorescein isothiocyanate (FITC) were used.

First, each well of a 24-well plate was covered with a 10-mm circular cover glass, disinfected with 500 µl of 70% ethanol, and then sterilized under UV for 30 minutes. The coverslip that has been sterilized was washed with PBS, and then MIA PaCa-2 cells were added at 4-10 × 10⁴/well and cultured.

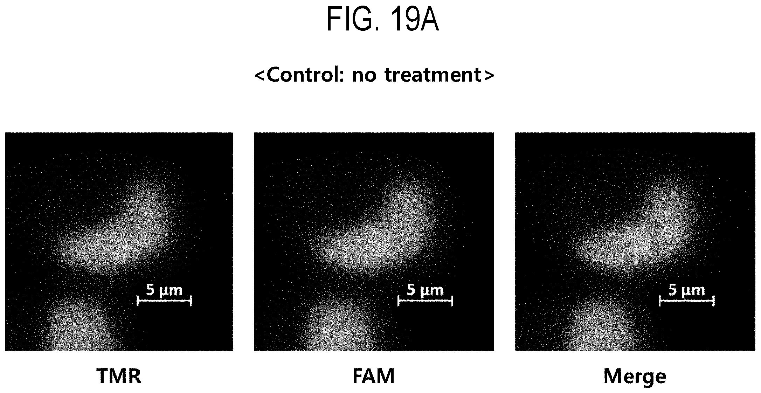

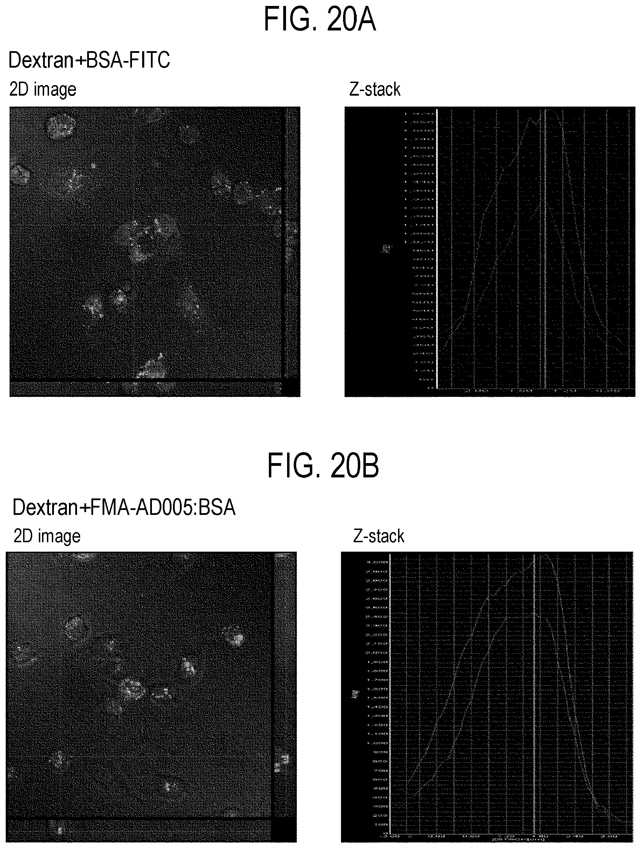

The cells were cultured in a cell incubator for 48 hours to adhere well to the cover glass, followed by exchange with a FBS- and glutamine-free medium, and then cultured for 16 hours. Thereafter, the cells were treated with 1 mg/ml of 70 KDa dextran-TMR, 2 nmol of BSA-FITC, and FAM-linked nucleic acid ligand conjugated to BSA albumin (Sigma-Aldrich, USA, product number: A8806) and cultured for 1 hour. Upon the completion of the culture, the cells were washed five times with cold PBS on ice and treated with 4% paraformaldehyde for 30 minutes for cell fixation. The cells were again washed three times with PBS, and then the cell nuclei were stained by addition of DAPI (4',6-diamino-2-phenylindole) and culturing for 30 minutes, and thereafter, the cells were again washed three times with PBS. A drop of mount media was added onto the slide glass, and PBS was removed from the cover glass on which the cells were grown, and then the cover glass was allowed to stand on the mount media of the slide glass, thereby preparing a sample. The completed sample was observed under a confocal microscope (FV3000RS Olympus, Japan) at a magnification of 100X by using a filter for each fluorescent substance, and the results are shown in FIGS. 19A to 19D and FIGS. 20A and 20B.

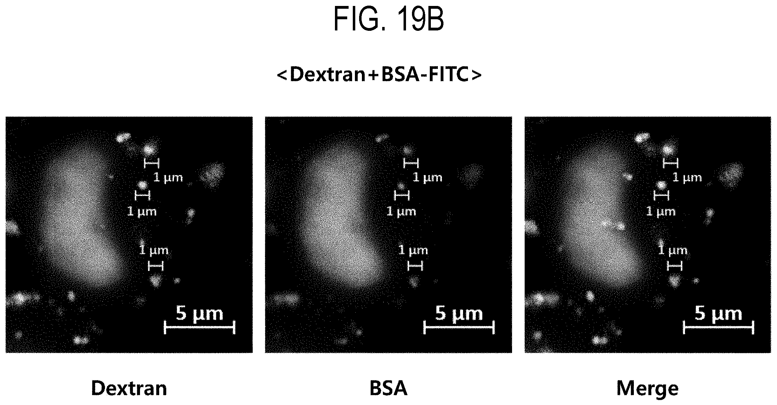

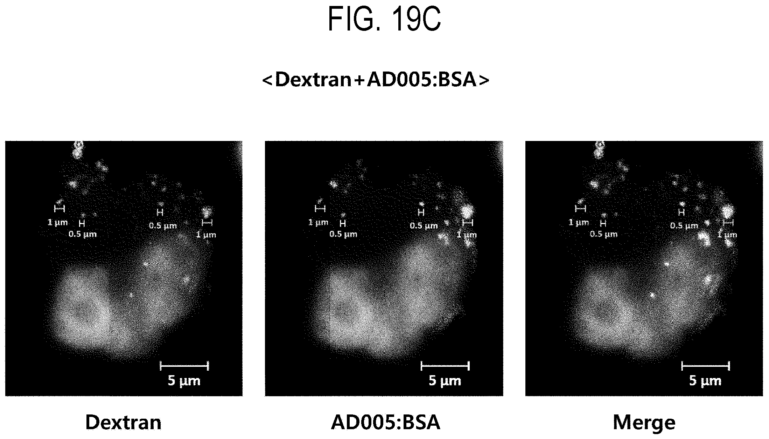

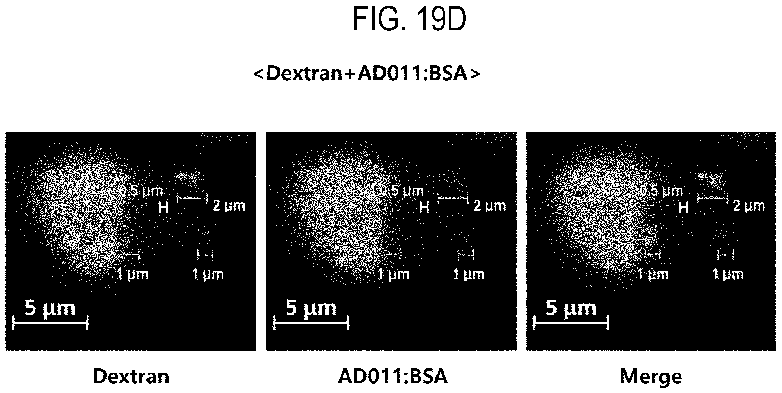

As a result of microscopic observation of MIA PaCa-2 cells, a large amount of fluorescent-labeled albumin (BSA-FITC) entered the cells under the nutritional deficiency test conditions (FIG. 19B). Particularly, the albumin entering the cytoplasm was observed as a cluster with a diameter of about 1 µm and colocalized with 70 KDa dextran-TMR as a macropinocytosis marker (FIG. 19B). This indicates that most albumin entered the cells by macropinocytosis under nutritional deficiency test conditions. As for the FAM-nucleic acid ligand reacted with the albumin protein, instead of the fluorescent-labeled albumin, the FAM fluorescence linked to the nucleic acid ligand was confirmed in the cells, and the presence of macropinosomes of 0.2 µm to 5 µm was confirmed (FIGS. 19C and 19D). Such macropinosomes were also confirmed to be colocalized with 70 KDa dextran-TMR.

The three-dimensional analysis of the intracellular position through Z-axis division also confirmed, more clearly, the colocalization of the macropinocytosis marker 70 KDa dextran-TMR and fluorescent-labeled albumin or dextran-TMR and fluorescent-labeled nucleic acid ligand (FIGS. 20A and 20B).

The above results confirmed that the nucleic acid ligands according to one embodiment of the present disclosure can be delivered into cells by macropinocytosis through the binding with albumin.

### 3. Cell growth inhibitory effect of nucleic acid ligands

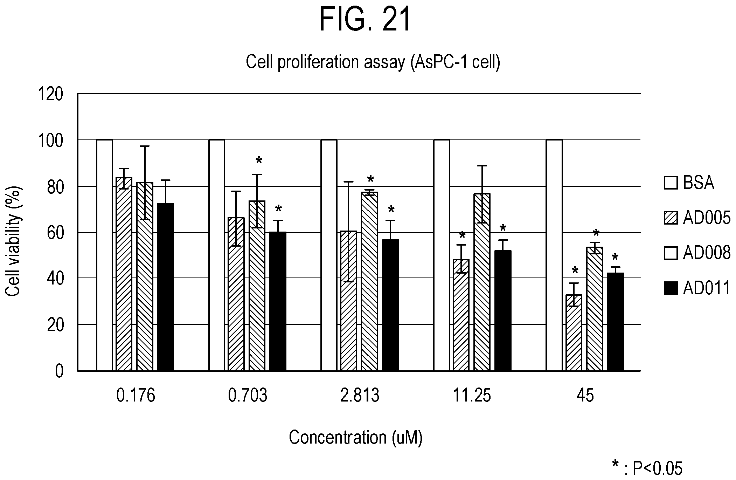

The cell growth inhibitory effect by the treatment with nucleic acid ligands was determined by WST assay. This method utilizes the property that WST, treated to cells, changes into formazan, which has orange color, by a dehydrogenase present in the mitochondrial electron transport system of the cells.
(1) Specifically, KRAS mutant protein-expressing cancer cells (2 × 10⁴ cells/well for AsPC-1 cells and 8 × 10³ cells/well for MIA PaCa-2 cells) were cultured for 24 hours to adhere to each well of a 96-well plate, followed by exchange with a medium not containing FBS and glutamine, and then cultured for 24 hours to activate macropinocytosis. Thereafter, the cells were treated with a nucleic acid ligand conjugated to a plasma protein or a nucleic acid ligand not conjugated to a plasma protein by the above-described method, followed by culturing for 24 hours, and then, 10 µl of a WST (Dongin LS, Korea) reagent was added to each well, followed by culturing for 2 hours, and then the produced formazan was measured by absorbance at 450 nm using a GloMax plate meter (Promega, USA). In the present test, the nucleic acid ligands AD005, AD011, and AD008 were used to determine the cell growth inhibitory effect.
(2) [Test 1] In the first test, the growth inhibitory effect on AsPC-1 cells was determined. The cells were treated with BSA for a control and the nucleic acid ligands (AD005, AD008, and AD011) conjugated to the plasma protein BSA for test groups. As a result, the treatment with all the nucleic acid ligands according to one embodiment of the present disclosure significantly reduced cell viability in a dose-dependent manner compared with the control treated with only BSA (see FIG. 21).

[Test 2] In the second test, the growth inhibitory effects of both AsPC-1 cells and MIA PaCa-2 cells were determined. The cells were treated with no nucleic acid ligand for a control and the nucleic acid ligand with or without BSA conjugated for test groups. To compare the effect with that of the treatment with a single target nucleic acid ligand, the cells were also treated with the aptamer KD006 with or without BSA conjugated, in the same manner as the above.

The nucleic acid sequence of the aptamer KD006 was G5G5CACC5G55GCAGCACC5A55CA5ACGGACGC5C5C5 (SEQ ID NO: 185). Each part denoted as 5 indicates 5-(N-benzylcarboxamide)-2'-deoxyuridine (Bz-dU).

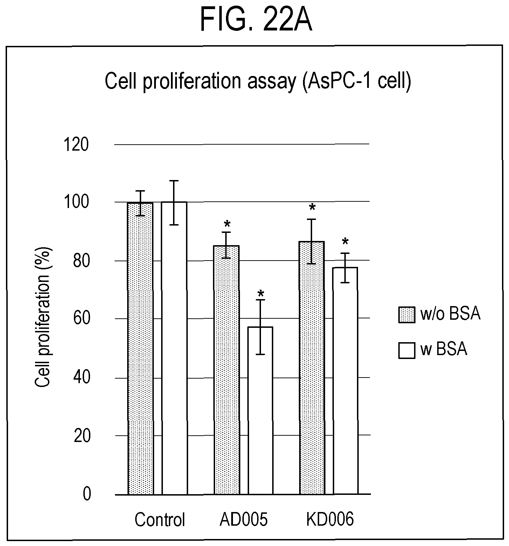

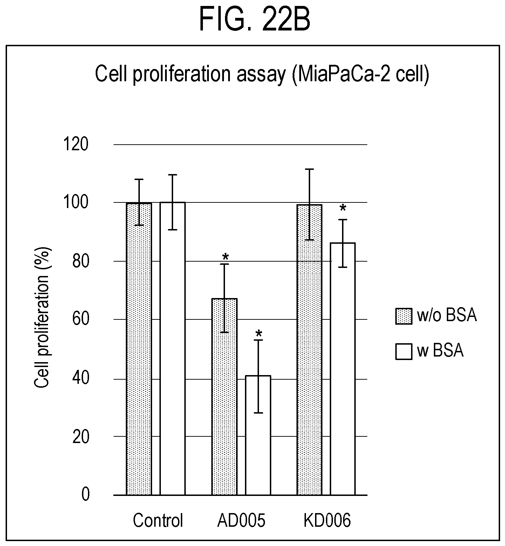

As a test result, the nucleic acid ligand according to one embodiment of the present disclosure had a significant cell growth inhibitory effect compared with the aptamer KD006, with a single target (see FIGS 22A and 22B).

The results of the first and second tests confirmed that AD005 showed a significant reduction in the cell growth inhibitory effect, in a dose-dependent manner, on AsPc-1 cells as a KRAS G12D mutant cell line and MiaPaCa-2 cells as a KRAS G12C mutant cell line, and increased the inhibitory effect when used with the plasma protein. This indicates that AD005, a nucleic acid ligand according to one embodiment of the present disclosure, significantly inhibited cell growth by binding to a plasma protein and a KRAS mutant protein.

(3) [Test 3] In an additional test, it was evaluated whether the treatment with an endocytosis inhibitor (NaN₃) also showed a cell growth inhibitory effect. In the test, AsPC-1 cells were used. The same method as in Section (1) above was conducted, and as for groups treated with the endocytosis inhibitor, the cells were first treated with the inhibitor and, 30 minutes later, treated with respective test substances. The cell growth rate was expressed as a percentage of the absorbance of each treatment group compared with a control (treated with only BSA).

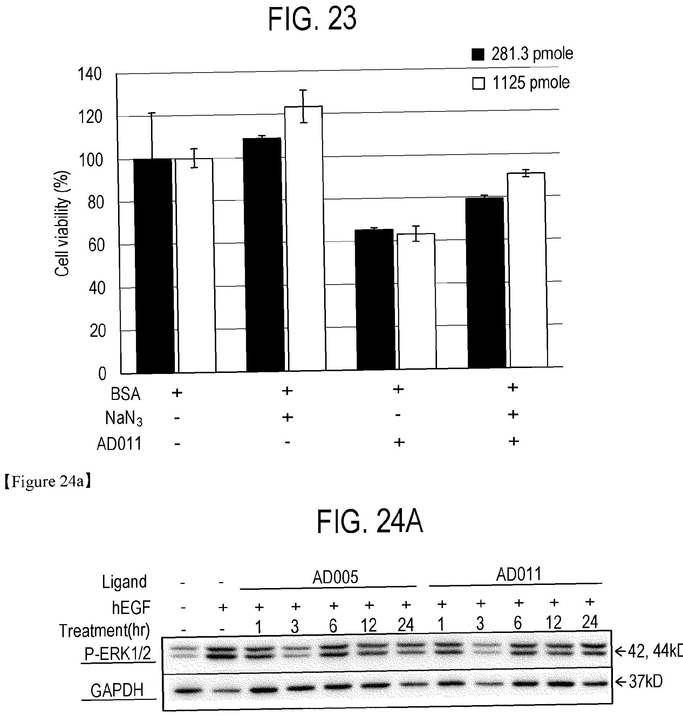

When the cells were co-treated with the endocytosis inhibitor (NaN₃) and the BSA albumin-nucleic acid ligand, the cell viability inhibition by the nucleic acid ligand was significantly recovered to some extent. These results indicate that the nucleic acid ligands according to one embodiment of the present disclosure entered the cells by various endocytosis pathways as well as macropinocytosis (see FIG. 23).

### 4. Intracellular signaling (phosphorylation) inhibitory effect of nucleic acid ligand

It was assumed that the above-described cell growth inhibitory effect by the treatment with nucleic acid ligands specifically binding to a plasma protein and a KRAS mutant was induced by inhibition of the KRAS signaling pathway activated in cancer cells. To prove this, it was determined whether the phosphorylation of extracellular signal-regulated kinase (ERK) downstream of the KRAS signaling pathway was inhibited.

KRAS mutant proteins, when activated by stimulation of a growth factor such as epidermal growth factor (EGF), continuously maintain an activated state thereof to induce the phosphorylation of the ERK protein downstream of the signaling pathway. It was expected that when a nucleic acid ligand delivered into the cells binds to the KRAS mutant protein to inhibit the signaling process, the phosphorylation of the ERK protein downstream thereof would be reduced, and this was proved by Western blotting.

Specifically, cells were added to a 35-mm dish (3 × 10⁵ AsPC-1 cells being added) and cultured to adhere thereto, followed by exchange with a medium not containing FBS or glutamine, and then the cells were cultured for 16 hours to activate macropinocytosis. The cells were treated with a plasma protein-conjugated nucleic acid ligand prepared by the above-described method, and then cultured for 3 hours. The cells were treated with 200 ng/ml human EGF protein (Merck, Germany) 15 minutes before the end of culturing to activate the KRAS signaling pathway. No treatment was made for a negative control, and the treatment with EGF only was made for a positive control.

The cells were washed three times with cold PBS and then collected by a scrapper, and the cells were collected by centrifugation at 3,000 × g and 4°C for 5 minutes. A radioimmunoprecipitation assay (RIPA) buffer containing 1X phosphatase inhibitor and 1X protease inhibitor was added to the collected cells, and the cells were well dispersed, and then reacted on ice for 30 minutes to remove the cell membrane. After the reaction, the cell liquid was centrifuged at 15,000 × g and 4°C for 30 minutes to collect proteins, and the proteins were quantified by bicinchoninic acid assay (BCA). The proteins extracted and quantified from the cells were mixed with an SDS loading dye and reacted at 85°C for 10 minutes to prepare a sample for analysis. After 15µg of the proteins for each sample was loaded on the 10% SDS-PAGE gel, electrophoresis was performed at 50 V for 15 minutes and at 150 V for 1 hour at 4°C. The proteins on the gel that have been electrophoresed were transferred to a polyvinylidene fluoride (PVDF) membrane (Bio-Red, USA) activated with methanol, wherein the transfer was performed at 250 mA for 2 hours at 4°C. After the completion of the transfer, the membrane was washed three times with distilled water, and non-specific proteins were blocked by reaction in a TBS-T buffer solution containing 5% skim milk for 1 hour. The cells were reacted with primary antibodies ERK, p-ERK1/2, and GAPDH (Cell Signaling Technologies, USA) diluted to 1/1000, at 4°C for 16 hours, followed by washing three times with TBS-T, and then the cells were reacted with rabbit IgG-HRP antibody (Santa Cruz Biotechnology, USA) diluted to 1/2000, at room temperature for 1 hour. The cells were washed three times with TBS-T and treated with a chemiluminescence solution, and expression of each protein was imaged using Fusion Solo (Vilber, France) imaging equipment. In each test, the phosphorylated protein (p-ERK) was detected, and then the antibodies on the membrane were removed using a stripping buffer. The membrane was washed three times with TBS-T, and then again subjected to skim-milk blocking, GAPDH primary antibody response, and HRP secondary antibody response as above, and then the internal standard protein was detected using a chemiluminescent solution. The band intensity of the phosphorylated protein (p-ERK) was normalized to each GAPDH.

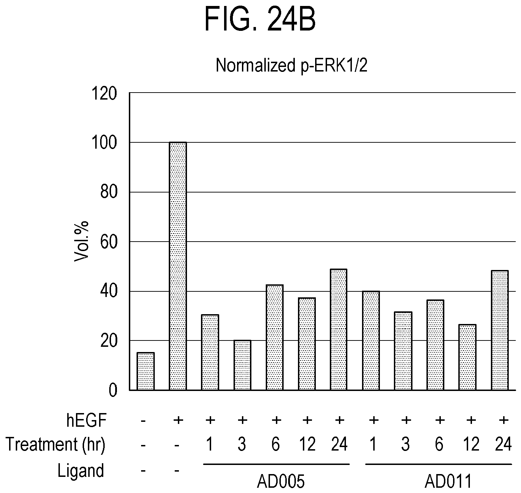

As a result, the ERK phosphorylation of pancreatic cancer cells by EGF treatment was significantly inhibited by treatment with albumin- and KRAS mutant protein-conjugated nucleic acid ligands. In addition, the nucleic acid ligands AD005 and AD011 continuously exhibited an excellent phosphorylation inhibitory effect from 1 hour to 24 hours after treatment (see FIGS. 24A and 24B). Through these results, one could predict that the nucleic acid ligands according to one embodiment of the present disclosure can prevent or treat a disease caused by the KRAS signaling pathway by binding to KRAS, a therapeutic target in cells, to inhibit the phosphorylation of downstream proteins.

Through the above in vitro results, it was confirmed that the nucleic acid ligands according to one embodiment of the present disclosure can exhibit a therapeutic effect through a pathway as shown in FIG. 25. In order to determine whether this effect was also shown in vivo, the following tests were conducted.

### [Example 10] In vivo efficacy of KRAS-targeted nucleic acid ligand

### 1. Materials and animals

10x Phosphate buffer saline (PBS), Dulbecco's modified eagle's medium (DMEM), fetal bovine serum (FBS), penicillin-streptomycin, and 0.05 % trypsin-EDTA were purchased from WelGene (Seoul, Korea). Matrigel matrix and gemcitabine hydrochloride were purchased from CORNINF (New York, USA) and Sigma Aldrich (St. Louis, USA), respectively. Balb/c-nu male mice (5 weeks old) were purchased from ORIENT BIO (Seongnam, Korea), and all animal experiments were performed according to the regulations of the Institutional Animal Care and Use Committee (IACUC) of Seoul National University.

### 2. Tumor inhibitory efficacy of nucleic acid ligand in pancreatic cancer xenograft animal model

First, a mouse pancreatic cancer tumor model (Balb/c-nu) was prepared. The MIA PaCa-2 cell line (1 × 10⁶ cells) was mixed with 100 µL of a Matrigel mixture (1xPBS: Matrigel = 50:50 vol.), and then subcutaneously injected (SC) into the right flank of the mice. When the tumor size increased to 30 mm³, PBS, the nucleic acid ligand according to one embodiment of the present disclosure and a positive control drug (Gemcitabine) were separately injected intravenously (IV) to compare the tumor inhibitory efficacy. Four experimental animals were prepared for each test group. The nucleic acid ligand AD005 (10 µg) was injected once every 2 days 7 times (Day 0, 2, 4, 6, 8, 12) and gemcitabine (1 mg) was injected once every 3 days 5 times (Day 0, 3, 6, 9, 12). The tumor size was measured every 2 days by using the equation (1/2 × longest diameter × shortest diameter). The body weight of the mice was measured every 2 days after drug administration.

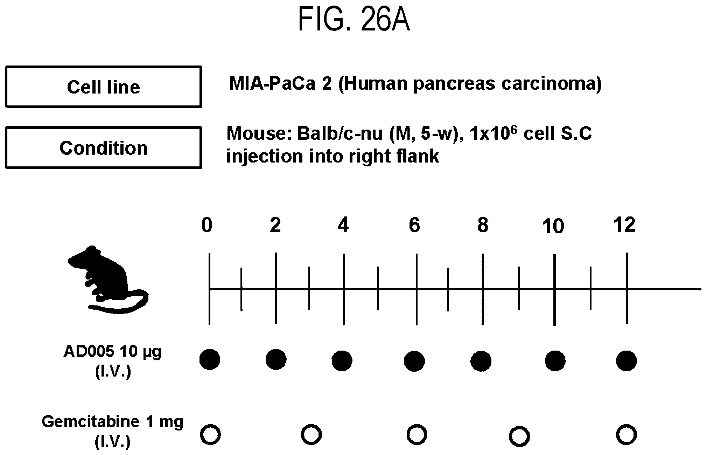

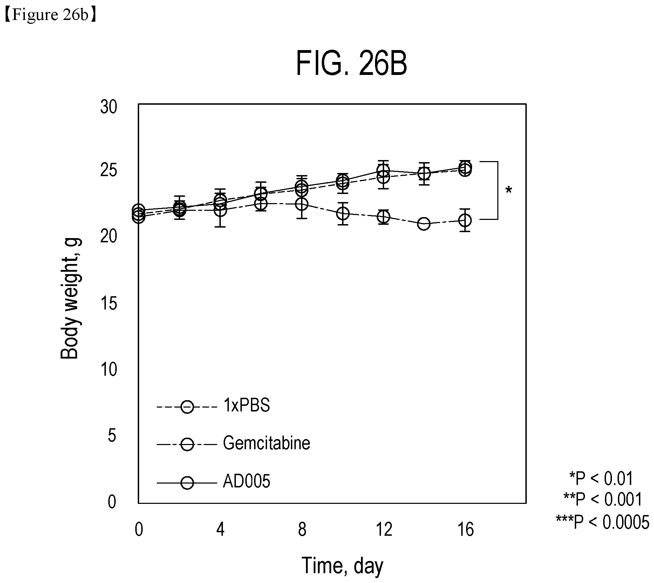

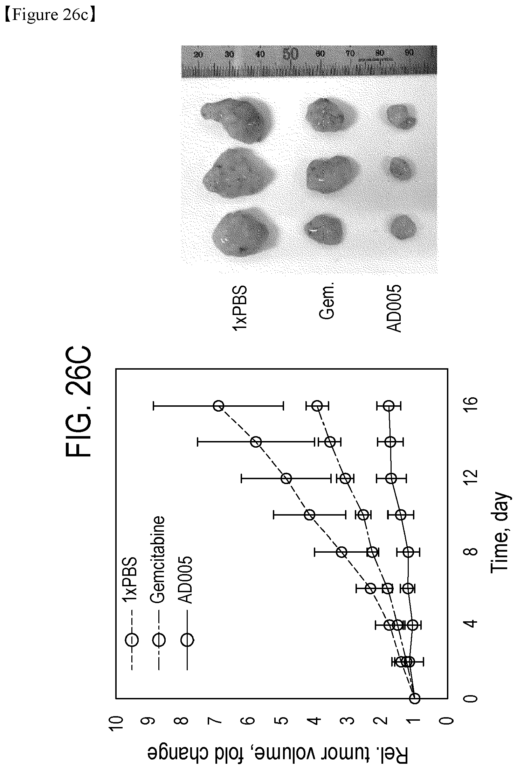

The nucleic acid ligand AD005 of the present disclosure, although administered at a much lower concentration (1/100 times) than the positive control drug (gemcitabine), showed a significantly superior tumor inhibitory effect compared with the positive control drug. In addition, the positive control drug (gemcitabine) showed a weight loss from 8 days after drug administration, but the nucleic acid ligand according to one embodiment of the present disclosure showed a normal increase in the body weight of the mice (see FIGS. 26A to 26C).

### 3. In vivo stability of nucleic acid ligand

24 Hours after drug administration in the test of Section 2 above, mice showing abnormal behavior were sacrificed and the organs were weighed to determine acute toxicity. In addition, chronic toxicity was determined by weighing the organs of the sacrificed mice after last drug injection.

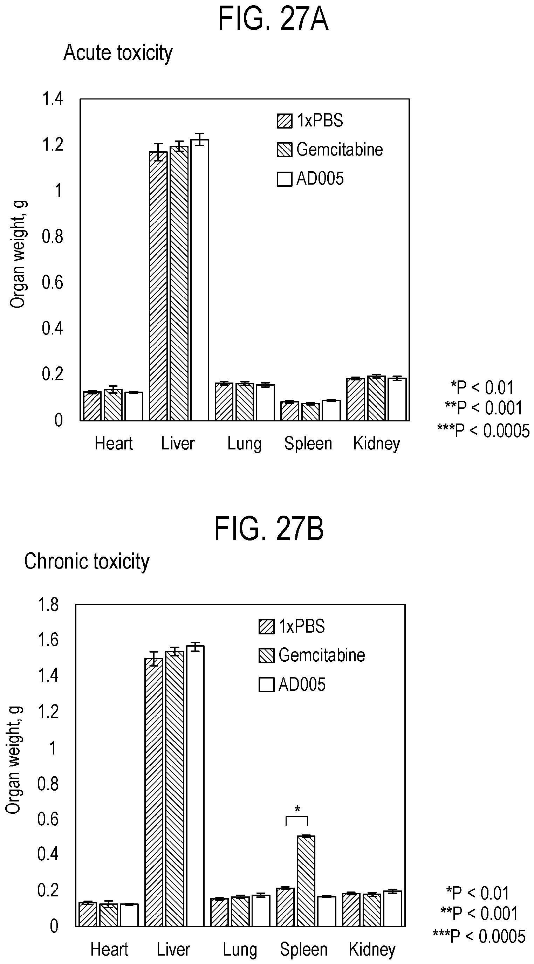

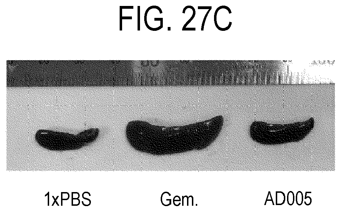

The positive control drug (gemcitabine) showed a serious side effect of spleen enlargement, but the nucleic acid ligand AD005 of the present disclosure showed no change in spleen size as in mice without drug injection. In addition, there were no severe side effects in the heart, liver, lung, kidney, and the like. These results indicate that the nucleic acid ligand according to one embodiment of the present disclosure exhibited higher tumor inhibitory efficacy than the positive control drug and had almost no toxicity or side effects (see FIGS. 27A and 27C).

### 4. Test for determining dose appropriate for optimal treatment by nucleic acid ligand - optimal dose for tumor inhibitory efficacy in pancreatic cancer xenograft animal model

First, a mouse pancreatic cancer tumor model (Balb/c-nu) was prepared in the same manner as in the test of Section 2 above. The MIA PaCa-2 cell line (1 × 10⁶ cells) was mixed with 100 µL of a Matrigel mixture (1xPBS: Matrigel = 50:50 vol.), and then subcutaneously injected into the right flank of the mice. When the tumor size increased to 30 mm³, PBS, the nucleic acid ligand (AD005) according to one embodiment of the present disclosure, and a positive control drug (gemcitabine) were separately injected intravenously (IV) to compare the tumor inhibitory efficacy. Four experimental animals were prepared for each test group. The nucleic acid ligand with different doses (5, 10, 20, and 50 µg) was injected once every 2 days 7 times (Day 0, 2, 4, 6, 8, and 12), and the positive control (1 mg) was injected once every 3 days 5 times (Day 0, 3, 6, 9, and 12). The tumor size was measured every 2 days by using the equation (1/2 × longest diameter × shortest diameter).

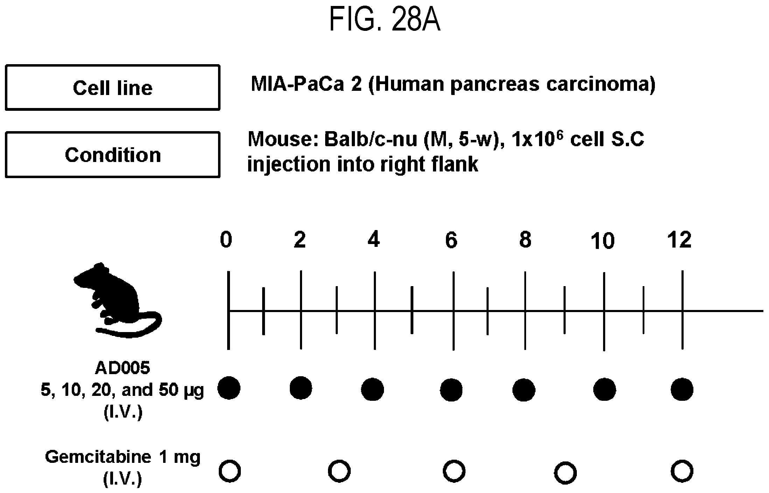

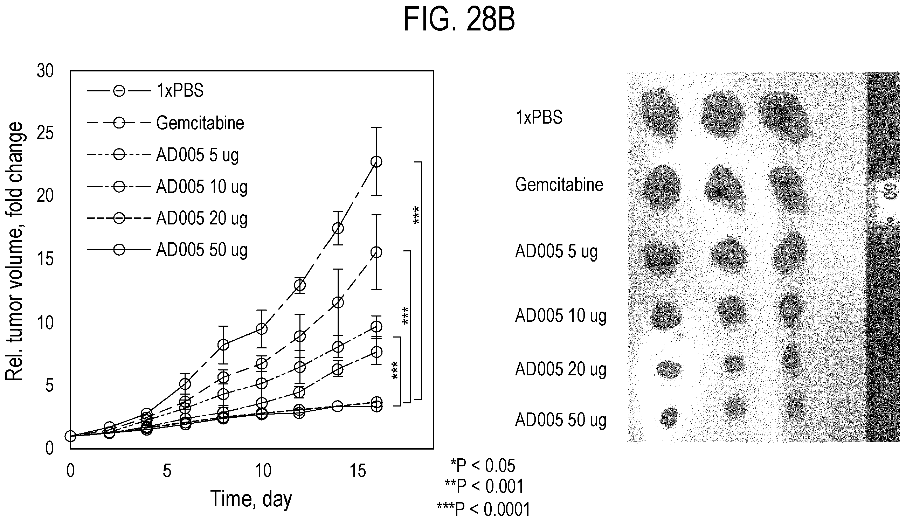

Compared with the positive control drug, the nucleic acid ligand according to one embodiment of the present disclosure exhibited a significantly excellent tumor inhibitory effect with an increasing dose. However, the administration of 20 µg and 50 µg of the nucleic acid ligand did not show a great difference in effect, indicating that the tumor inhibitory efficacy was not increased above a certain dose (see FIGS. 28A and 28B).

### 5. In vivo safety with dose of nucleic acid ligand

The mice sacrificed after the last drug injection in Section 4 above were measured for organ weights, and their blood was analyzed to determine the toxicity due to drug administration.

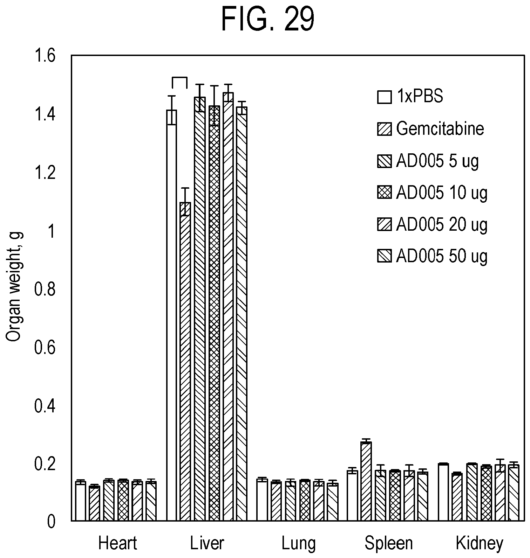

The positive control drug (gemcitabine) showed serious side effects of liver shrinkage and spleen enlargement. The nucleic acid ligand according to one embodiment of the present disclosure, even with an increasing dose, did not show changes in spleen and liver sizes as in the mice without drug injection. There were no significant side effects in heart, liver, lung, kidney, and the like (see FIG. 29).

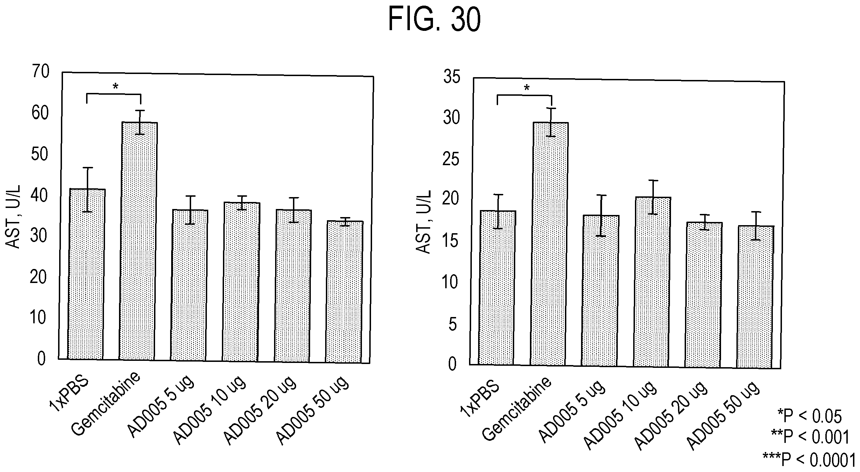

As a result of measuring liver damage indexes (AST and ALT), the positive control drug showed severe liver damage, but the nucleic acid ligand according to one embodiment of the present disclosure, even with an increasing dose, did not show changes in liver damage indexes as in the mice without drug injection (see FIG. 30).

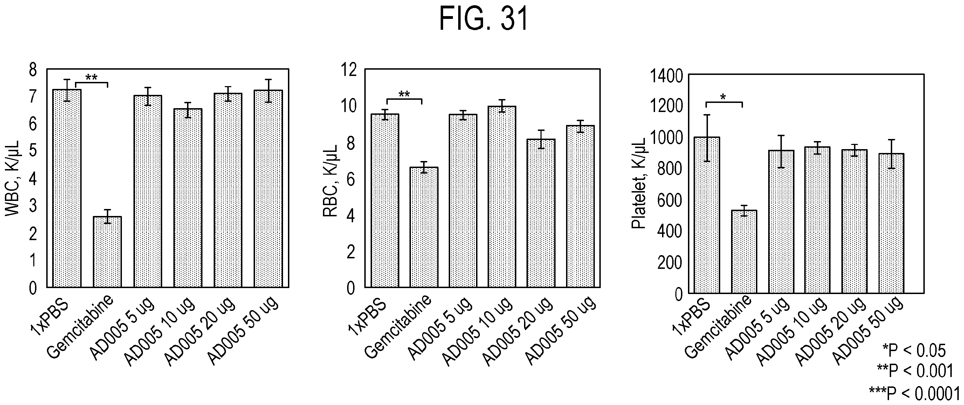

The positive control drug showed side effects of decreasing white blood cell (WBC), red blood cell (RED), and platelet levels, but the nucleic acid ligand according to one embodiment of the present disclosure showed no abnormal values with an increasing dose (see FIG. 31).

### [Test Example 8] Efficacy of PD-L1-targeted nucleic acid ligands

The PD-1/PD-L1 binding inhibitory effect of the prepared nucleic acid ligands binding to albumin and PD-L1 protein was determined by protein-based tests and cell-based tests.

The binding inhibition at the protein level was performed by ELISA using a purified protein-based PD-1/PD-L1 inhibitor screening assay kit (BPS, USA). The product included Immunobuffer 1, PD-1 protein, PD-L1 protein, a blocking buffer, and a chemiluminescent solution, which were used for tests. This system corresponded to an inhibitory activity measurement method designed such that the fluorescence signal decreased as the inhibitory activity increased, and a commercially available PD-L1 neutralizing antibody (BPS, USA) was used as a positive control.

First, the PD-L1 protein purified according to the manual of the product was immobilized onto a 96-well plate, and then each well was washed a total of three times with Immunobuffer 1 to remove unbound PD-L1 protein. The nucleic acid ligand according to one embodiment of the present disclosure was structurally stabilized for each test concentration in buffer SB 18, and thereafter, 5 µL of this nucleic acid ligand was added to each well having PD-L1 protein immobilized thereto, and then 20 µL of the biotin-conjugated PD-1 protein prepared according to the manual was added to each well. Each well was washed three times with Immunobuffer 1, and then 100 µL of streptavidin-HRP diluted in a blocking buffer according to the manual was additionally added, followed by reaction for 1 hour. After 100 µL of a chemiluminescent solution was added to each well with a supernatant removed therefrom, the fluorescence intensity was measured by a GloMax plate meter (Promega, USA) to determine the inhibition of PD-1/PD-L1 binding.

The inhibition of PD-1/PD-L1 binding at the cellular level was determined by a cell-based PD-1/PD-L1 inhibition bioassay (Promega, USA). This is a luciferase reporter-based assay wherein PD-1- and luciferase gene-expressed Jurkat effector cells and TCR activator- and PD-L1-expressed CHO-K1 cells were mixed and then treated with an inhibitor (a nucleic acid ligand or neutralizing antibody), and thus the PD-1/PD-L1 binding inhibitory activity was tested at the cellular level. This system corresponded to a cell-based inhibitory activity measurement method designed such that the fluorescence signal increased as the inhibitory activity increased, and the PD-L1 neutralizing antibody was used as a positive control.

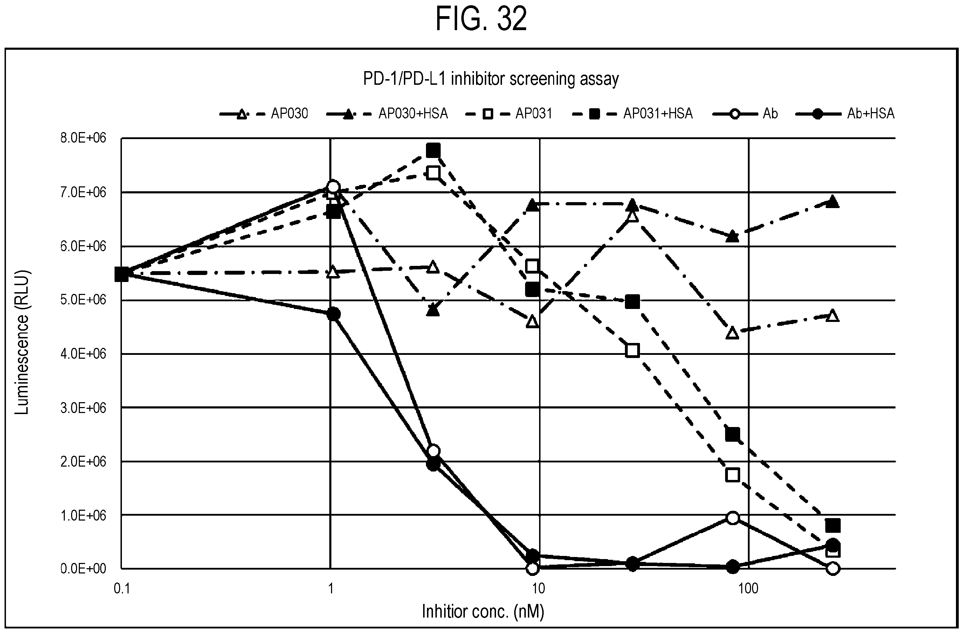

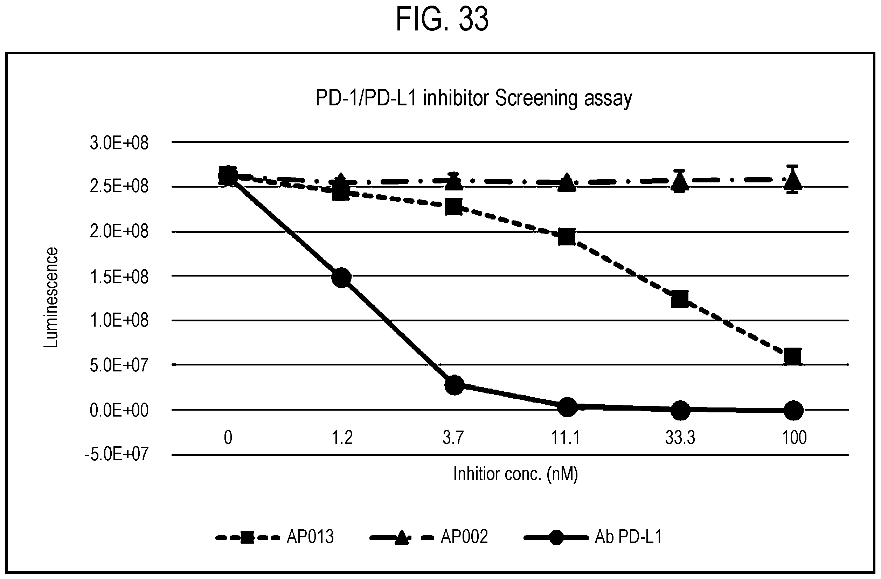

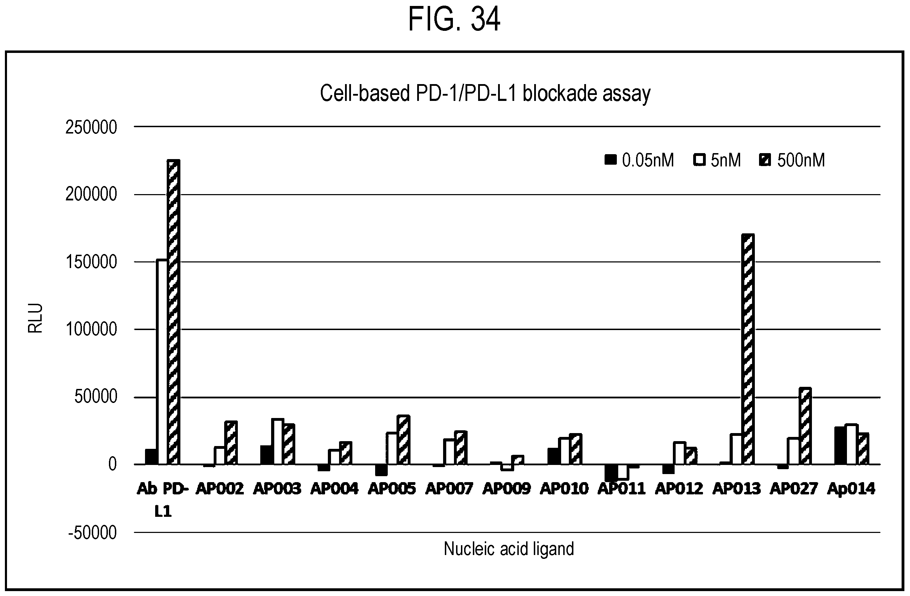

According to the test results at the protein level, the inhibitory effect of PD-1 and PD-L1 binding was shown by the treatment with the nucleic acid ligands capable of binding to PD-L1 according to one embodiment of the present disclosure (see FIGS. 32 and 33). These results confirmed that the nucleic acid ligands AP013 and AP031 according to one embodiment of the present disclosure inhibited the binding of PD-1 and PD-L1 by binding to PD-L1, thereby blockading the immune cell attack evasion pathway of cancer cells. In particular, this PD-1/PDL1 binding inhibition was also confirmed in the cellular level tests, indicating that the nucleic acid ligands according to one embodiment of the present disclosure can be utilized in the treatment of PD-L1-overexpressed cancer (see FIG. 34).

## Claims

1. A non-natural nucleic acid ligand having specific binding affinities for two or more different targets,
wherein each of the targets has a three-dimensional structure, and
wherein the non-natural nucleic acid ligand is not a coupled body of a plurality of aptamers (non-coupling).

2. A non-natural nucleic acid ligand having specific binding affinities for two or more different targets,
wherein each of the targets has a three-dimensional structure, and
wherein in the nucleic acid ligand, all or part of a nucleic acid sequence forming a binding site to one target forms all or part of a nucleic acid sequence forming a binding site to another target.

3. A non-natural nucleic acid ligand having specific binding affinities for two or more different targets,
wherein each of the targets has a three-dimensional structure, and
wherein in the nucleic acid ligand, binding sites to two or more targets are not present separately from each other.

4. A non-natural nucleic acid ligand having specific binding affinities for two or more different targets,
wherein each of the targets has a three-dimensional structure, and
wherein binding sites to two or more targets are formed in one single nucleic acid ligand.

5. The non-natural nucleic acid ligand of claim 2, wherein the part of the nucleic acid sequence forming binding sites to the targets is about 1% to about 99%, about 5% to about 95%, about 10% to about 90%, about 20% to about 80%, about 30% to about 70%, or about 40% to about 60% of the entire sequence of the non-natural nucleic acid ligand.

6. The non-natural nucleic acid ligand of any one of claims 1 to 4, wherein the non-natural nucleic acid ligand has specific binding specificities to two different targets.

7. The non-natural nucleic acid ligand of any one of claims 1 to 4, wherein the targets are selected from the group consisting of cells, viruses, and proteins.

8. The non-natural nucleic acid ligand of claim 6, wherein one of the targets is a plasma protein and the other is a therapeutic target.

9. The non-natural nucleic acid ligand of claim 8, wherein the plasma protein is selected from the group consisting of albumin, alpha 1 globulin, alpha 2 globulin, beta globulin, gamma globulin, immunoglobulin A, immunoglobulin G, lipoproteins, fibrinogen, transferrin, and transthyretin.

10. The non-natural nucleic acid ligand of claim 8, wherein the therapeutic target is a protein present in a cell or a protein present in a cellular membrane.

11. The non-natural nucleic acid ligand of claim 8, wherein the therapeutic target is selected from the group consisting of membrane proteins, transmembrane proteins, glycoproteins, immune antibodies, viruses, viral envelope glycoproteins, viral enzymes, secretory proteins, serine proteases, peptide hormones, neurotransmitters, hormones, dehydrogenases, cytokines, E3 ubiquitin ligases, neuropeptides, hydrolases, serine protease inhibitors, phosphatase, chemokine proteins, methyl transferases, oxidases, growth factors, bacteria, bacterial proteins, intracellular proteins, extracellular matrices, receptors, transcription factors, and tumor proteins.

12. The non-natural nucleic acid ligand of claim 8, wherein the therapeutic target is selected from the group consisting of 4-1BB, acetylcholine receptors, alpha thrombin, amylin, angiopoietin 1, angiopoietin 2, AXL, BCL-2, BMPR-1R, BRD1, BRD2, BRD3, BRD4, BRDT, BTLA, calcitonin gene-related peptides, CREB-binding protein (CPB), CCK4/PTK7, CD16a, CD16b, CD19, CD20, CD200, CD200R, CD27, CD28, CD3, CD30, CD32A, CD32B, CD33, CD4, CD40L, CD52, CD80, CD94, CSF1R, CTLA-4, DDR1, DDR2, E2F1, EGFR, EPH, ERBB2, FGF, FGFR, ghrelin, GITR, glypican 3, gonadotropin-releasing hormone 1, HIV gp120, HIV-1 integrase, HIV-1 reverse transcriptase, HRAS, HVEM, ICOS, IDH1, IGF1R, immunoglobulin E, interferon-gamma, KIT, KRAS, LAG3, LFA, L-selectin, LTK, MDM2, MDMX, mucin 1, MYC, neurotensin 1, neutrophil elastase, NF-Kb, NKG2D, nociceptin, NTRK1, NTRK2, OX-40, PD-1, PDGF, PDGFR family, PD-L1, PD-L2, phospholipase A2, plasminogen activation inhibitor 1, PP2A, PPM1D, PPP2CA, protein tyrosine phosphatase, P-selectin, PSMA, respiratory syncytial virus, RET, SDF1b, SetDB1, SLAMF7, Staphylococcus enterotoxin B, STAT3, tenascin, TGFBR, TIGIT, TIM3, TNFSF7, tyrosinase, VCAM-1, VEGF, VEGFR family, αᵥβ₃ integrin, and mutants thereof.

13. The non-natural nucleic acid ligand of claim 8, wherein the therapeutic target is selected from the group consisting of KRAS G12D, KRAS G12V, KRAS G12C, KRAS G12A, KRAS G12S, KRAS G12R, KRAS G13D, KRAS Q61H, and combinations thereof.

14. The non-natural nucleic acid ligand of any one of claims 1 to 4, wherein the non-natural nucleic acid ligand consists of about 100 or less nucleotides.

15. The non-natural nucleic acid ligand of claim 14, wherein the nucleotides are selected from the group consisting of DNA nucleotides, RNA nucleotides, modified nucleotides thereof, and combinations thereof.

16. The non-natural nucleic acid ligand of any one of claims 1 to 4, wherein the non-natural nucleic acid ligand consists of one nucleic acid sequence selected from the group consisting of SEQ ID NO: 6 to SEQ ID NO: 183.

17. The non-natural nucleic acid ligand of any one of claims 1 to 4, wherein the nucleic acid is selected from the group consisting of single-stranded RNA, double-stranded RNA, single-stranded DNA, and double-stranded DNA.

18. A pharmaceutical composition for prevention or treatment of cancer, wherein the pharmaceutical composition comprises the non-natural nucleic acid ligand of any one of claims 1 to 4.

19. The pharmaceutical composition of claim 18, wherein the non-natural nucleic acid ligand has specific binding affinities for a plasma protein as a first target and a therapeutic target protein as a second target.

20. The pharmaceutical composition of claim 18, wherein the pharmaceutical composition is administered intravenously to a patient.

21. The pharmaceutical composition of claim 18, wherein the cancer is a solid cancer.

22. The pharmaceutical composition of claim 18, wherein the cancer expresses a PD-L1 protein on the surface of cancer cells.

23. The pharmaceutical composition of claim 22, wherein the cancer is at least one selected from the group consisting of brain cancer, lung cancer, colorectal cancer, small intestine cancer, stomach cancer, testicular cancer, thyroid cancer, cervical cancer, skin cancer, bladder cancer, ovarian cancer, kidney cancer, liver cancer, pancreatic cancer, urothelial cell cancer, and breast cancer.

24. The pharmaceutical composition of claim 18, wherein cancer cells of the cancer have a KRAS mutant protein.

25. The pharmaceutical composition of claim 24, wherein the cancer is at least one selected from the group consisting of lung cancer, colorectal cancer, pancreatic cancer, breast cancer, ovarian cancer, endometrial cancer, cervical cancer, bladder cancer, gallbladder cancer, biliary tract cancer, skin cancer, stomach cancer, brain cancer, kidney cancer, and acute myeloid leukemia.

26. The pharmaceutical composition of claim 18, wherein the non-natural nucleic acid ligand consists of one nucleic acid sequence selected from the group consisting of SEQ ID NO: 24, SEQ ID NO: 27, SEQ ID NO: 30, SEQ ID NO: 84, and SEQ ID NO: 100.

27. A diagnostic composition comprising the non-natural nucleic acid ligand of any one of claims 1 to 4.

28. A contrast medium comprising the non-natural nucleic acid ligand of any one of claims 1 to 4.

29. A radiopharmaceutical comprising the non-natural nucleic acid ligand of any one of claims 1 to 4.

30. A composition for diagnosis of cancer, the composition comprising the non-natural nucleic acid ligand of any one of claims 1 to 4.

31. A method for identifying a non-natural nucleic acid ligand having specific binding affinities for two or more different targets, the method comprising:
contacting one or more nucleic acid ligand candidate sequences with each of two or more different targets in a sequential manner,
wherein the contacting with each of the targets comprises identifying one or more nucleic acid ligand candidate sequences having specific binding affinity for said target, and
wherein each of the targets has a three-dimensional structure.

32. The method of claim 31, wherein the method comprises:
(a) contacting one or more first nucleic acid ligand candidate sequences with a target and identifying one or more second nucleic acid ligand candidate sequences specifically binding to the target; and
(b) contacting (i) the one or more second nucleic acid ligand candidate sequences obtained in step (a) or (ii) one or more third nucleic acid ligand candidate sequences prepared using the one or more second nucleic acid ligand candidate sequences obtained in step (a) with a target different from the target in step (a) and identifying one or more fourth nucleic acid ligand candidate sequences specifically binding to the different target.

33. The method of claim 32, wherein step (b) further comprises preparing the one or more third nucleic acid ligand candidate sequences by linking a random sequence consisting of a plurality of nucleotides to at least one of both ends of the one or more second nucleic acid ligand candidate sequences obtained in step (a).

34. The method of claim 33, wherein the random sequence is linked to any one of both ends of the second nucleic acid ligand candidate sequences, and
wherein in the third nucleic acid ligand candidate sequences, the second nucleic acid ligand candidate sequence or a part thereof is used as a primer sequence for nucleic acid amplification.

35. The method of claim 33, wherein the random sequence consists of about 20 to about 40 consecutive nucleotides, or the third nucleic acid ligand candidate sequences consist of about 40 to about 100 consecutive nucleotides.

36. The method of claim 31, wherein the method is performed by systematic evolution of ligands by exponential enrichment (SELEX).

37. A method for identifying a non-natural nucleic acid ligand having specific binding affinities for two or more different targets, the nucleic acid ligand being identified by systematic evolution of ligands by exponential enrichment (SELEX) starting from one nucleic acid library, the method comprising:
selecting one target from the group consisting of two or more different targets each having a three-dimensional structure in each SELEX round; and
selecting a nucleic acid ligand having specific binding affinity for the selected target in each SELEX round.

38. The method of claim 36 or 37, wherein after SELEX performed in one or more rounds for one target is completed, SELEX performed in one or more rounds for another target is performed.

39. The method of claim 36 or 37, wherein the method comprises:
(i) performing one or more SELEX rounds on one target;
(ii) performing one or more SELEX rounds on a target different from the target in step (i) by using a nucleic acid library obtained in step (i);
(iii) performing step (i) again by using a nucleic acid library obtained in step (ii); and
(iv) repeating steps (i) to (iii).

40. The method of claim 36 or 37, further comprising performing negative SELEX or counter SELEX for another trait of the target.

41. The method of claims 36 or 37, wherein the SELEX is at least one selected from the group consisting of classic SELEX, Advanced SELEX, IP-SELEX, Capture-SELEX, Cell-SELEX, CE-SELEX, M-SELEX, AFM-SELEX, AEGIS-SELEX, and Animal-SELEX.

42. The method of any one of claims 31 to 37, further comprising evaluating specific binding affinities of the identified nucleic acid ligand candidate sequence for targets.

43. The method of any one of claims 31 to 37, wherein the targets are selected from the group consisting of cells, virus, and proteins.

44. The method of any one of claims 31 to 37, wherein the targets are two different targets.

45. The method of claim 44, wherein one of the targets is a plasma protein and the other is a therapeutic target.

46. The method of any one of claims 31 to 37, wherein the non-natural nucleic acid ligand consists of about 40 to about 100 nucleotides.

47. The method of any one of claims 31 to 37, wherein the method comprises removing nucleotides that are uninvolved in specific binding affinity for a target from each of the identified nucleic acid ligand candidate sequences.

48. A method for detecting a target from a sample, the method comprising contacting a target from the sample with the nucleic acid ligand of any one of claims 1 to 4.

49. A non-natural nucleic acid ligand prepared by the method of any one of claims 31 to 37.
